# EUROPEAN PATENT SPECIFICATION

(11) **EP 4 093 730 B1**
(45) Date of publication and mention of the grant of the patent: **08.05.2024**
(21) Application number: 21702304.3
(22) Date of filing: 22.01.2021
(51) Int. Cl.: C07D 207/16, C07D 401/04, C07D 417/12, C07D 417/14, A61P 35/00, A61K 31/4015, A61K 31/427

(54) **HDAC DEGRADER**
HDAC-DEGRADER
AGENT DE DÉGRADATION DE HDAC

(30) Priority: 24.01.2020 GB 202001023
(43) Date of publication of application: 30.11.2022
(73) Proprietor: University of Leicester, Leicester, Leicestershire LE1 7RH (GB)
(72) Inventor: COWLEY, Shaun, Leicester Leicestershire LE1 7RH (GB); HODGKINSON, James, Leicester Leicestershire LE1 7RH (GB); CROSS, Jasmine M., Leicester Leicestershire LE1 7RH (GB); SCHWABE, John, Leicester Leicestershire LE1 7RH (GB); SMALLEY, Joshua, Leicester Leicestershire LE1 7RH (GB); ADAMS, Grace, Leicester Leicestershire LE1 7RH (GB); MILLARD, Chris, Leicester Leicestershire LE1 7RH (GB)
(74) Representative: Petty, Catrin Helen
(86) International application number: PCT/GB2021/050156
(87) International publication number: WO 2021/148811

(56) References cited:
- WO-A1-2007/118137
- WO-A1-2017/004522
- US-A1- 2010 311 794
- SMALLEY JOSHUA P. ET AL: "PROTAC-mediated degradation of class I histone deacetylase enzymes in corepressor complexes", CHEMICAL COMMUNICATIONS, [Online] vol. 56, no. 32, 11 March 2020 (2020-03-11), pages 4476-4479, XP055778976, ISSN: 1359-7345, DOI: 10.1039/D0CC01485K Retrieved from the Internet: URL:https://pubs.rsc.org/en/content/articl epdf/2020/cc/d0cc01485k?page=search>
- ZHANG QING-WEI ET AL: "Cap-Modified Hydroxamate Analogues as Histone Deacetylases Inhibitors and Antitumor Agents", BULLETIN OF THE KOREAN CHEMICAL SOCIETY, vol. 35, no. 1, 20 January 2014 (2014-01-20), pages 129-134, XP055778990, KR ISSN: 0253-2964, DOI: 10.5012/bkcs.2014.35.1.129
- ZHANG XUAN ET AL: "Design, synthesis and biological evaluation of 4'-demethyl-4-deoxypodophyllotoxin derivatives as novel tubulin and histone deacetylase dual inhibitors", RSC ADV., vol. 4, no. 76, 22 August 2014 (2014-08-22), pages 40444-40448, XP055779044, DOI: 10.1039/C4RA05508J Retrieved from the Internet: URL:https://pubs.rsc.org/en/content/articl epdf/2014/ra/c4ra05508j?page=search>
- Jiang Yuqi ET AL: "Discovery of Multi-target Anticancer Agents Based on HDAC Inhibitor MS-275 and 5-FU", Medicinal Chemistry, vol. 12, no. 1 5 January 2016 (2016-01-05), pages 30-36, XP055779054, Retrieved from the Internet: URL:https://www.rsc.org/journals-books-dat abases/about-journals/rsc-medicinal-chemis try/ [retrieved on 2021-02-23]
- CHRISTIAN STEINEBACH ET AL: "PROTAC-mediated crosstalk between E3 ligases", CHEMICAL COMMUNICATIONS, vol. 55, no. 12, 16 January 2019 (2019-01-16), pages 1821-1824, XP055588704, ISSN: 1359-7345, DOI: 10.1039/C8CC09541H

## Description

The present invention is concerned with compounds which may degrade the Histone Deacetylase (HDAC) family of enzymes, particularly HDAC1, 2 and 3 that exist in corepressor complexes. The invention extends to pharmaceutical compositions comprising these compounds, and the use of these compounds in therapy. The invention explains how these compounds may be used to treat cancer.

Targeting enzymes involved in epigenetic modifications have provided therapeutic drugs in treating cancer and have potential in treating other diseases including neurological disorders and cardiovascular disease. The Histone Deacetylase (HDAC) family of enzymes, often termed epigenetic erasers, function by removing the acetyl moiety from histone tails thereby modifying chromatin structure and gene expression. Currently five HDAC inhibitors have been approved for clinical use to treat T-cell lymphoma and multiple myeloma with other compounds in clinical trials. Humans contain 18 HDAC enzymes, 11 with a divalent zinc cation in the catalytic site and 7 sirtuins whose activity is NAD+ dependent. Inhibitors of the Zn²⁺-dependent enzymes, such as Valproic acid or SAHA (Zolinza), are currently used in the clinic to treat CTCL and bipolar disorder. However, these drugs exhibit limited selectivity and this lack of selectivity has been attributed as a cause of debilitating side-effects exhibited by patients using HDAC inhibitor drugs.

HDACs 1, 2 and 3 are localized in the nucleus, constitute approximately 50% of total cellular deacetylase activity and are the most prominent HDACs in gene expression. They do not function as singular entities, but exist *in vivo* as catalytic subunits in much larger multiprotein corepressor complexes, including Sin3, NuRD, CoREST, MiDAC and NCoR. These complexes play an essential role incorporating the HDAC enzyme to specific regions in the genome and demonstrate distinct cell type functions.

Smalley et al. (J. P. Smalley, G. E. Adams, C. J. Millard, Y. Song, J. K.S. Norris, J. W.R. Schwabe, S. Cowley and J. T. Hodgkinson, Chem. Commun., 2020, 32(56), 4476-4479) describes PROTAC-mediated degradation of class I HDAC enzymes in corepressor complexes.

US 2010/0311794 A1 describes cycloalkylcarbamate benzamide aniline HDAC inhibitor compounds. Similarly, WO 2017/004522 A1 also describes compounds which can be used to inhibit HDAC enzymes.

Zhang et al. (Q-W. Zhang, J. Feng and J-Q. Li, Bull. Korean Chem. Soc., 2014, 1(35), 129-134) describes cap-modified hydrozamate analogues as HDAC inhibitors and antitumor agents.

Zhang et al. (X. Zhang, J. Zhang, M. Su, Y. Zhou, Y. Chen, J. Li and W. Lu, RSC Adv., 2014, 4(76), 40444-40448) describes the design, synthesis and biological evaluation of 4-demethyl-4-deoxypodophyllotoxin derivatives as novel tubulin and HDAC dual inhibitors.

Jiang et al. (Y. Jiang, X. Li, X. Li, J. Hou, Y. Ding, J. Ziang, W. Xu and Y. Zhang, Medicinal Chemistry, 2016, 12, 30-36) reports the discovery of multi-target anticancer agents based on HDAC inhibitor MS-275 and 5-FU.

WO 2007/118137 A1 discloses benzamide derivatives which display HDAC inhibitory activity and compositions for treating cell proliferative diseases and conditions.

Steinebach et al. (C. Steinebach, H. Kehm, S. Lindner, L. P. Vu, S. Köpff, Á. López Mármol, C. Weiler, K. G. Wahner, M. Reichenzeller, J. Krönke and M. Gütschow, Chem. Commun., 2019, 12(55), 1821-1824) discloses proteolysis targeting chimeras (PROTACs) assembled from a cereblon (CRBN) and a von-Hippel-Lindau (VHL) ligase ligand and demonstrates PROTAC-induced heterodimerization of two E3 ligases leading to CRBN degradation.

The present invention arises from the inventors' work in attempting to develop alternative compounds to target the HDAC family of enzymes.

The present invention is defined by the claims.

In accordance with a first aspect, there is provided a compound of formula (I): 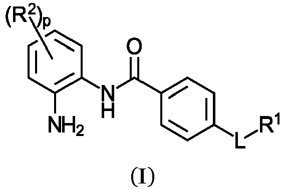 wherein L is a linker with a backbone comprising at least one group, the or each group being independently selected from the list consisting of an optionally substituted C₁-C₃₀ alkylene, an optionally substituted C₂-C₃₀ alkenylene, an optionally substituted C₂-C₃₀ alkynylene, NR₃, O, S, SO, SO₂, an optionally substituted C₆-C₁₂ arylene and an optionally substituted 5 to 10 membered heteroarylene, wherein the backbone of the linker is between 7 and 50 atoms in length;
R¹ is an E3 ligand for an E3 ligase, which is: 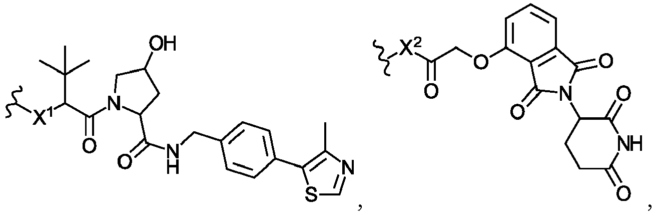 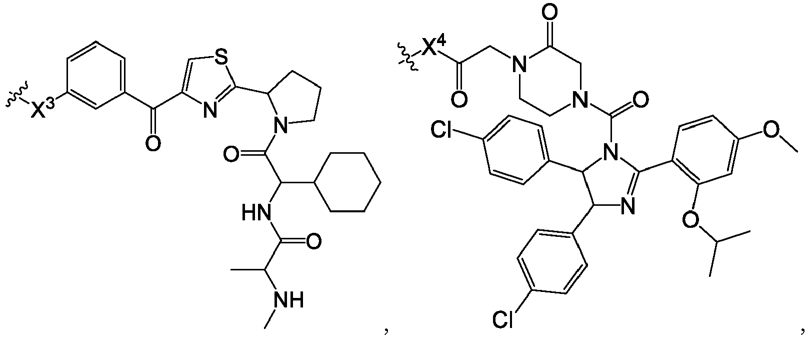 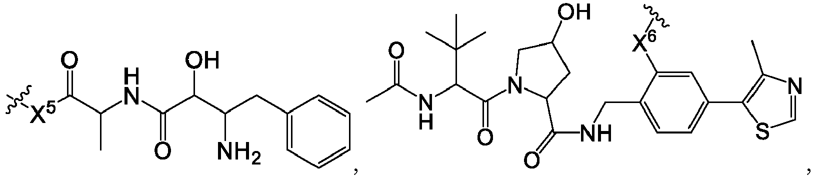 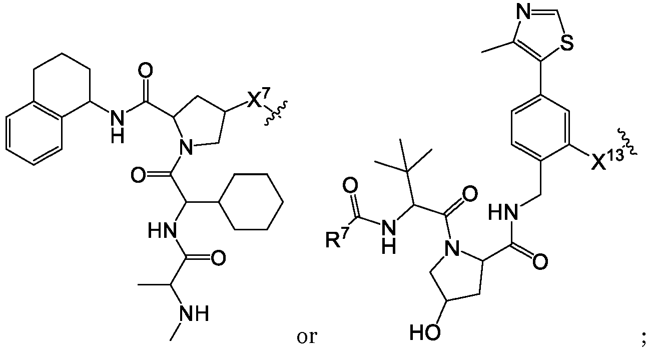
each R² is independently a halogen, OR³, NR³R⁴, C₁-C₆ alkyl, C₂-C₆ alkenyl, C₂-C₆ alkynyl, a C₆-C₁₂ aryl group or a 5 to 10 membered heteroaryl group;
R3 and R4 are independently H, C₁-C₆ alkyl, C₂-C₆ alkenyl or C₂-C₆ alkynyl;
p is o or an integer between 1 and 4;
X¹ to X⁷ and X¹³ are each NH or O and R⁷ is H, an optionally substituted C₁-C₆ alkyl, an optionally substituted C₂-C₆ alkenyl, an optionally substituted C₂-C₆ alkynyl, an optionally substituted C₃-C₆ cycloalkyl, an optionally substituted 3 to 6 membered heterocycle, an optionally substituted phenyl or an optionally substituted 5 or 6 membered heteroaryl;
wherein the or each optionally substituted alkylene, optionally substituted alkenylene, optionally substituted alkynylene, optionally substituted heteroaryl and optionally substituted heteroarylene may be unsubstituted or substituted with one or more of halogen, oxo, OH, NH₂, C₁-C₆ alkyl, C₂-C₆ alkenyl, C₂-C₆ alkynyl, a C₃-C₆ cycloalkyl, a 3 to 10 membered heterocycle, a C₆-C₁₂ aryl and/or a 5 to 10 membered heterocycle; and the or each optionally substituted arylene may be unsubstituted or substituted with one or more of halogen, OH, NH₂, C₁-C₆ alkyl, C₂-C₆ alkenyl, C₂-C₆ alkynyl, a C₃-C₆ cycloalkyl, a 3 to 10 membered heterocycle, a C₆-C₁₂ aryl and/or a 5 to 10 membered heterocycle;
or a pharmaceutically acceptable salt, solvate, tautomeric form or polymorphic form thereof.

The compound of formula (I) is a Proteolysis Targeting Chimera (PROTAC). PROTACs are heterobifunctional molecules that couple a ligand for the protein of interest (POI) with a ligand to an E3 ligand and thus recruit an E3 ligase resulting in polyubiquitination of the POI and degradation. Advantageously, a compound of formula (I) is able to induce a successful protein-protein interaction with a HDAC enzyme, and degradation. In particular, the inventors have found that in an HCT116 colon cancer cell line a compound of formula (I) can increase histone acetylation levels and reduce cell viability comparable to clinical candidate CI-994.

As far as the inventors are aware this is the first time anyone has taken a selective Benzamide HDAC inhibitor, such as CI-994, and transformed it into a PROTAC and then demonstrated HDAC1, 2 and 3 degradation. Given the significance and importance of HDAC drugs in disease, and the benefits of the PROTAC technology, the medical significance and potential applications could be very high.

The term "alkylene", as used herein, unless otherwise specified, refers to a bivalent saturated straight hydrocarbon. The terms "alkenylene" and "alkynylene", as used herein, unless otherwise specified, refer, respectively, to a bivalent olefinically unsaturated straight hydrocarbon or a bivalent acetylenically unsaturated straight hydrocarbon. The term "arylene" refers to a bivalent aromatic 6 to 12 membered hydrocarbon group. The term "heteroarylene" refers to a bivalent aromatic 5 to 10 membered ring system in which at least one ring atom is a heteroatom.

The or each optionally substituted alkylene, optionally substituted alkenylene, optionally substituted alkynylene and optionally substituted heteroaryl may be unsubstituted or substituted with one or more of halogen, oxo, OH, NH₂, C₁-C₆ alkyl, C₂-C₆ alkenyl, C₂-C₆ alkynyl, a C₃-C₆ cycloalkyl, a 3 to 10 membered heterocycle, a C₆-C₁₂ aryl and/or a 5 to 10 membered heterocycle. The or each optionally substituted aryl may be unsubstituted or substituted with one or more of halogen, OH, NH₂, C₁-C₆ alkyl, C₂-C₆ alkenyl and/or C₂- C₆ alkynyl, a C₃-C₆ cycloalkyl, a 3 to 10 membered heterocycle, a C₆-C₁₂ aryl and/or a 5 to 10 membered heterocycle.

It may be appreciated that the term "backbone of the linker" refers to the shortest continuous chain of bonded atoms between 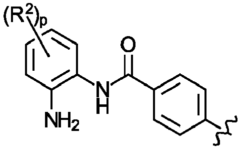 and R¹.

In some embodiments, p is 1. Accordingly, the compound of formula (I) may be a compound of formula (Iai) or (Iaii):

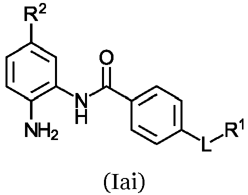

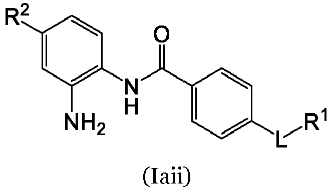

R² may be a halogen or a 5 or 10 membered heteroaryl group. In embodiments where R² is a 5 or 10 membered heteroaryl group it may be a 5 or 6 membered heteroaryl group. Accordingly, R² may be 1H-pyrrolyl, furanyl or thiophenyl. In embodiments where R² is a halogen it may be fluorine, chlorine, iodine or bromine. Preferably, R² is fluorine or chlorine, and most preferably is fluorine.

Accordingly, the compound of formula (I) may be a compound of formula (Iaiii) or (Iaiv):

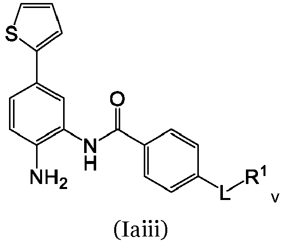

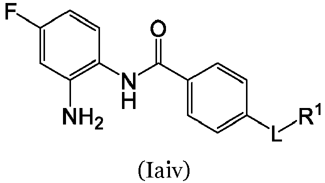

In an alternative embodiment, p is o. Accordingly, the compound of formula (I) may be a compound of formula (Ib): 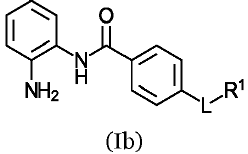 R¹ has the following structure: 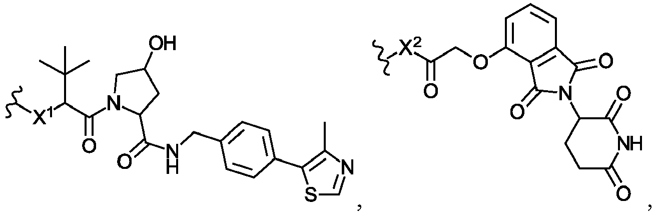 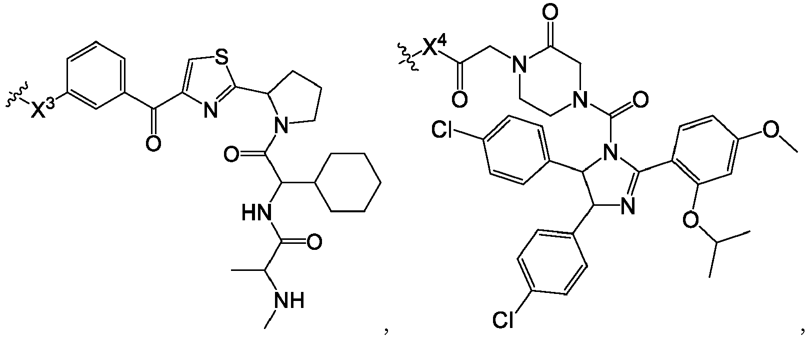 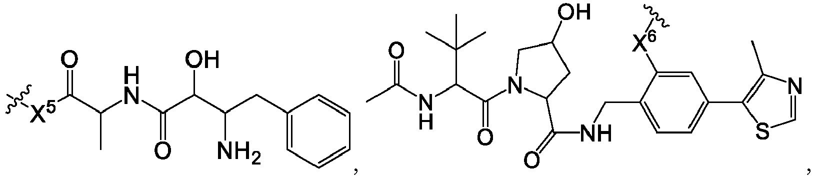 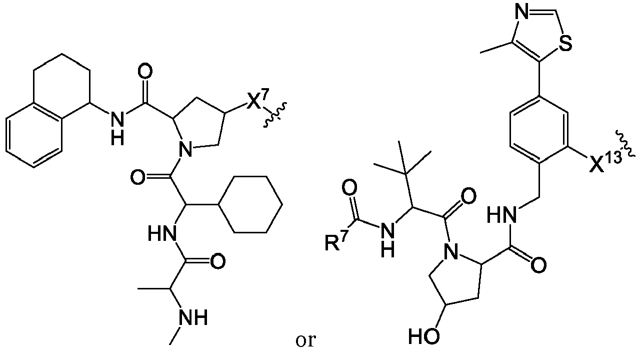 wherein X¹ to X7 and X¹³ are each NH or O and R⁷ is H, an optionally substituted C₁-C₆ alkyl, an optionally substituted C₂-C₆ alkenyl, an optionally substituted C₂-C₆ alkynyl, an optionally substituted C₃-C₆ cycloalkyl, an optionally substituted 3 to 6 membered heterocycle, an optionally substituted phenyl or an optionally substituted 5 or 6 membered heteroaryl. The alkyl, alkenyl, alkynyl, cycloalkyl, heterocycle, phenyl or heteroaryl may be unsubstituted or substituted with one or more of halogen, oxo, OH or NH₂. The halogen may be fluorine, chlorine, bromine or iodine, and is preferably fluorine or chlorine, and more preferably is fluorine.

More preferably, R¹ is 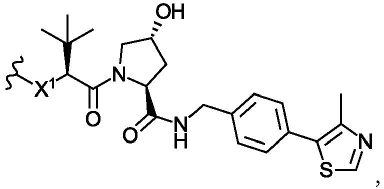 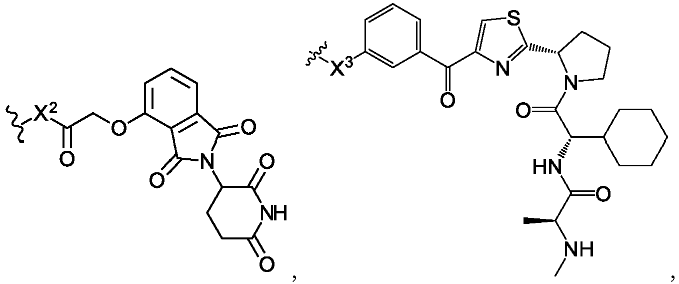 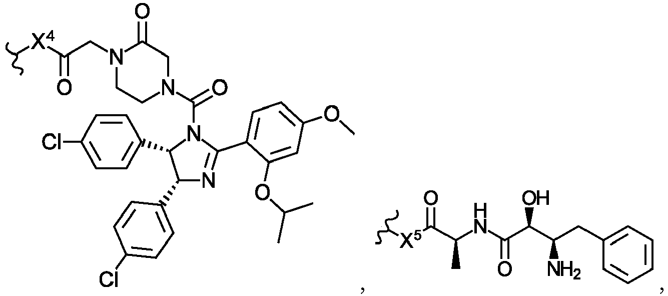 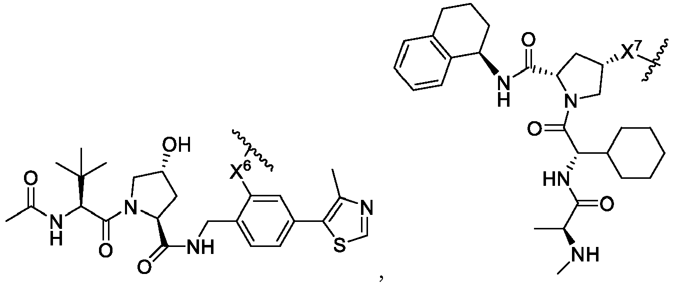 or

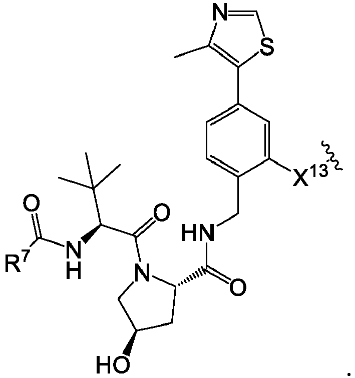

Preferably, X¹ and X² are NH. Preferably X3 is O. Preferably X⁶ is O. Preferably X7 is NH. Preferably, X¹³ is O.

Preferably, R⁷ is an optionally substituted C₁-C₃ alkyl, an optionally substituted C₂-C₃ alkenyl, an optionally substituted C₂-C₃ alkynyl, an optionally substituted cyclopropyl or an optionally substituted 3 membered heterocycle. More preferably, R⁷ is methyl or

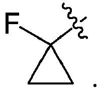

Most preferably, R¹ is ,

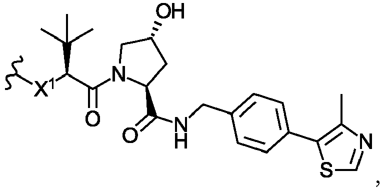

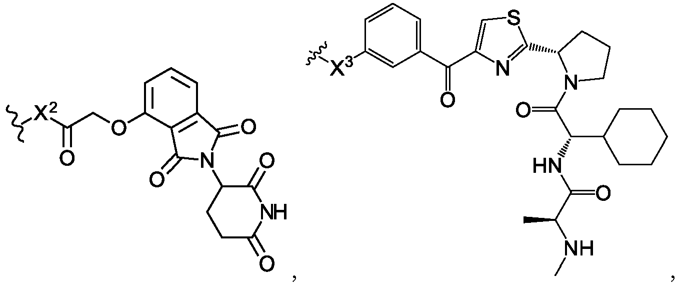

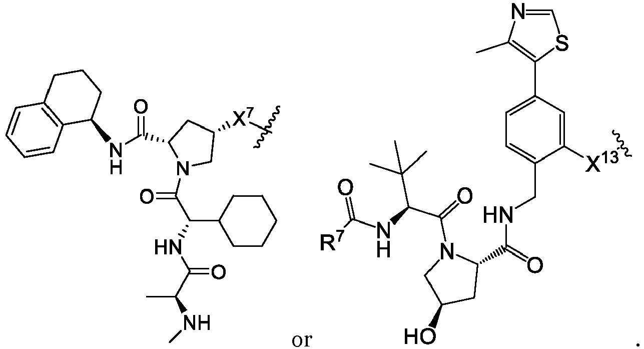

The backbone of the linker may be between 7 and 40 atoms in length, more preferably between 8 and 30, between 9 and 25, between 10 and 20 or between 11 and 15 atoms in length, and most preferably 13 atoms in length.

The backbone of the linker may be between 7 and 40 atoms in length, more preferably between 8 and 30, between 9 and 25, between 10 and 20 or between 11 and 18 atoms in length, and most preferably between 13 and 16 atoms in length.

As explained in the examples, the inventors had particularly promising results in *vivo* for compounds with longer linker lengths. This is surprising because the results in *vitro* indicated that molecules with shorter linkers were more active. Additionally, the inventors would have expected compounds with longer linkers to have difficulty in passing through the cell membrane. The fact that compounds with longer linker lengths are more active could not have been predicted.

L may be -L¹-L²-, wherein:
L¹ is absent or is -L³-L⁴-L⁵-L⁶- and L² is -L⁷-L⁸-L⁹-L¹⁰-, wherein:
L³ is an optionally substituted C₁-C₆ alkylene, an optionally substituted C₂-C₆ alkenylene or an optionally substituted C₂-C₆ alkynylene;
L⁴ is NR⁵, O, S, 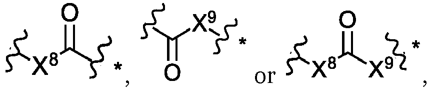 where an asterisk indicates a point of bonding to L⁵ or, if L⁵ is absent, L⁶;
L⁵ is absent, an optionally substituted C₁-C₆ alkylene, an optionally substituted C₂-C₆ alkenylene, an optionally substituted C₂-C₆ alkynylene, an optionally substituted C₆-C₁₂ arylene or an optionally substituted 5 to 10 membered heteroarylene;
L⁶ is an optionally substituted C₆-C₁₂ arylene or an optionally substituted 5 to 10 membered heteroarylene;
L⁷ is absent, an optionally substituted C₁-C₆ alkylene, an optionally substituted C₂-C₆ alkenylene or an optionally substituted C₂-C₆ alkynylene;
L⁸ is absent, 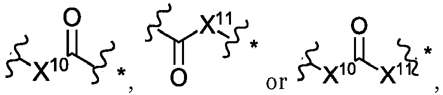 where an asterisk indicates a point of bonding to L⁹;
L⁹ is an optionally substituted C₁-C₂₀ alkylene, an optionally substituted C₂-C₂₀ alkenylene or an optionally substituted C₂-C₂₀ alkynylene, wherein the backbone of the alkylene, alkenylene or alkynylene group is optionally interrupted by one or more heteroatoms selected from O or NR⁶, or L⁹ is 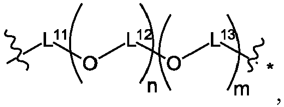 where an asterisk indicates a point of bonding to L¹⁰ or, if L¹⁰ is absent, R¹;
L¹⁰ is absent, C(O) or 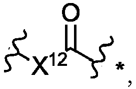 where an asterisk indicates a point of bonding to R¹;
L¹¹ is absent, an optionally substituted C₁-C₅ alkylene, an optionally substituted C₂-C₅ alkenylene or an optionally substituted C₂-C₅ alkynylene;
L¹² and L¹³ are independently an optionally substituted C₁-C₅ alkylene, an optionally substituted C₂-C₅ alkenylene or an optionally substituted C₂-C₅ alkynylene;
X⁸ to X¹² are independently O or NR⁶;
R5 and R⁶ are independently H, a C₁-C₆ alkyl, a C₂-C₆ alkenyl or a C₂-C₆ alkynyl;
m is o an integer between 1 and 10; and
n is an integer between 1 and 10.

In some embodiments, L¹ is present.
L³ may be an optionally substituted C₁-C₃ alkylene, an optionally substituted C₂-C₃ alkenylene or an optionally substituted C₂-C₃ alkynylene. Accordingly, L³ may be -CH₂-.
L4 may be NR⁵ or 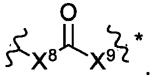 Preferably, X⁸ is NR⁵. Preferably, X⁹ is O. Preferably, R5 is H.
L⁵ may be an optionally substituted C₁-C₃ alkylene, an optionally substituted C₂-C₃ alkenylene or an optionally substituted C₂-C₃ alkynylene. Accordingly, L⁵ may be -CH₂-.

Alternatively, L⁵ may be an optionally substituted phenyl, or an optionally substituted 5 or 6 membered heteroaryl. The 5 or 6 membered heteroaryl may be an optionally substituted pyridinyl, an optionally substituted pyridazinyl, an optionally substituted pyrimidinyl or an optionally substituted triazinyl. The phenyl or heteroaryl may be unsubstituted.

Alternatively, L⁵ may be absent.

L⁶ may be an optionally substituted phenyl, or an optionally substituted 5 or 6 membered heteroaryl. The 5 or 6 membered heteroaryl may be an optionally substituted pyrrolyl, an optionally substituted imidazolyl, an optionally substituted pyrazolyl, an optionally substituted triazolyl, an optionally substituted pyridinyl, an optionally substituted pyridazinyl, an optionally substituted pyrimidinyl or an optionally substituted triazinyl. The phenyl or heteroaryl may be unsubstituted or substituted with a phenyl or a 5 or 6 membered heteroaryl.

Accordingly, in some embodiments, L¹ may be: 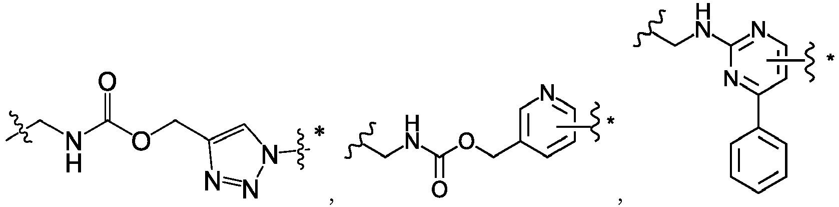 or 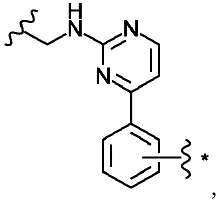 where an asterisk indicates a point of bonding to L².

Alternatively, L¹ may be absent.

L⁷ may be an optionally substituted C₁-C₃ alkylene or an optionally substituted C₂-C₃ alkylyne. Accordingly, L⁷ may be -CH₂-.

In some embodiments, L⁷ is absent.

L⁸ may be 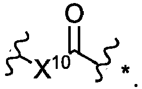 Preferably, X¹⁰ is NR⁶. Preferably, R⁶ is H.

In some embodiments, L⁸ is absent.

In some embodiments, L⁹ may be an optionally substituted C₅-C₁₅ alkylene, an optionally substituted C₅-C₁₅ alkenylene or an optionally substituted C₅-C₁₅ alkynylene. The backbone of the alkylene, alkenylene or alkynylene group may not be interrupted by any heteroatoms. More preferably, L⁹ is an optionally substituted C₈-C₁₃ alkylene, an optionally substituted C₈-C₁₃ alkenylene or an optionally substituted C_{S}-C₁₃ alkynylene, and most preferably a C₁₀-C₁₂ alkylene. Accordingly, in some embodiments, L⁹ may be - (CH₂)₁₁-.

Alternatively, L⁹ may be an optionally substituted C₃-C₁₀ alkylene, an optionally substituted C₃-C₁₀ alkenylene or an optionally substituted C₃-C₁₀ alkynylene. The backbone of the alkylene, alkenylene or alkynylene group may not be interrupted by any heteroatoms. More preferably, L⁹ is an optionally substituted C₅-C₉ alkylene, an optionally substituted C₅-C₉ alkenylene or an optionally substituted C₅-C₉ alkynylene, and most preferably a C₆-C₈ alkylene. Accordingly, in some embodiments, L⁹ may be - (CH₂)₇-.

Alternatively, L⁹ may be an optionally substituted C₃-C₁₅ alkylene, an optionally substituted C₃-C₁₅ alkenylene or an optionally substituted C₃-C₁₅ alkynylene, wherein the backbone of the alkylene, alkenylene or alkynylene group is interrupted by between 1 or more heteroatoms. More preferably, L⁹ is an optionally substituted C₅-C₁₂ alkylene, an optionally substituted C₅-C₁₂ alkenylene or an optionally substituted C₅-C₁₂ alkynylene, and most preferably a C₇-C₁₀ alkylene alkynylene, wherein the backbone of the alkylene, alkenylene or alkynylene group is interrupted by between 1 or more heteroatoms. Preferably, the backbone of the alkylene, alkenylene or alkynylene group is interrupted by between 1 and 4 heteroatoms, more preferably between 1 and 3 and most preferably between 1 and 2. Preferably, the or each heteroatom is O.

In some embodiments, L⁹ may be 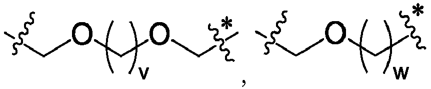 or 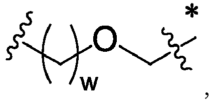 where v is an integer of at least 1 and w is an integer of at least 2 and an asterisk indicates a point of bonding to L¹⁰ or, if L¹⁰ is absent, R¹.

In alternative embodiments, L⁹ may be 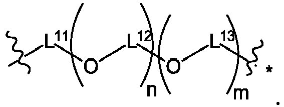 L¹¹ may independently be absent, an optionally substituted C₁-C₃ alkylene, an optionally substituted C₂-C₃ alkenylene or an optionally substituted C₂-C₃ alkynylene. More preferably, L¹¹ is CH₂. L¹² may be an optionally substituted C₂-C₃ alkylene, an optionally substituted C₂-C₃ alkenylene or an optionally substituted C₂-C₃ alkynylene. In some embodiments, L¹² is -CH₂CH₂-. n may be an integer between 1 and 5. In some embodiments, n may be 1, 2 or 3. L¹³ may independently be an optionally substituted C₁-C₃ alkylene, an optionally substituted C₂-C₃ alkenylene or an optionally substituted C₂-C₃ alkynylene. More preferably, L¹³ is CH₂. In some embodiments, m is o. In alternative embodiments, m is 1.

In some embodiments, L¹⁰ is C(O).

In alternative embodiments, L¹⁰ is absent.

Accordingly, L² may be 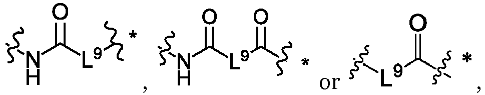 where an asterisk indicates a point of bonding to R¹.

In a preferred embodiment, L² may be 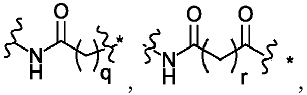 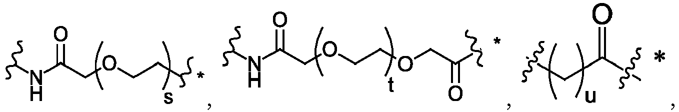 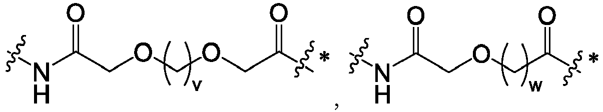 or 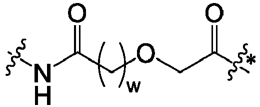 wherein:
an asterisk indicates a point of bonding to R¹;
q is an integer or at least 5;
r is an integer of at least 4;
s is an integer of at least 2;
t is an integer of at least 1;
u is an integer of at least 3;
v is an integer of at least 1; and
w is an integer of at least 2.

In a preferred embodiment, L may be 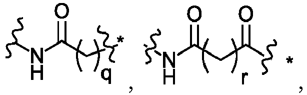 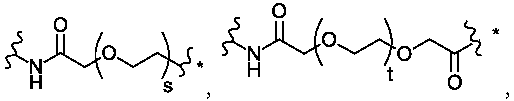 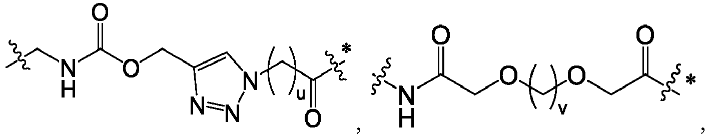 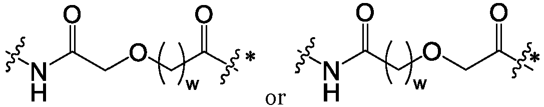 wherein q, r, s, t, u, v and w are as defined above.

Preferably, q is an integer between 5 and 20, more preferably between 7 and 15, and most preferably between 10 and 12. In some embodiments, q is 5, 8 or 11. In a preferred embodiment, q is 11.

Preferably, r is an integer between 4 and 20, more preferably between 7 and 15, and most preferably between 9 and 11. In some embodiments, r is 4, 7, 10 or 12. In a preferred embodiment, r is 10. In an alternative embodiment, r is 12.

Preferably, s is an integer between 2 and 10, more preferably between 2 and 5. In a preferred embodiment, s is 2 or 3.

Preferably, t is an integer between 1 and 10, more preferably between 1 and 5. In a preferred embodiment, t is 1 or 2.

Preferably, u is an integer between 2 and 15, between 3 and 12, between 4 and 10 or between 6 and 8. In some embodiments, u is 7.

Preferably, v is an integer between 1 and 13, between 3 and 12, between 4 and 10 or between 6 and 9. In some embodiments, v is 6. In other embodiments, v is 9.

Preferably, w is an integer between 2 and 14, between 3 and 12, between 5 and 10 or between 7 and 9. In some embodiments, w is 8.

The compound of formula (I) may be a compound of formula (Ic), (Id), (Ie) or (If): 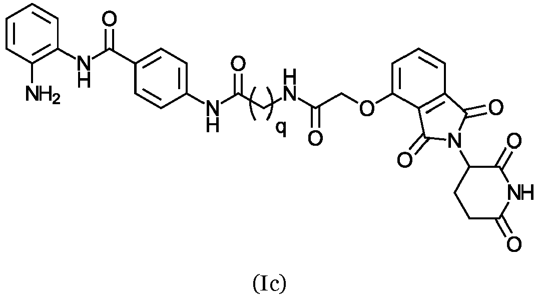 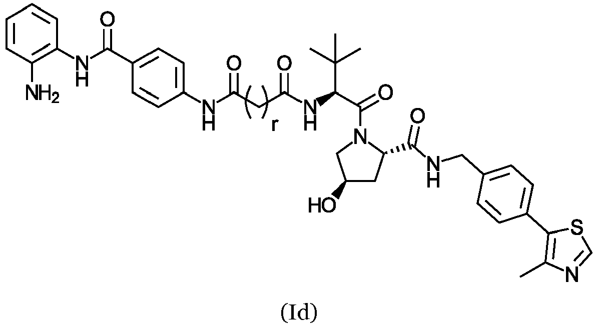 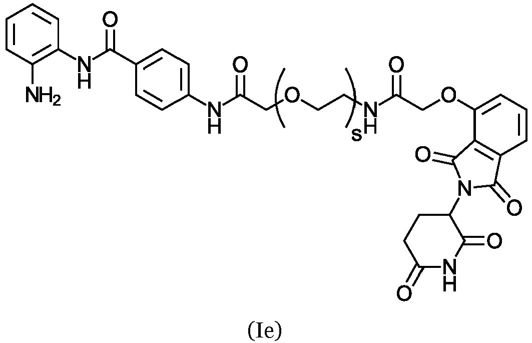 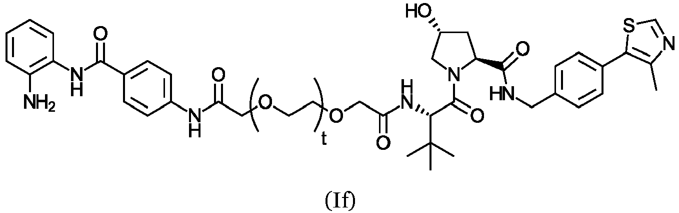 , wherein q, r, s and t are as defined above.

The compound of formula (I) may be a compound of formula (101) to (116):

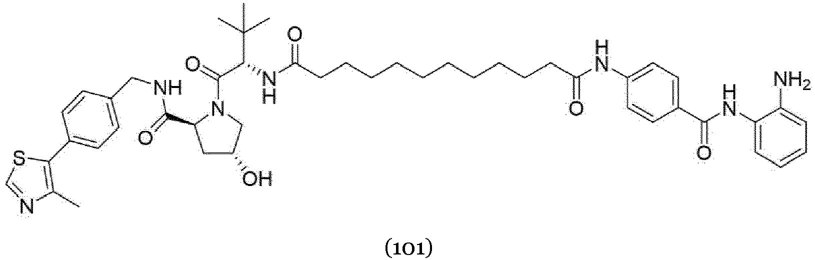

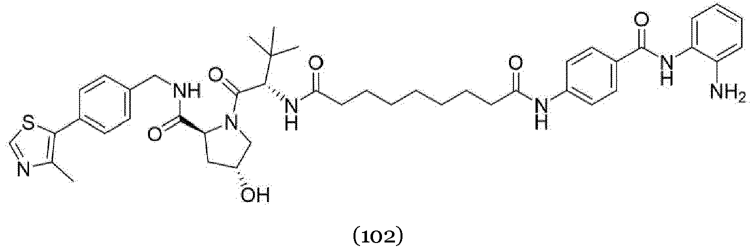

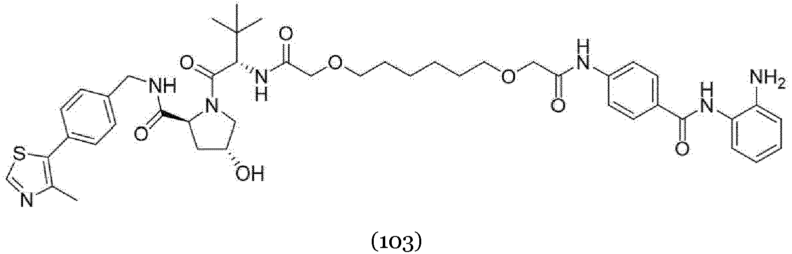

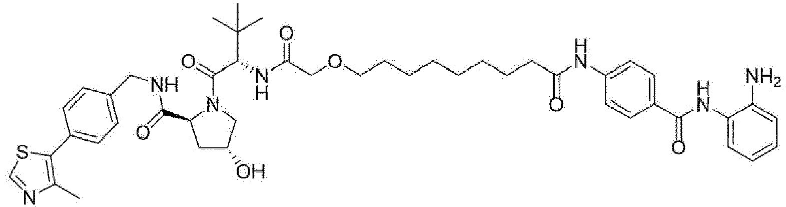

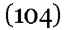

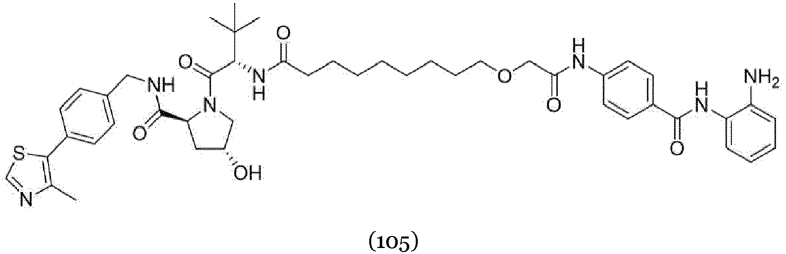

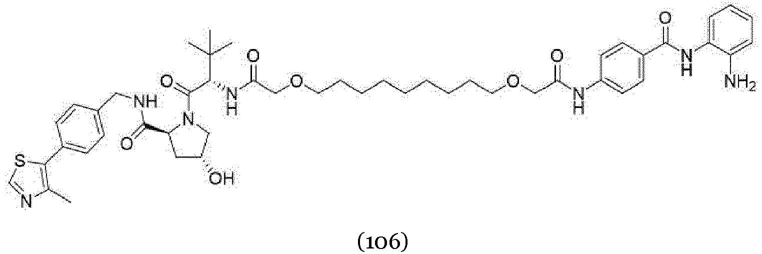

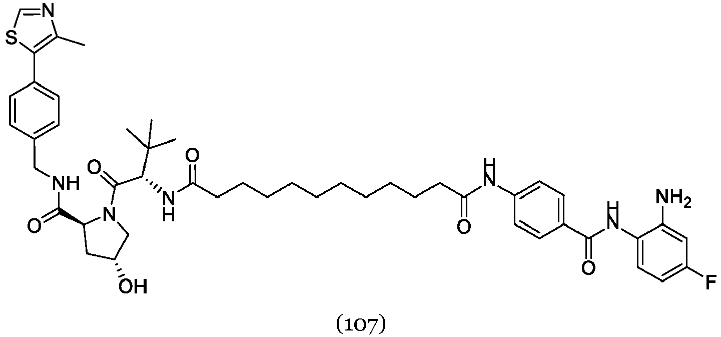

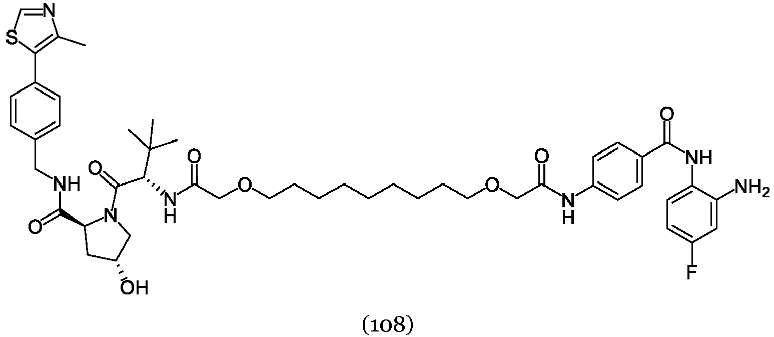

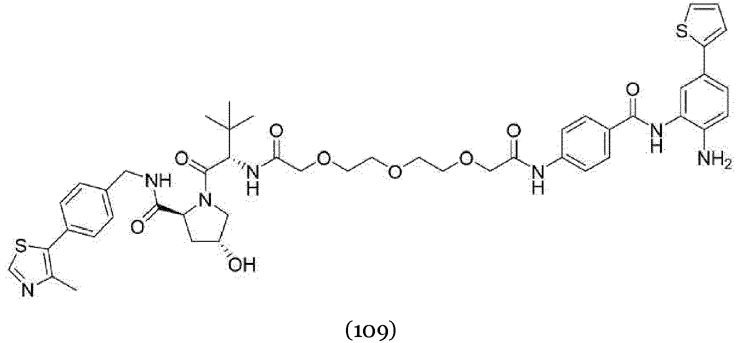

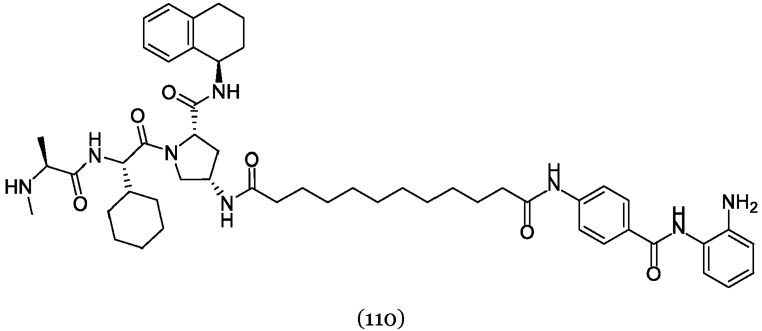

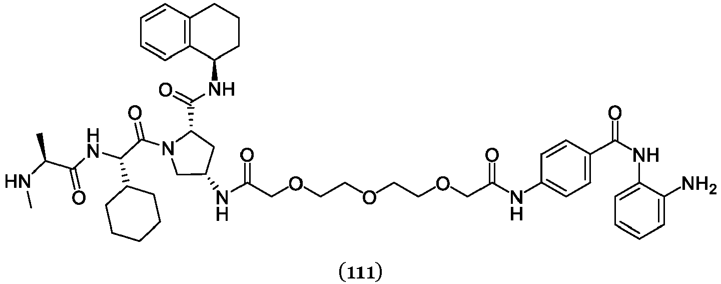

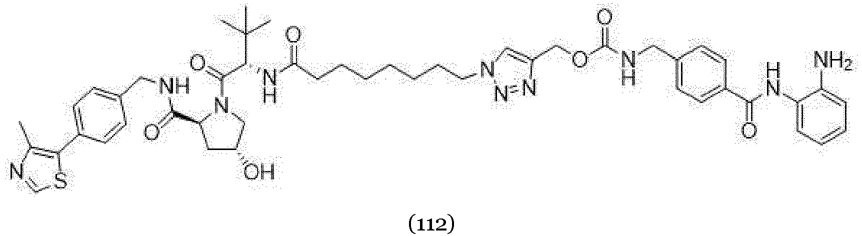

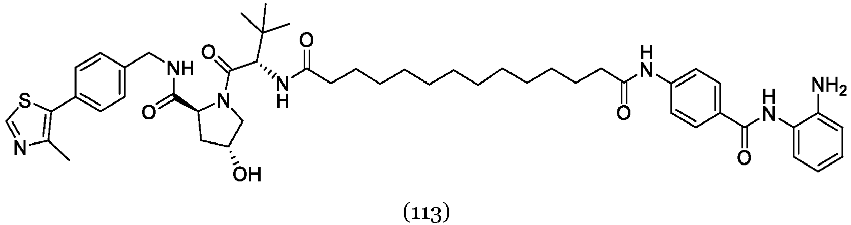

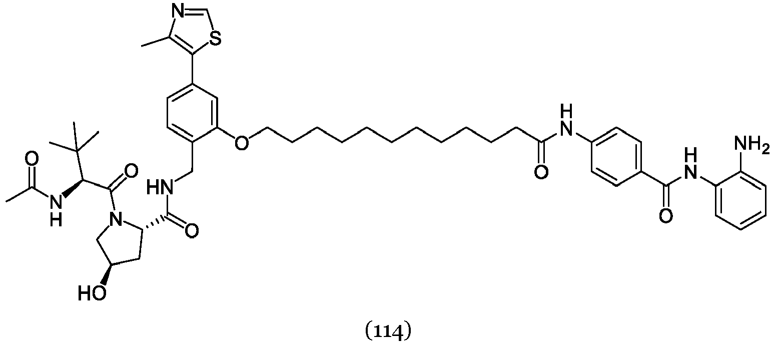

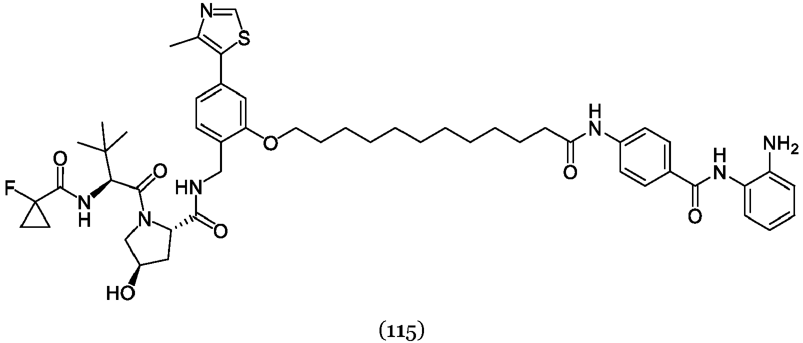

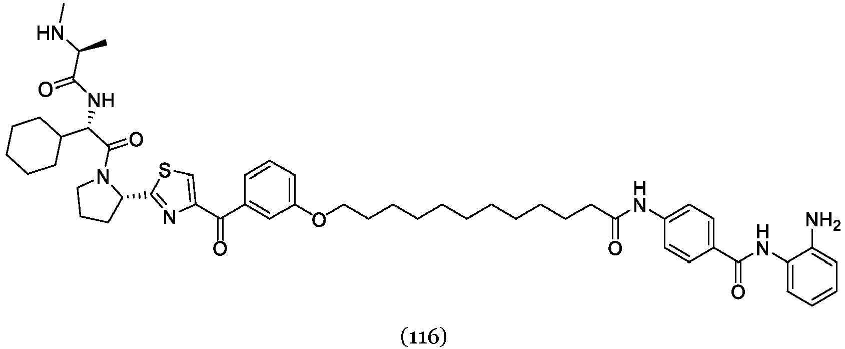

The term "pharmaceutically acceptable salt" may be understood to refer to any salt of a compound provided herein which retains its biological properties and which is not toxic or otherwise undesirable for pharmaceutical use. Such salts may be derived from a variety of organic and inorganic counter-ions well known in the art. Such salts include, but are not limited to: (1) acid addition salts formed with organic or inorganic acids such as hydrochloric, hydrobromic, sulfuric, nitric, phosphoric, sulfamic, acetic, adepic, aspartic, trifluoroacetic, trichloroacetic, propionic, hexanoic, cyclopentylpropionic, glycolic, glutaric, pyruvic, lactic, malonic, succinic, sorbic, ascorbic, malic, maleic, fumaric, tartaric, citric, benzoic, 3-(4-hydroxybenzoyl)benzoic, picric, cinnamic, mandelic, phthalic, lauric, methanesulfonic, ethanesulfonic, 1,2-ethane-disulfonic, 2-hydroxyethanesulfonic, benzenesulfonic, 4-chlorobenzenesulfonic, 2-naphthalenesulfonic, 4-toluenesulfonic, camphoric, camphorsulfonic, 4-methylbicyclo[2.2.2]-oct-2-ene-1-carboxylic, glucoheptonic, 3-phenylpropionic, trimethylacetic, tert-butylacetic, lauryl sulfuric, gluconic, benzoic, glutamic, hydroxynaphthoic, salicylic, stearic, cyclohexylsulfamic, quinic, muconic acid and the like acids; or (2) base addition salts formed when an acidic proton present in the parent compound either (a) is replaced by a metal ion, e.g., an alkali metal ion, an alkaline earth ion or an aluminium ion, or alkali metal or alkaline earth metal hydroxides, such as sodium, potassium, calcium, magnesium, aluminium, lithium, zinc, and barium hydroxide, ammonia or (b) coordinates with an organic base, such as aliphatic, alicyclic, or aromatic organic amines, such as ammonia, methylamine, dimethylamine, diethylamine, picoline, ethanolamine, diethanolamine, triethanolamine, ethylenediamine, lysine, arginine, ornithine, choline, N,N'-dibenzylethylene-diamine, chloroprocaine, diethanolamine, procaine, N-benzylphenethylamine, N-methylglucamine piperazine, tris(hydroxymethyl)-aminomethane, tetramethylammonium hydroxide, and the like.

Pharmaceutically acceptable salts may include, sodium, potassium, calcium, magnesium, ammonium, tetraalkylammonium and the like, and when the compound contains a basic functionality, salts of non-toxic organic or inorganic acids, such as hydrohalides, e.g. hydrochloride, hydrobromide and hydroiodide, carbonate or bicarbonate, sulfate or bisulfate, borate, phosphate, hydrogen phosphate, dihydrogen phosphate, pyroglutamate, saccharate, stearate, sulfamate, nitrate, orotate, oxalate, palmitate, pamoate, acetate, trifluoroacetate, trichloroacetate, propionate, hexanoate, cyclopentylpropionate, glycolate, glutarate, pyruvate, lactate, malonate, succinate, tannate, tartrate, tosylate, sorbate, ascorbate, malate, maleate, fumarate, tartarate, camsylate, citrate, cyclamate, benzoate, isethionate, esylate, formate, 3-(4-hydroxybenzoyl)benzoate, picrate, cinnamate, mandelate, phthalate, laurate, methanesulfonate (mesylate), methylsulphate, naphthylate, 2-napsylate, nicotinate, ethanesulfonate, 1,2-ethane-disulfonate, 2-hydroxyethanesulfonate, benzenesulfonate (besylate), 4-chlorobenzenesulfonate, 2-naphthalenesulfonate, 4-toluenesulfonate, camphorate, camphorsulfonate, 4-methylbicyclo[2.2.2]-oct-2-ene-1-carboxylate, glucoheptonate, 3-phenylpropionate, trimethylacetate, tert-butylacetate, lauryl sulfate, gluceptate, gluconate, glucoronate, hexafluorophosphate, hibenzate, benzoate, glutamate, hydroxynaphthoate, salicylate, stearate, cyclohexylsulfamate, quinate, muconate, xinofoate and the like.

Hemisalts of acids and bases may also be formed, for example, hemisulphate salts.

The term "solvate" may be understood to refer to a compound provided herein or a salt thereof, that further includes a stoichiometric or non-stoichiometric amount of solvent bound by non-covalent intermolecular forces. Where the solvent is water, the solvate is a hydrate.

In a second aspect, there is provided a compound of formula (I), as defined in the first aspect, or a pharmaceutically acceptable complex, salt, solvate, tautomeric form or polymorphic form thereof for use in therapy.

In a third aspect, there is provided a compound of formula (I), as defined in the first aspect, or a pharmaceutically acceptable complex, salt, solvate, tautomeric form or polymorphic form thereof for use in treating cancer.

In a fourth aspect, there is provided a pharmaceutical composition, the composition comprising a compound of formula (I), as defined in the first aspect, or a pharmaceutically acceptable complex, salt, solvate, tautomeric form or polymorphic form thereof and a pharmaceutically acceptable vehicle.

This disclosure also provides a method of treating, preventing or ameliorating cancer in a subject, the method comprising administering to a subject in need of such treatment, a therapeutically effective amount of a compound of formula (I), as defined in the first aspect, or a pharmaceutically acceptable complex, salt, solvate, tautomeric form or polymorphic form thereof. It will be appreciated that a method of treatment is not included within the scope of the claims.

The cancer may be a solid tumour or solid cancer. The cancer may be blood cancer, bowel cancer, brain cancer, breast cancer, cervical cancer, endometrial cancer, gastric cancer, liver cancer, lung cancer, ovarian cancer, pancreatic cancer, prostate cancer or skin cancer. The blood cancer may be myeloma, leukaemia or lymphoma. Lymphoma may be cutaneous T-cell lymphoma (CTCL). The bowel cancer may be colon cancer or rectal cancer. The brain cancer may be a glioma or a glioblastoma. The breast cancer may be a BRCA positive breast cancer. The breast cancer may be a HER2 positive breast cancer or HER2 negative breast cancer. The breast cancer may be triple negative breast cancer. The liver cancer may be hepatocellular carcinoma. The lung cancer may be non-small cell lung cancer or small cell lung cancer. The skin cancer may be a melanoma.

It will be appreciated that the compound of formula (I) described herein, or a pharmaceutically acceptable salt or solvate thereof, may be used in a medicament which may be used in a monotherapy (i.e. use of the inhibitor alone), for treating, ameliorating, or preventing cancer. Alternatively, the inhibitor or a pharmaceutically acceptable salt or solvate thereof may be used as an adjunct to, or in combination with, known therapies for treating, ameliorating, or preventing cancer.

The compound of formula (I) may be combined in compositions having a number of different forms depending, in particular, on the manner in which the composition is to be used. Thus, for example, the composition may be in the form of a powder, tablet, capsule, liquid, ointment, cream, gel, hydrogel, aerosol, spray, micellar solution, transdermal patch, liposome suspension or any other suitable form that may be administered to a person or animal in need of treatment. It will be appreciated that the vehicle of medicaments according to the invention should be one which is well-tolerated by the subject to whom it is given.

Medicaments comprising the compound of formula (I) may be used in a number of ways. Compositions comprising the compound of formula (I) may be administered by inhalation (e.g. intranasally). Compositions may also be formulated for topical use. For instance, creams or ointments may be applied to the skin.

The compound of formula (I) may also be incorporated within a slow- or delayed-release device. Such devices may, for example, be inserted on or under the skin, and the medicament may be released over weeks or even months. The device may be located at least adjacent the treatment site. Such devices may be particularly advantageous when long-term treatment with the inhibitor used according to the invention is required and which would normally require frequent administration (e.g. at least daily injection).

The compound of formula (I) and compositions comprising the compound may be administered to a subject by injection into the blood stream or directly into a site requiring treatment, for example into a cancerous tumour or into the blood stream adjacent thereto. Injections may be intravenous (bolus or infusion) or subcutaneous (bolus or infusion), intradermal (bolus or infusion) or intramuscular (bolus or infusion).

In a preferred embodiment, the compound of formula (I) is administered orally. Accordingly, the compound of formula (I) may be contained within a composition that may, for example, be ingested orally in the form of a tablet, capsule or liquid.

It will be appreciated that the amount of the compound of formula (I) that is required is determined by its biological activity and bioavailability, which in turn depends on the mode of administration, the physiochemical properties of the compound of formula (I), and whether it is being used as a monotherapy, or in a combined therapy. The frequency of administration will also be influenced by the half-life of the compound of formula (I) within the subject being treated. Optimal dosages to be administered may be determined by those skilled in the art, and will vary with the particular inhibitor in use, the strength of the pharmaceutical composition, the mode of administration, and the advancement of the cancer. Additional factors depending on the particular subject being treated will result in a need to adjust dosages, including subject age, weight, sex, diet, and time of administration.

The compound of formula (I) may be administered before, during or after onset of the cancer to be treated. Daily doses may be given as a single administration. Alternatively, the compound of formula (I) is given two or more times during a day, and may be given twice a day.

Generally, a daily dose of between 0.01µg/kg of body weight and 500mg/kg of body weight of the compound of formula (I) may be used for treating, ameliorating, or preventing cancer. More preferably, the daily dose is between o.oimg/kg of body weight and 400mg/kg of body weight, more preferably between 0.1mg/kg and 200mg/kg body weight, and most preferably between approximately 1mg/kg and 100mg/kg body weight.

A patient receiving treatment may take a first dose upon waking and then a second dose in the evening (if on a two dose regime) or at 3- or 4-hourly intervals thereafter. Alternatively, a slow release device may be used to provide optimal doses of the inhibitor according to the invention to a patient without the need to administer repeated doses.

Known procedures, such as those conventionally employed by the pharmaceutical industry (e.g. in *vivo* experimentation, clinical trials, etc.), may be used to form specific formulations comprising the inhibitor according to the invention and precise therapeutic regimes (such as daily doses of the inhibitor and the frequency of administration). The inventors believe that they are the first to describe a pharmaceutical composition for treating cancer based on the use of the compound of formula (I).

There is provided a pharmaceutical composition for treating cancer in a subject, the composition comprising a compound of formula (I), as defined in the first aspect, or a pharmaceutically acceptable complex, salt, solvate, tautomeric form or polymorphic form thereof and a pharmaceutically acceptable vehicle.

The pharmaceutical composition can be used in the therapeutic amelioration, prevention or treatment in a subject of cancer.

The pharmaceutical composition may further comprise a known therapy for treating, ameliorating, or preventing cancer.

There is also provided a process for making the composition for treating cancer in a subject, the process comprising contacting a therapeutically effective amount of a compound of formula (I), or a pharmaceutically acceptable complex, salt, solvate, tautomeric form or polymorphic form thereof, and a pharmaceutically acceptable vehicle.

A "subject" may be a vertebrate, mammal, or domestic animal. Hence, the compound of formula (I), compositions and medicaments according to the invention may be used to treat any mammal, for example livestock (e.g. a horse), pets, or may be used in other veterinary applications. Most preferably, however, the subject is a human being.

A "therapeutically effective amount" of the compound of formula (I) is any amount which, when administered to a subject, is the amount of drug that is needed to treat the cancer.

For example, the therapeutically effective amount of the compound of formula (I) used may be from about 0.01 mg to about 800 mg, and preferably from about 0.01 mg to about 500 mg. It is preferred that the amount of the compound of formula (I) is an amount from about 0.1 mg to about 250 mg, and most preferably from about 0.1 mg to about 20 mg.

A "pharmaceutically acceptable vehicle" as referred to herein, is any known compound or combination of known compounds that are known to those skilled in the art to be useful in formulating pharmaceutical compositions.

In one embodiment, the pharmaceutically acceptable vehicle may be a solid, and the composition may be in the form of a powder or tablet. A solid pharmaceutically acceptable vehicle may include one or more substances which may also act as flavouring agents, lubricants, solubilisers, suspending agents, dyes, fillers, glidants, compression aids, inert binders, sweeteners, preservatives, dyes, coatings, or tablet-disintegrating agents. The vehicle may also be an encapsulating material. In powders, the vehicle is a finely divided solid that is in admixture with the finely divided active agents (i.e. the inhibitor) according to the invention. In tablets, the inhibitor may be mixed with a vehicle having the necessary compression properties in suitable proportions and compacted in the shape and size desired. The powders and tablets preferably contain up to 99% of the inhibitor. Suitable solid vehicles include, for example calcium phosphate, magnesium stearate, talc, sugars, lactose, dextrin, starch, gelatin, cellulose, polyvinylpyrrolidine, low melting waxes and ion exchange resins. In another embodiment, the pharmaceutical vehicle may be a gel and the composition may be in the form of a cream or the like.

However, the pharmaceutical vehicle may be a liquid, and the pharmaceutical composition is in the form of a solution. Liquid vehicles are used in preparing solutions, suspensions, emulsions, syrups, elixirs and pressurized compositions. The compound of formula (I) may be dissolved or suspended in a pharmaceutically acceptable liquid vehicle such as water, an organic solvent, a mixture of both or pharmaceutically acceptable oils or fats. The liquid vehicle can contain other suitable pharmaceutical additives such as solubilisers, emulsifiers, buffers, preservatives, sweeteners, flavouring agents, suspending agents, thickening agents, colours, viscosity regulators, stabilizers or osmo-regulators. Suitable examples of liquid vehicles for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the vehicle can also be an oily ester such as ethyl oleate and isopropyl myristate. Sterile liquid vehicles are useful in sterile liquid form compositions for parenteral administration. The liquid vehicle for pressurized compositions can be a halogenated hydrocarbon or other pharmaceutically acceptable propellant.

Liquid pharmaceutical compositions, which are sterile solutions or suspensions, can be utilized by, for example, intramuscular, intrathecal, epidural, intraperitoneal, intravenous and particularly subcutaneous injection. The compound of formula (I) may be prepared as a sterile solid composition that may be dissolved or suspended at the time of administration using sterile water, saline, or other appropriate sterile injectable medium.

The compound of formula (I) and compositions of the invention may be administered in the form of a sterile solution or suspension containing other solutes or suspending agents (for example, enough saline or glucose to make the solution isotonic), bile salts, acacia, gelatin, sorbitan monoleate, polysorbate 80 (oleate esters of sorbitol and its anhydrides copolymerized with ethylene oxide) and the like. The compound of formula (I) used according to the invention can also be administered orally either in liquid or solid composition form. Compositions suitable for oral administration include solid forms, such as pills, capsules, granules, tablets, and powders, and liquid forms, such as solutions, syrups, elixirs, and suspensions. Forms useful for parenteral administration include sterile solutions, emulsions, and suspensions.

All features described herein (including any accompanying claims, abstract and drawings), and/or all of the steps of any method or process so disclosed, may be combined with any of the above aspects in any combination, except combinations where at least some of such features and/or steps are mutually exclusive.

For a better understanding of the invention, and to show how embodiments of the same may be carried into effect, reference will now be made, by way of example, to the accompanying drawings, in which:-
**Figure 1** shows benzamide based HDAC inhibitors. CI-994 demonstrates selective inhibition for HDAC 1, 2 & 3, inhibits cell proliferation, induces apoptosis and has anti-tumor activity. MS-275 is a HDAC1 & 3 selective inhibitor with anti-tumor activity currently in phase III clinical trials for breast cancer. MGCD0103 is a HDAC 1, 2, 3 and 11 inhibitor in clinical trials for solid tumors and non-small cell lung cancer;
**Figure 2A** is a schematic showing how when CI-994 is located in the binding pocket of HDAC2, the acetyl group of CI-994 is surface exposed from the HDAC2 catalytic active site (PDB: 4LY1); and **Figures 2B and 2C** show the structures of compounds synthesized by the inventors;
**Figure 3A** is a graph showing AMC-Fluorescence Histone Deacetylase inhibition assay with the LSD1-CoREST-HDAC complex; and **Figure 3B** shows Histone 3 Lysine 56 Acetylation (H3K56Ac) levels in E14 mouse embryonic stem cells after 24h; CI-994 = 40µM, Panobinostat = 30 nM;
**Figure 4A** shows an immunoblot with HDAC 1, 2 and 3 antibodies after 24h treatment with the indicated reagents in HCT116 colon cancer cell line. Numerical value represents percentage with respect to DMSO control =100%; **Figure 4B** shows Histone H3 Lysine 9 acetylation levels after 24 hours of the indicated treatments in HCT116 colon cancer cell line; and **Figure 4C** shows **NC-PROTAC4** with the inactive VHL diastereoisomer does not induce degradation in HCT116 cells. CI-994 = 40µM, Panobinostat = 30 nM;
**Figure 5** shows Fluorescence-Activated Cell Sorting (FACS) data of compound **4** and CI-994 in HCT116 colon cancer cell line;
**Figure 6** shows the structures of further compounds synthesized by the inventors;
**Figure 7** shows the effect of compound **2** on the levels of HDAC 1/2 in HCT116 cells following 24h treatment; and
**Figure 8** shows the effects of other representative compounds on HDAC ½ in HCT116 cells following 24h treatment.

### Example 1: Synthesis of four preliminary Proteolysis Targeting Chimeras (PROTACs)

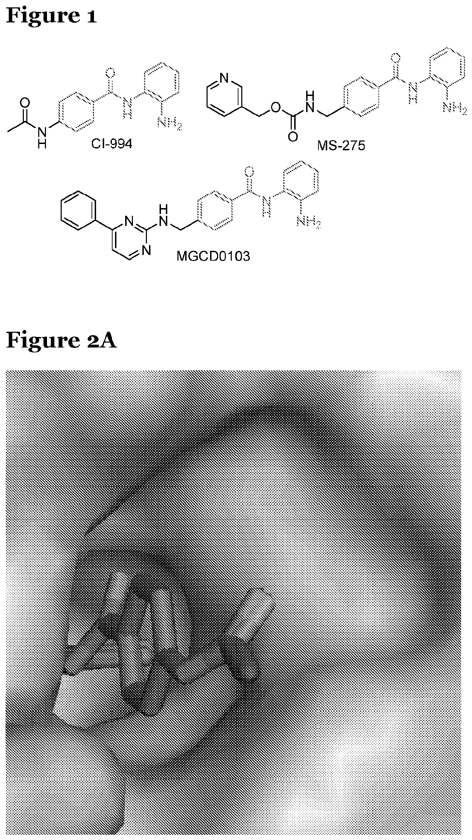

For their PROTAC design, the inventors chose Benzamide based HDAC inhibitors that demonstrate selectivity for HDAC1, 2 and 3. CI-994 (see Figure 1) has reported Ki values of 0.41 µM for HDAC1, 0.75 µM for HDAC3, and approximately 100 µM for HDAC8. This inhibitor exhibits phenotypes related to HDAC 1, 2 and 3, inhibiting cell proliferation, inducing apoptosis, and exhibiting broad antitumor activity in vitro and in vivo. CI-994 has been in clinical trials for its antitumor properties and analogous benzamides MS-275 and MGCD0103 (see Figure 1) are currently in phase III clinical trials for breast cancer and non-small cell lung cancer. More recently CI-994 has also been reported for its neuroprotective effects in mice following spinal cord injury, the treatment of cognitive disorders, and reducing atrial fibrillation.

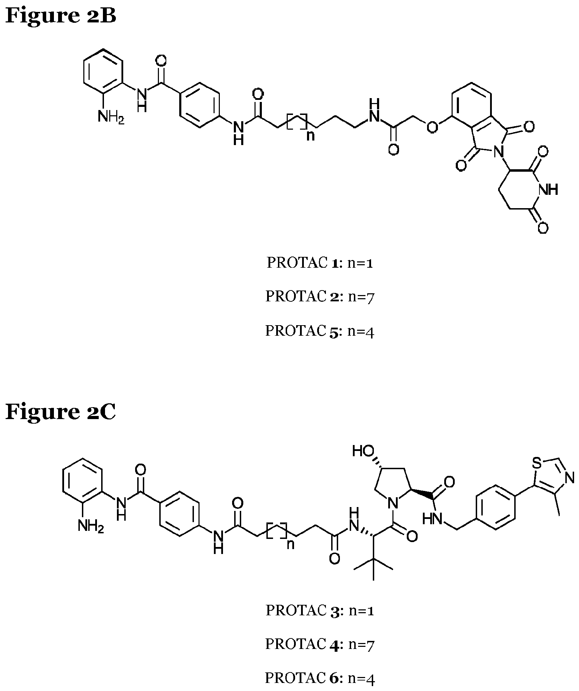

The inventors functionalized CI-994 from the acetyl group of the phenyl moiety (see **PROTACs 1-4** shown in Figure 2B and C) as the acetyl group of an analogous benzamide inhibitor protrudes from the catalytic active site and is surface exposed in HDAC2 (see Figure 2A). Accordingly, the inventors hypothesized that functionalization at this position may maintain HDAC binding. A short alkyl linker length was prepared (n=6) and a longer linker length prepared (n=12) as in previous PROTAC studies it has been shown the linker length can play an essential role in inducing degradation. Alkyl linkers were chosen to hasten synthesis. Two varying E3 ligands where also chosen; the von Hippel-Lindau (VHL) ligand, and the cereblon ligand, as the choice of E3 ligand, as well as linker length, can also influence successful protein-protein engagement with E3 ligase, and hence degradation.

### Example 2: Evaluation of preliminary PROTACs in vitro

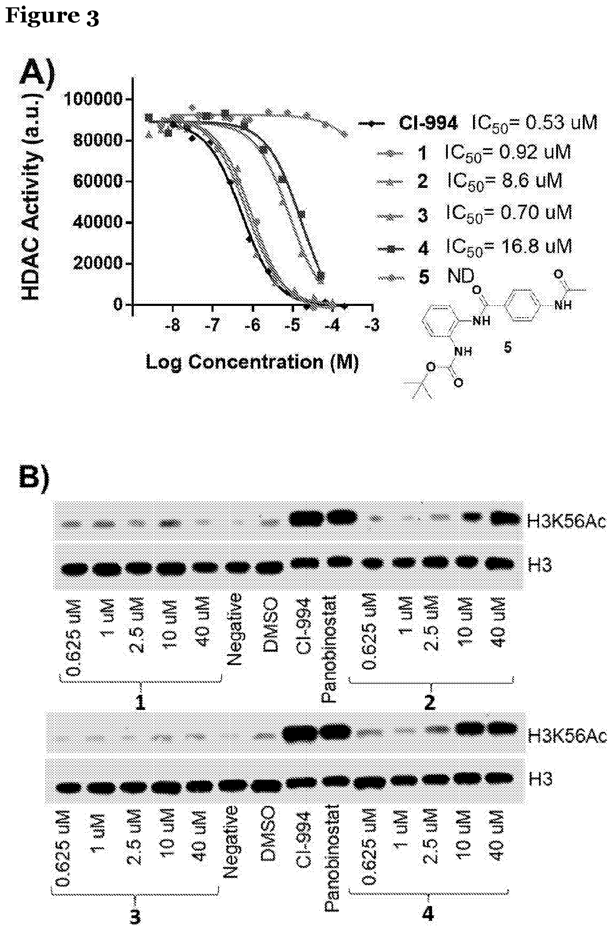

The inventors evaluated their preliminary PROTACs *in vitro* using an established fluorescent deacetylase assay with the LSD1-CoREST-HDAC1 complex. This complex was used as an exemplary HDAC multiprotein complex to determine if such heterobifunctional molecules **PROTACs 1-4** can still engage the HDAC enzyme in the context of an intact multiprotein complex. The IC₅₀ values of **PROTACs 1-4** were determined side by side with CI-994 as a positive control; as a negative control the inventors also synthesized Boc protected CI-994, compound **5** (see Figure 3). Compound **5** is not capable of chelating zinc in the HDAC active site and should not demonstrate HDAC inhibition.

### Discussion

The inventors observed that CI-994 had an IC₅₀ value of approx. 0.5 µM consistent with the literature and **5** demonstrated no HDAC inhibition (see Figure 3A). Preliminary **PROTACs 1** and **3** with the shorter linker lengths all engaged HDAC1 in the CoREST complex with IC₅₀ values directly comparable to CI-994, while the longer linker lengths, **PROTACs 2** and **4**, still maintained inhibition but had an approx. 10-15 fold inhibitory reduction compared to **PROTACs 1** and **3**.

### Example 3: Evaluation of preliminary PROTACs in cells

The inventors proceeded to assess their compounds effects on HDAC activity in cells. In a previous study, the inventors demonstrated that acetylated histone H3 Lys56 (H3K56ac) is a direct substrate of HDAC1 in embryonic stem (ES) cells.

### Discussion

To examine the ability of **PROTACs 1-4** to reduce HDAC1, 2 and 3 activity in cells they began by measuring H3K56ac using quantitative western blotting (see Figure 3B). CI-994 and the pan-HDAC inhibitor Panobinostat were used as positive controls. Acetylation levels of H3K56 where increased with Panobinostat and CI-994 as expected, intriguingly, only the PROTACs with longer linker lengths, i.e. **PROTACs 2** and **4**, caused an increase in acetylation to similar levels (see Figure 3B).

The inventors speculate that **PROTACs 1** and **3** although active *in vitro,* may be unable to reach their cellular target for HDAC inhibition or degradation.

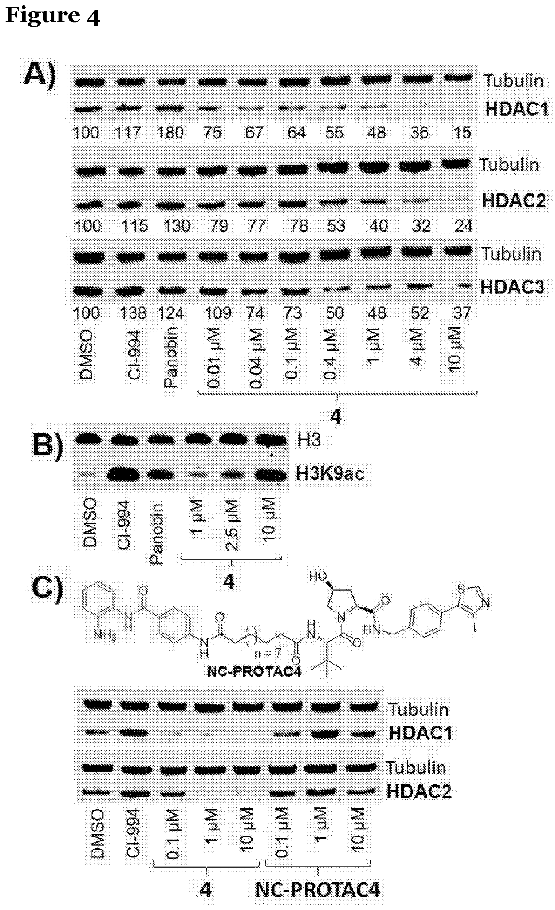

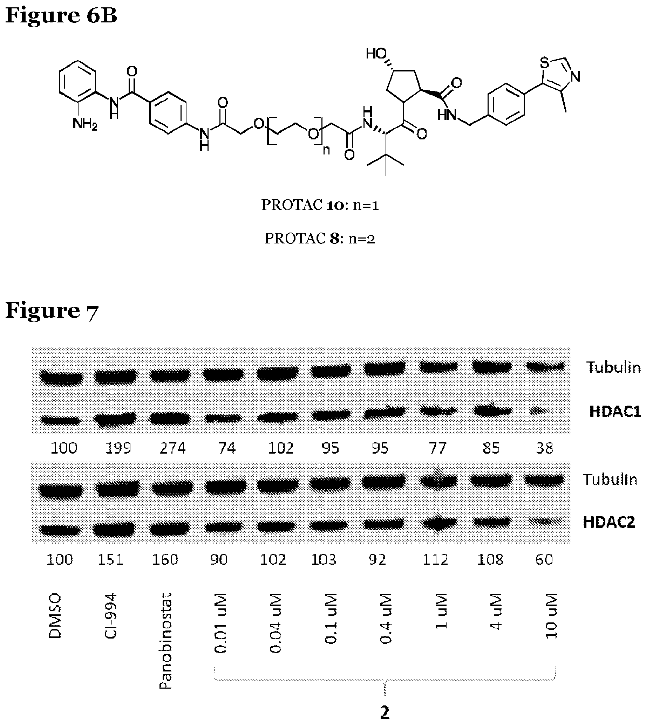

With conformation that **PROTACs 2** and **4** decrease Histone Deacetylation activity in *vitro* and *in cells,* the inventors proceeded to quantify HDAC1, 2 and 3 protein abundance. In ES cells partial degradation was observed (data not shown) however degradation was even more prominent in human colon cancer cell line HCT116. After 24 hours degradation was observed in a dose dependent manner (see Figure 4A). The VHL based **PROTAC 4** was a more effective degrader than the Cereblon **PROTAC 2**, see Figure 7. HDAC1 and 2 underwent near complete degradation at 10 µM with **PROTAC 4**, while HDAC3 levels were also decreased. At 1 µM approximately 50% degradation was observed for HDAC 1, 2 and 3. Intriguingly, even at a 10 nM of **PROTAC 4** complete HDAC 1 and 2 levels were not recovered.

Acetylation levels of Histone H3 Lys9 (H3K9ac), another established residue for HDAC activity, were also determined in HCT116 cells after 24 hours treatment with **PROTAC 4**. At 10µM of **PROTAC 4** acetylation levels were highly elevated compared to the DMSO control (Figure 4B), consistent with a reduction in HDAC1 and 2 levels at 10 µM (Figure 4A). However, at 1µM with HDAC1, 2 & 3 at approximately 40-48% levels H3K9 acetylation levels were similar to the DMSO control. Indicating for significantly increased acetylation, in H3K9ac at least, near complete HDAC degradation is required and partial degradation is not sufficient. To confirm HDAC degradation was occurring via a VHL mediated proteasome degradation pathway the inventors synthesised NC-**PROTAC4** with the inactive diastereoisomer of VHL (Figure 4C). No degradation of HDAC 1 & 2 was observed when compared side by side with **PROTAC 4** confirming degradation is occurring via a VHL mediated E3 ligase pathway.

### Example 4: Evaluation of the effects of PROTAC 4 on the cell cycle in the colon cancer HCT116 cell line using Fluorescence-Activated Cell Sorting (FACs)

The inventors then investigated the effect of **PROTAC 4** on the cell cycle in the colon cancer HCT116 cell line.

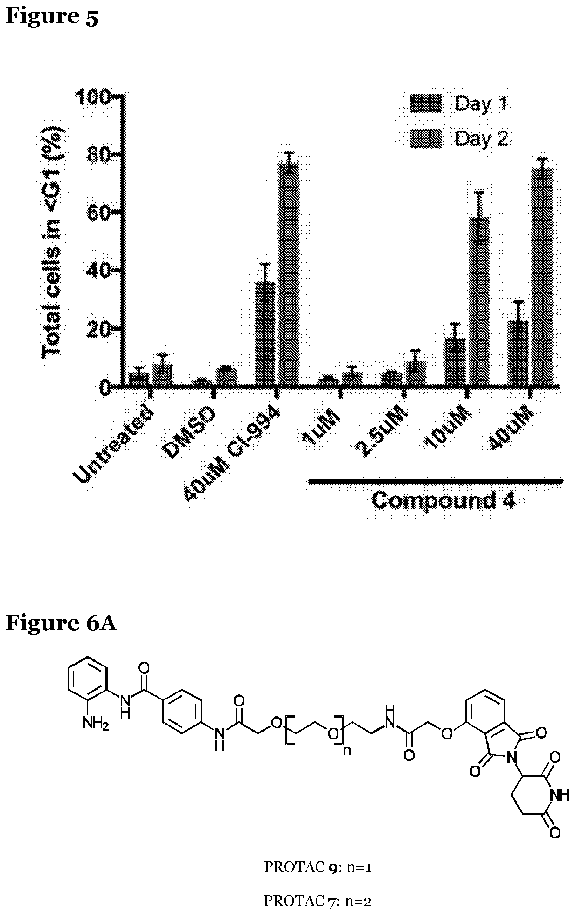

After 48 hours significant cell death was observed at 10µM of **PROTAC 4** (Figure 5). At 40µM cell death after 48 hours was at similar levels to CI-994. Although the effect of **PROTAC 4** on cell viability was comparable to CI-994 it's important to note the IC₅₀ of **PROTAC 4** against the LSD1-CoREST-HDAC1 complex *in vitro* is 16.8 µM, compared to 0.5 µM for CI-994, with near complete HDAC1 and 2 degradation being observed with **PROTAC 4** at 10 µM. Hence, the observed phenotype with **PROTAC 4** is most likely due to degradation, rather than inhibition, of the CoREST complex and other HDAC1 & 2 containing complexes in the cell.

### Example 5: Synthesis of further PROTACs

The inventors further synthesized PROTACs 5-10 (see Figures 2B, 2C 6A, and 6B).

### Example 6: Synthesis and evaluation of still further PROTACs

The inventors further synthesized the following PROTACs.

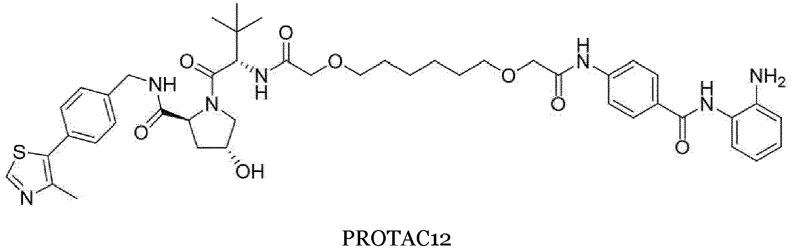

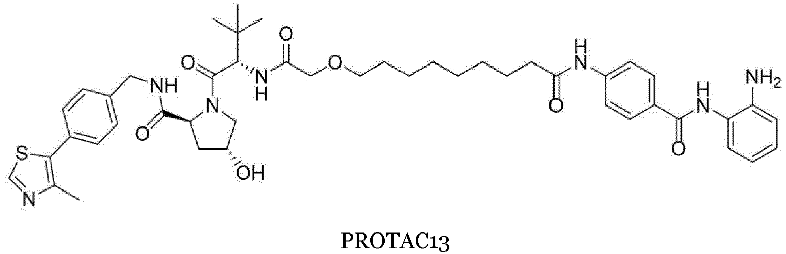

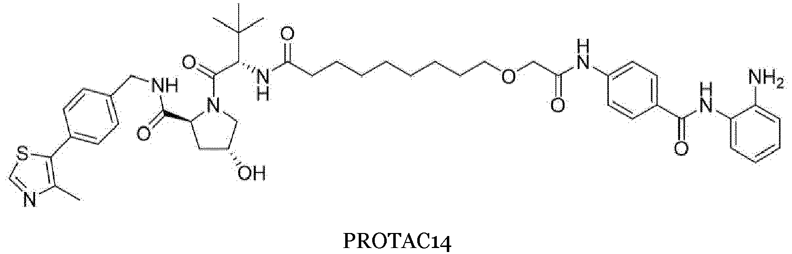

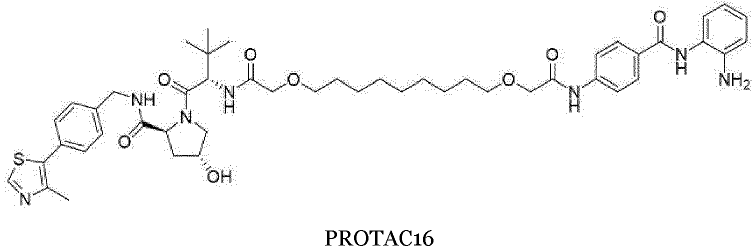

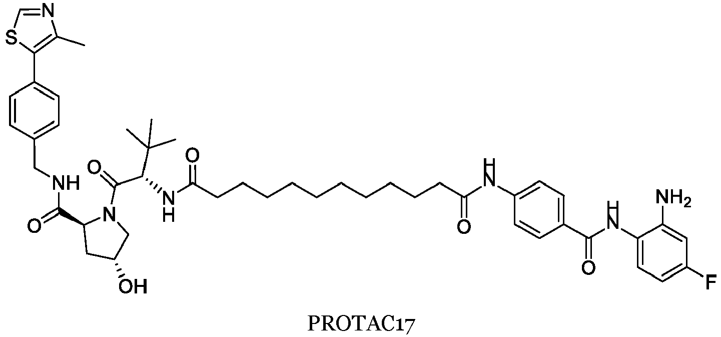

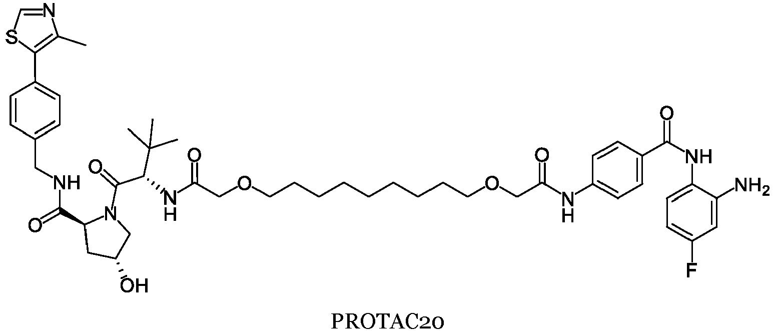

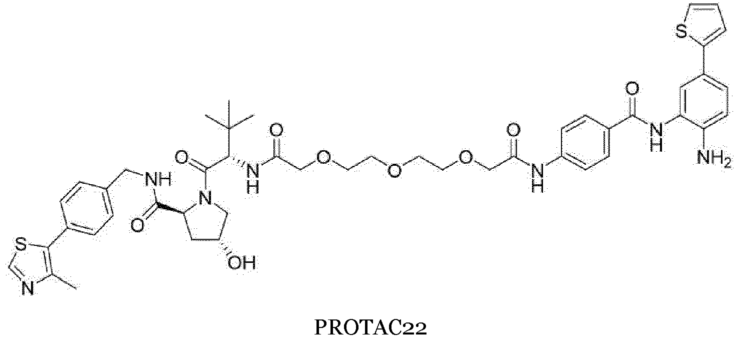

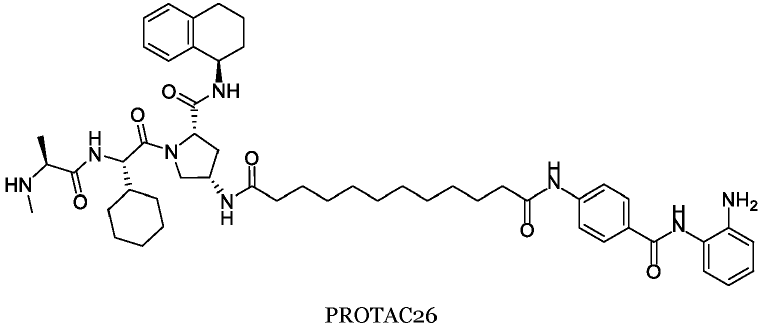

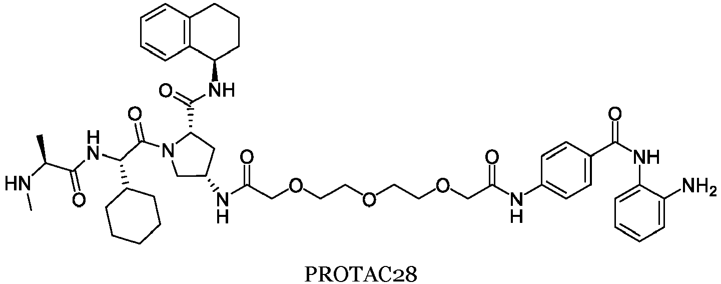

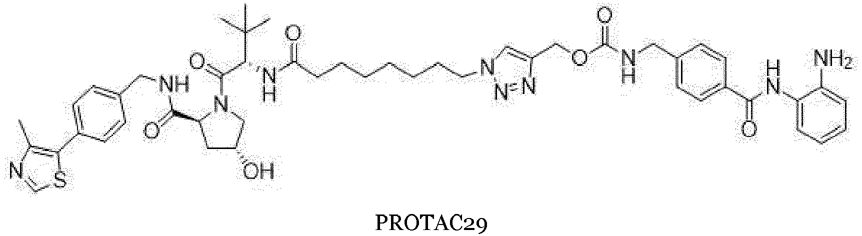

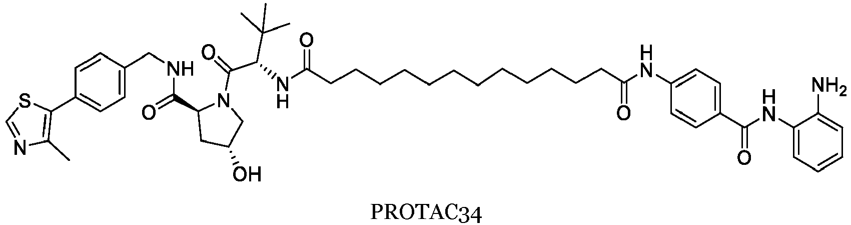

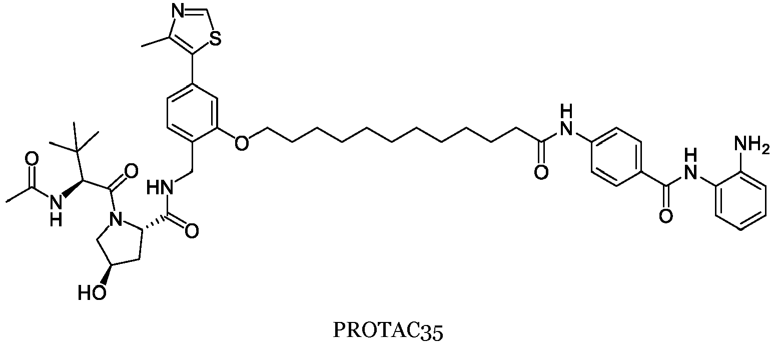

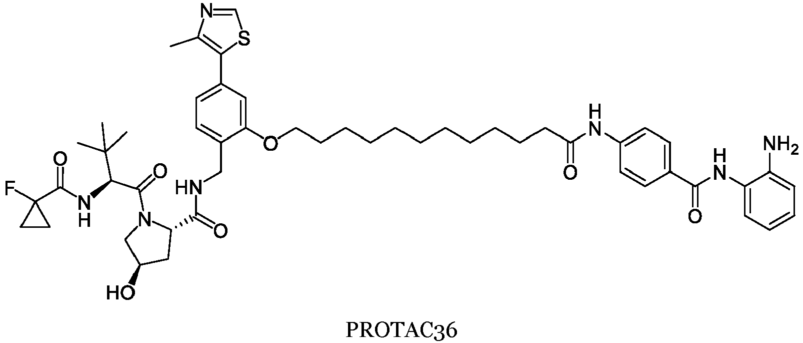

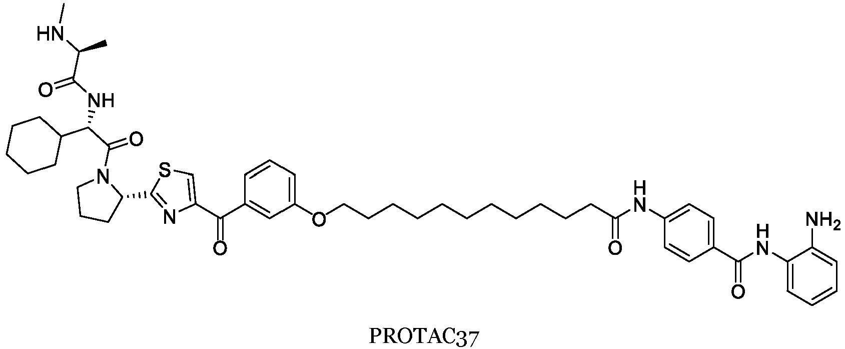

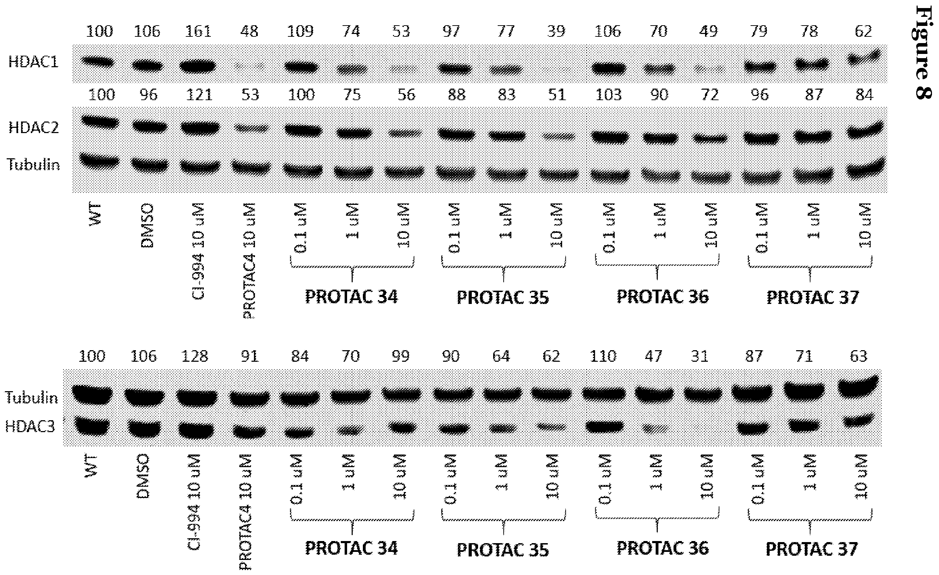

The compounds were screened at 0.1 µM, 1 µM and 10 µM in a HCT116 cell line. The maximum degradation observed irrespective of the concertation was recorded. The screening of the new compounds was done in 6-well plates, 5mL media, with 400,000 cells per well to increase throughput. Figure 8 shows representative compounds.

The activity of the compounds was analyzed and compared to PROTAC4 and the results are provided in the table below. CI-994 and BocCI-994 are provided as positive and negative controls, respectively, for CoREST, NuRD, MiDAC and SMRT assays.

It will be noted that the IC₅₀ value for PROTAC4 differs from the one in Figure 3 due to the assay having been run under different conditions.

**Table 1: Biological activity of PROTACs**

| **Compound Name** | CoREST IC₅₀ (µM) | NuRD IC₅₀ (µM) | MiDAC IC₅₀ (µM) | SMRT IC₅₀ (µM) | HDAC1 Degrader (%Degradation) |
|---|---|---|---|---|---|
| **CI-994** | 1.79 | 1.37 | 9.69 | 0.423 | N/A |
| **BocCI-994** | ND | ND | ND | | N/A |
| **PROTAC4** | 0.394 | 0.418 | 0.336 | 0.414 | 85% |
| **PROTAC6** | 0.244 | 0.338 | 1.69 | | 60% |
| **PROTAC7** | 3.7 | 3.24 | 17.23 | | 28% |
| **PROTAC12** | 7.17 | 8.13 | 5.15 | | 42% |
| **PROTAC13** | 0.845 | 1.18 | 1.43 | 0.345 | 58% |
| **PROTAC14** | 0.993 | 1.15 | 1.39 | 0.406 | 34% |
| **PROTAC16** | 4.13 | 11.8 | | | 67% |
| **PROTAC22** | | | | | 32% |
| **PROTAC17** | 3.81 | | | 0.86 | 58% |
| **PROTAC20** | | | | | 51% |
| **PROTAC26** | | | | | 88% |
| **PROTAC28** | | | | | No |
| **PROTAC29** | | | | | 39% |
| **PROTAC34** | | | | | 47% |
| **PROTAC35** | | | | | 61% |
| **PROTAC36** | | | | | 51% |
| **PROTAC37** | | | | | 38% |

| **Compound Name** | HDAC 2 Degrader (%Degradation) | HDAC 3 degrader (%Degradation) | Increase H3K56Ac (Y/N/partial) |
|---|---|---|---|
| **CI-994** | N/A | N/A | Y |
| **BocCI-994** | N/A | N/A | |
| **PROTAC4** | 76% | 63% | Y |
| **PROTAC6** | partial | not tested | Y |
| **PROTAC7** | No | 43% | Y |
| **PROTAC12** | 46% | 32% | Y |
| **PROTAC13** | 52% | 69% | Y |
| **PROTAC14** | 39% | 83% | Y |
| **PROTAC16** | 58% | 67% | Y |
| **PROTAC22** | No | 32% | Y |
| **PROTAC17** | 21% | 46% | partial |
| **PROTAC20** | 26% | 67% | partial |
| **PROTAC26** | 58% | 88% | Y |
| **PROTAC28** | No | 57% | partial |
| **PROTAC29** | 23% | 64% | |
| **PROTAC34** | 44% | 30% | |
| **PROTAC35** | 49% | 38% | |
| **PROTAC36** | 28% | 69% | |
| **PROTAC37** | 16% | 37% | |

### Discussion

The inventors have synthesized a number of compounds with IC₅₀ values comparable to CI-994 in the CoREST, NuRD, MiDAC and SMRT complexes. Furthermore, when tested in cells, the compounds were also found to be HDAC degraders.

Without wishing to be bound by theory, the inventors note that where low degradation is observed, it could indicate that a PROTAC targets a specific complex. For instance, the thiophene group in the head group of PROTAC22 is thought to make the PROTAC selective for CoREST. Accordingly, the relatively low %HDAC degradation which is observed for this compound could be due to this selectivity.

### Conclusions

The PROTAC approach has been applied to a number of protein targets, yet, importantly, not all proteins are as amenable to degradation. This has been observed with PROTACs based on non-selective pan-kinase inhibitors. Non-complex forming, cytoplasmic localized HDAC6 has also recently been identified as a preferential target for degradation with hydroxamic acid based PROTACs. The inventors have identified degraders of nucleus localized HDAC 1, 2 and 3, components of multiprotein corepressor complexes. Near complete HDAC 1, 2 & 3 degradation is required to significantly influence acetylation levels of certain HDAC markers in cells. However, it can be envisaged that with potential complex selective degraders global acetylation levels, and certain HDAC acetylation markers, may not be as increased in comparison to pan-HDAC inhibitors, yet may exhibit greater efficacy or reduced side effects to a HDAC corepressor complex related to a specific disease. Unexpectedly, the inventors found that the length of the alkyl linker was critical for cell permeability. Compounds 1 and 3 despite being a lower molecular weight and better HDAC inhibitors in vitro than 2 and 4, failed to alter histone acetylation levels in cells. It seems likely that the length and chemical nature of PROTAC linkers will have a profound effect on their activity in cells. HDAC degraders, such as those identified above, may offer an alternative strategy to important HDAC corepressor complex chemical probes and therapeutics.

### Materials and Methods

### General Methods

Starting materials and solvents used were purchased from commercially available sources and used without further purification. Dried THF and DCM were dried using an Innovative Technology inc. PureSolv solvent purification system. All chemical names have been generated using ChemDraw Professional. Unless otherwise stated, all reactions were stirred and carried out under nitrogen. All reactions were observed using TLC which was run on aluminium-backed silica. Preparative column chromatography and flash column chromatography using a Biotage Isolera purification system was perfomed using silica gel 60 (230-400 mesh).

Analytical and semi-preparative HPLC were performed on a ThermoFisher Ultimate 3000 system with Chromeleon software on a Phenomenex Luna C18 column. Method 1, A= H₂O, B= CH₃CN, 5-100% B, 10 mL/min flow, 45 min gradient. Method 2, A= 0.1% TFA in H₂O, B= 0.1% TFA in CH₃CN, 5-100% B, 10 mL/min flow, 45 min gradient.

Nuclear magnetic resonance (NMR) spectra were acquired using a Bruker 500 (¹H, 500 MHz; ¹³C 125 MHz) or Bruker 400 (¹H, 400 MHz; ¹³C 100 MHz) instrument at ambient temperature using deuterated solvent as reference - CDCl₃ (*δ*_{H} = 7.26 ppm, *δ*_{C} = 77.00 ppm), DMSO-*d₆* (*δ*_{H} = 2.50 ppm, *δ*_{C} = 39.51 ppm), CD₃OD (*δ*_{H} = 3.31 ppm, *δ*_{C} = 49.15 ppm), or CD₃CN (*δ*_{H} = 1.94 ppm, *δ*_{C} =1.39, 118.69 ppm). ACDLabs software (Chemsketch and Spectrus Processor) was used for peak picking, integration and calculating coupling constants. High resolution mass spectra (HRMS) were recorded on a Water Aquity XEVO Q ToF machine and measured in m/z. The structures of all products were confirmed by analysis of the ¹H NMR spectra and mass spectra.

**General Method A.** DIPEA (1.5 equiv.) was added to a solution of substituted aniline (1 equiv.) in dry DCM (2.5 mL/mmol) at 0 °C, followed by the dropwise addition of 4-nitrobenzoyl chloride (1.1 equiv.) as a solution in dry DCM. The mixture was stirred at 0 °C for 30 minutes, then at room temperature overnight. The reaction mixture was diluted with DCM and then washed with sat. NaHCO₄, 1M HCl and sat. NaCl. The organic layer was then dried over Na₂SO₄, concentrated in *vacuo,* then purified accordingly to afford the desired compound.

**General Method B.** Triethylamine (3 equiv.) was added to a solution of 4-aminobenzamide starting material (1 equiv.) in dry THF (10 mL/mmol) at 0 °C, followed by the dropwise addition of acetyl chloride (1.2 equiv.). The mixture was stirred at 0 °C for 30 minutes, then at room temperature for 2 hours. The reaction mixture was concentrated *in vacuo* to give the corresponding crude, which was chromatographically purified to afford the desired compound.

**General Method C.** TFA (20 equiv.) was added to a stirring solution of Boc-protected HDAC inhibitor/inhibitor-linker (1 equiv.) in DCM (10 mL/mmol) and the resulting reaction mixture stirred at room temperature for 6 hours. The reaction mixture was concentrated *in vacuo,* dissolved in MeOH (0.1 M), agitated in MP-carbonate resin (3.02 mmol/g loading capacity) for 2-3 hours and then filtered. The filtrate was concentrated *in vacuo* to afford the desired HDAC inhibitor/inhibitor-linker.

**General Method D.** To a solution of dicarboxylic acid (1 equiv.) in 1,4-dioxane/DMF (1:1, 4 mL/mmol), was added benzyl bromide (1 equiv.), followed by the addition of NaHCO₃ (1 equiv.). The resulting suspension was heated at 90 °C overnight. The reaction mixture was left to cool to room temperature and then concentrated in *vacuo.* The crude residue was then suspended in EtOAc and washed with sat. NaCl and water. The organic phase was dried over MgSO₄, filtered and concentrated in *vacuo* to afford the corresponding crude, which was chromatographically purified to afford the desired compound.

**General Method E.** To a solution of acid linker (1.1-1.3 equiv) in dry DMF (10 mL/mmol) at 0 °C, DIPEA (3 equiv.) and HATU (1.3-1.5 equiv.) were added. The reaction mixture was stirred for 15 minutes, after which a solution of amine (1 equiv.) in DMF was added slowly and the resultant solution stirred at room temperature overnight. The reaction mixture was diluted in EtOAc, then washed with sat. NaHCO₃ and sat. NaCl. The organic layer was dried over MgSO₄, filtered and concentrated in *vacuo* to give the corresponding crude, which was chromatographically purified to afford the desired compound.

**General Method F.** To a solution of the benzyl ester protected HDACi-linker conjugate (1 equiv.) in THF, 10% Pd/C (10% wt) was added. The reaction flask was filled with nitrogen and evacuated 3 times using a Shlenk line, before a balloon of hydrogen was added and the resultant mixture stirred vigorously for 4-18 hours. The balloon of hydrogen was removed and the flask was flushed with nitrogen The reaction mixture was filtered through a glass microfiber filter paper, and the filtrate concentrated in *vacuo* to afford the desired compound.

**General Method G.** To a solution of HDACi-linker acid (1.2 equiv.) in dry DMF (1 mL) at 0 °C, DIPEA (3 equiv.) and HATU (1.3 equiv.) were added. The reaction mixture was stirred for 15 minutes, after which a solution of (4R)-3-Methyl-L-valyl-4-hydroxy-N-[[4-(4-methyl-5-thiazolyl)phenyl]methyl]-L-prolinamide hydrochloride **(VH_032 amine,** 0.08-0.10 mmol) in DMF (1 mL) was added slowly and the resultant solution stirred at room temperature for 16 hours. The reaction mixture was diluted in EtOAc (10 mL), then washed with sat. NaHCO₃ (2 × 5 mL) and sat. NaCl (2 × 5 mL). The organic layer was dried over MgSO₄, filtered, and concentrated *in vacuo* to give the corresponding crude, which was chromatographically purified to afford the desired compound.

**General Method H.** TFA (0.4 mL or 20 equiv.) was added to a stirring solution of Boc-protected PROTAC (1 equiv.) in DCM (2 mL) and the resulting reaction mixture stirred at room temperature for 4-6 hours. The reaction mixture was concentrated *in vacuo,* dissolved in MeOH (2 mL), agitated in MP-carbonate resin (3.02 mmol/g loading capacity) for 2-3 hours and then filtered. The filtrate was concentrated *in vacuo* and the resulting solid dissolved in MeCN:H₂O (1:1) and lyophilised to remove residual TFA impurities, affording the final PROTAC.

**General Method I.** A mixture of E3 ligand phenol (1 equiv.), alkyl bromide (1 equiv.) and K₂CO₃ (3 equiv.) in dry DMF (0.8 mL) was stirred at 70 °C overnight. The reaction was concentrated *in vacuo* to give the corresponding crude, which was chromatographically purified to afford the desired compound.

**General Method J.** To a solution of 2-((2-(2,6-dioxopiperidin-3-yl)-1,3-dioxoisoindolin-4-yl)oxy)acetic acid (**CRBN acid,** l equiv.) in dry DMF (2 mL) at 0 °C, DIPEA (3 equiv.) and HATU (1.2 equiv.) were added. The reaction mixture was stirred for 15 minutes, after which a solution of HDACi-linker amine (1 equiv.) in DMF (1 mL) was added slowly and the resultant solution stirred at room temperature for 18 hours. The reaction mixture was diluted in EtOAc (10 mL), then washed with sat. NaHCO₃ (2 × 10 mL) and sat. NaCl (2 × 10 mL). The organic layer was dried over MgSO₄, filtered and concentrated *in vacuo* to give the corresponding crude, which was chromatographically purified to afford the final PROTAC.

**General Method K.** To a solution of HDACi-linker acid (1.2 equiv.) in dry DMF (1 mL) at 0 °C, DIPEA (3 equiv.) and HATU (1.3 equiv.) were added. The reaction mixture was stirred for 15 minutes, after which a solution of tert-butyl ((S)-1-(((S)-2-((2S,4S)-4-amino-2-(((R)-1,2,3,4-tetrahydronaphthalen-1-yl)carbamoyl)pyrrolidin-1-yl)-1-cyclohexyl-2-oxoethyl)amino)-1-oxopropan-2-yl)(methyl)carbamate hydrochloride (A **410099.1 amine,** 0.04 mmol) in DMF (1 mL) was added slowly and the resultant solution stirred at room temperature for 16 hours. The reaction mixture was diluted in EtOAc (10 mL), then washed with sat. NaHCO₃ (2 × 5 mL) and sat. NaCl (2 × 5 mL). The organic layer was dried over MgSO₄, filtered, and concentrated in *vacuo* to give the corresponding crude, which was chromatographically purified to afford the desired compound.

### Synthesis of HDAC Inhibitor (HDACi)

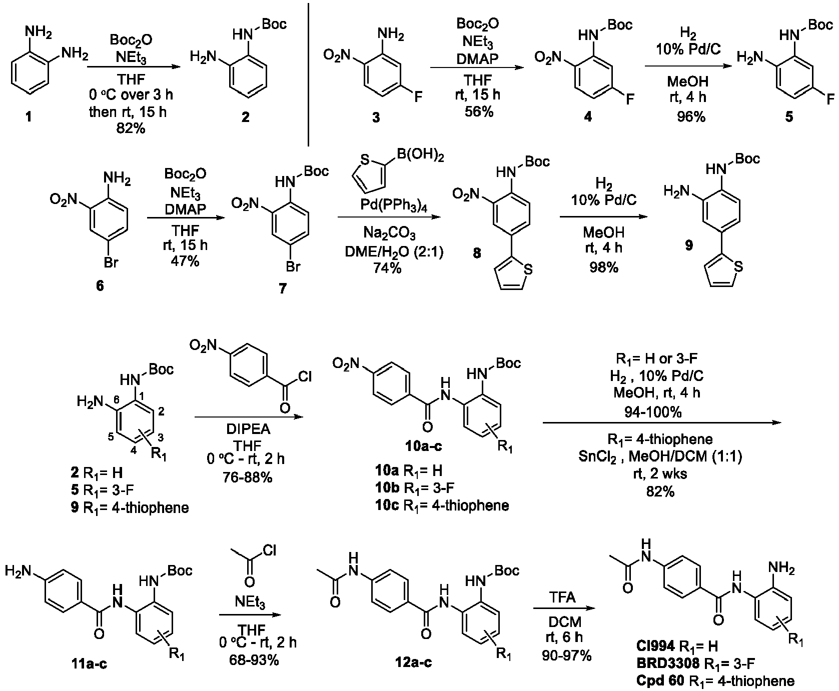

**Tert-butyl (2-aminophenyl)carbamate (2):** A solution of Boc₂O (6.05 g, 27.7 mmol) in THF (50 mL) was added dropwise over 3 hours to a solution of 7 (3.00 g, 27.7 mmol) and triethylamine (4.64 mL, 33.3 mmol) in THF (25 mL) at 0 °C, then the mixture was stirred at room temperature for 15 hours. The reaction was mixture concentrated *in vacuo* to afford a grey crystalline solid and then re-dissolved in EtOAc (50 mL). This solution was washed with water (2 × 30 mL) and sat. brine (2 × 30 mL), filtered over Na₂SO₄, then concentrated *in vacuo* to afford a yellow/grey solid. The crude solid was purified by column chromatography (solid load, 10-25% EtOAc in hexane) to afford 8 (4.72 g, 22.5 mmol, 82% yield) as a yellow/grey solid. HRMS (ESI) m/z: [M+Na]⁺ calculated for C₁₁H₁₆N₂O₂Na: 231.1109, found 231.1112.

**Tert-butyl (5-fluoro-2-nitrophenyl)carbamate (4):** A solution of **2** (4.61 g, 19.2 mmol) in THF (5 mL) was added dropwise to a solution of **3** (3.00 g, 19.2 mmol), DMAP (59 mg, 0.48 mmol) and triethylamine (1.54 mL, 11.06 mmol) in THF (35 mL) at 0°C, and then the mixture was stirred at 80 °C for 15 hours. The reaction mixture was concentrated *in vacuo,* then redissolved in EtOAc (100 mL) and 2M HCl (100 mL) added. The EtOAc layer was collected and then the aqueous layer extracted with further EtOAc (2 x 100 mL). The combined organic layers were dried over Na₂SO₄, filtered and concentrated in *vacuo* to afford an orange oil (5.10 g). The crude product was purified by column chromatography (10-20% EtOAc in hexane) to afford **4** (2.77 g, 10.7 mmol, 56% yield) as a yellow crystalline solid. HRMS (ESI, direct infusion) m/z: [M-H]-calculated for C₁₁H₁₂N₂O₄F: 255.0781, found 255.0780.

**Tert-butyl (2-amino-5-fluorophenyl)carbamate (5):** To a solution of **4** (1.68 g, 6.56 mmol) in MeOH (50 mL), 10% Pd/C (170 mg) was added. The reaction flask was filled with nitrogen and evacuated 3 times using a Shlenk line, before a balloon of hydrogen was added and the resultant mixture stirred vigorously for 4.5 hours. The balloon of hydrogen was removed and the flask was flushed with nitrogen. The reaction mixture was filtered through a glass microfiber filter and then the filtrate was concentrated in *vacuo* to afford **5** (1.43 g, 6.26 mmol, 96% yield) as a beige solid. MS (ESI) m/z: 227 [M+H]⁺.

**Tert-butyl (4-bromo-2-nitrophenyl)carbamate (7):** A solution of Boc₂O (2.21 g, 10.14 mmol) in THF (5 mL) was added dropwise to a solution of **6** (2.00 g, 9.22 mmol), DMAP (0.113 g, 0.92 mmol) and triethylamine (1.54 mL, 11.06 mmol) in THF (35 mL) at 0°C, and then the mixture was stirred at 60 °C for 16 hours. The reaction mixture was concentrated *in vacuo* and then purified by column chromatography (0-50% EtOAc in hexane) to afford 7 (1.524 g, 4.76 mmol, 47% yield) as a yellow crystalline solid. HRMS (ESI) m/z: [(M-Boc)+H]⁺ calculated for C₆H₆BrN₂O₄(⁸¹Br): 218.9592, found 218.9594.

**Tert-butyl (2-nitro-4-(thiophen-2-yl)phenyl)carbamate (8)**: To a solution of DME/water (2:1, 36 mL) was added thiophen-2-ylboronic acid (0.77 g, 6.05 mmol), 7 (1.60 g, 5.05 mmol), Na₂CO₃ (0.80 g, 7.57 mmol) and Pd(PPh₃)₄ (0.38 g, 0.33 mmol). The resulted mixture was stirred vigorously at 110 °C for 16 hours. The reaction mixture was diluted with more water (40 mL) and then the product was extracted with EtOAc (3 x 50 mL). The organic layers were combined, washed with water (2 x 70 mL), dried over MgSO₄, filtered and concentrated in vacuo to afford a brown solid (1.920 g). The crude product was purified by column chromatography (dry load, 0-50% EtOAc in hexane) to give **8** (1.21 g, 3.74 mmol, 74% yield) as an orange crystalline solid. HRMS (ESI) m/z: [M-H]- calculated for C₁₅H₁₅N₂O₄S: 319.0753, found 319.0753.

**Tert-butyl (2-amino-4-(thiophen-2-yl)phenyl)carbamate (9):** To a solution of **8** (1.181 g, 3.69 mmol) in MeOH (15 mL), 10% Pd/C (0.120 g) was added. The reaction flask was filled with nitrogen and evacuated 3 times using a Shlenk line, before a balloon of hydrogen was added and the resultant mixture stirred vigorously for 4 hours. The balloon of hydrogen was removed and the flask was flushed with nitrogen. The reaction mixture was filtered through a glass microfiber filter and then the filtrate was concentrated in *vacuo* to afford **9** (1.057 g, 3.60 mmol, 98% yield) as a brown solid. HRMS (ESI) m/z: [M+H]⁺ calculated for C₁₅H₁₉N₂O₂S: 291.1167, found 291.1167.

**Tert-butyl (2-(4-nitrobenzamido)phenyl)carbamate (10a):** Following general method A, **10a** was obtained from **2** (4.17 g, 20.0 mmol) and 4-nitrobenzoyl chloride (4.09 g, 22.0 mmol). The crude product was triturated in EtOH and then filtered to afford **10a** (5.52 g, 15.3 mmol, 76% yield) as a pale yellow solid. HRMS (ESI) m/z: [M+Na]⁺ calculated for C₁₈H₁₉N₃O₅Na: 380.1222, found 380.1223.

**Tert-butyl (5-fluoro-2-(4-nitrobenzamido)phenyl)carbamate (10b):** Following general method A, **10b** was obtained from **5** (1.27 g, 5.60 mmol) and 4-nitrobenzoyl chloride (1.15 g, 6.17 mmol). The crude product was purified by column chromatography (0-50% EtOAc in hexane) to afford **10b** (1.87 g, 4.93 mmol, 88% yield) as a fluffy, pale brown solid. HRMS (ESI) m/z: [M+Na]⁺ calculated for C₁₈H₁₈N₃O₅FNa: 398.1128, found 398.1125.

**Tert-butyl (2-(4-nitrobenzamido)-4-(thiophen-2-yl)phenyl)carbamate (10c):** Following general method A, **10c was** obtained from **9** (400 mg, 1.38 mmol) and 4-nitrobenzoyl chloride (284 mg, 1.53 mmol). The crude product was triturated in EtOH and then filtered to afford **10c** (459 mg, 1.04 mmol, 76% yield) as a pale green solid. HRMS (ESI) m/z: [M+H]⁺ calculated for C₂₂H₂₂N₃O₅S: 440.1280, found 440.1280.

**Tert-butyl (2-(4-aminobenzamido)phenyl)carbamate (11a):** To a solution of **10a** (5.52 g, 15.3 mmol) in MeOH/THF (1:1, 100 mL), 10% Pd/C (0.55 g) was added. The reaction flask was filled with nitrogen and evacuated 3 times using a Shlenk line, before a balloon of hydrogen was added and the resultant mixture stirred vigorously for 18 hours. The balloon of hydrogen was removed and the flask was flushed with nitrogen. The reaction mixture was filtered through celite, then the celite was washed with more MeOH (3 x 50 mL) and the filtrate concentrated in *vacuo* to afford **11a** (5.23 g, 15.3 mmol, 100% yield) as a fluffy white crystalline solid. HRMS (ESI) m/z: [M+Na]⁺ calculated for C₁₈H₂₁N₃O₃Na: 350.1481, found 350.1486.

**Tert-butyl (2-(4-aminobenzamido)-5-fluorophenyl)carbamate (11b):** To a solution of **10b** (1.525 g, 4.063 mmol) in MeOH (100 mL), 10% Pd/C (0.155 g) was added. The reaction flask was filled with nitrogen and evacuated 3 times using a Shlenk line, before a balloon of hydrogen was added and the resultant mixture stirred vigorously for 7 hours. The balloon of hydrogen was removed and the flask was flushed with nitrogen. The reaction mixture was filtered through a glass microfiber filter and then the filtrate was concentrated in *vacuo* to afford **11b** (1.335 g, 3.827 mmol, 94% yield) as a pale grey crystalline solid. HRMS (ESI) m/z: [M+H]⁺ calculated for C₁₈H₂₁N₃O₃F: 346.1567, found 346.1563.

**Tert-butyl (2-(4-aminobenzamido)-4-(thiophen-2-yl)phenyl)carbamate (11c):** To a solution of **10c** (0.333 g, 0.76 mmol) in MeOH/DCM (1:1, 120 mL), SnCl₂ (0.863 g, 4.55 mmol) was added and the resultant mixture stirred at room temperature for 1 week. The reaction mixture was cooled to 0°C, then saturated Na₂CO₃ (40 mL) was added slowly. The product was extracted into DCM (4 x 40 mL), then the organic layers were combined and washed with brine (2 x 80 mL). The organic layer was dried over Na₂SO₄, filtered and concentrated in *vacuo* to afford **11c** (0.283 g, 0.62 mmol, 82% yield) as a yellow crystalline solid. HRMS (ESI) m/z: [M+H]⁺ calculated for C₂₂H₂₄N₃O₃S: 410.1538, found 410.1530.

**Tert-butyl (2-(4-acetamidobenzamido)phenyl)carbamate (12a):** Following general method B, **12a** was obtained from **11a** (200 mg, 0.611 mmol) and acetyl chloride (0.052 mL, 0.733 mmol). The crude product was purified by column chromatography (dry load, 100% EtOAc) to afford **12a** (193 mg, 0.517 mmol, 85% yield) as a white solid. HRMS (ESI) m/z: [M+Na]⁺ calculated for C₂₀H₂₃N₃O₄Na: 392.1586, found 392.1586.

**Tert-butyl (2-(4-acetamidobenzamido)-5-fluorophenyl)carbamate (12b):** Following general method B, **12b** was obtained from **11b** (95.7 mg, 0.277 mmol) and acetyl chloride (0.024 mL, 0.333 mmol). The crude product was purified by column chromatography (dry load, 50-100% EtOAc) to afford **12b** (101.0 mg, 0.258 mmol, 93% yield) as a pale yellow/brown solid. HRMS (ESI) m/z: [M+H]⁺ calculated for C₂₀H₂₃FN₃O₄: 388.1673, found 388.1672.

**Tert-butyl (2-(4-acetamidobenzamido)-4-(thiophen-2-yl)phenyl)carbamate (12c):** Following general method B, **12c** was obtained from **11c** (96.6 mg, 0.236 mmol) and acetyl chloride (0.020 mL, 0.283 mmol). The crude product was purified by column chromatography (0-100% EtOAc) to afford **12c** (72.8 mg, 0.160 mmol, 68% yield) as a pale yellow/green solid. HRMS (ESI) m/z: [M+H]⁺ calculated for C₂₄H₂₆N₃O₄S: 452.1644, found 452.1643.

**4-acetamido-N-(2-aminophenyl)benzamide (CI-994):** Following general method C, Boc deprotection of **12a** (47.5 mg, 0.129 mmol) was performed to afford **CI-994** (35.6 mg, 0.126 mmol, 97 % yield) as a yellow/white solid. HRMS (ESI) m/z: [M+H]⁺ calculated for C₁₅H₁₆N₃O₂: 270.1243, found 270.1247.

**4-acetamido-N-(2-amino-4-fluorophenyl)benzamide (BRD3308):** Following general method C, Boc deprotection of **12b** (87.0 mg, 0.225 mmol) was performed to afford **BRD3308** (59.3 mg, 0.202 mmol, 90 % yield) as a yellow/brown solid. HRMS (ESI) m/z: [M+H]⁺ calculated for C₁₅H₁₅FN₃O₂: 288.1148, found 288.1139.

**4-acetamido-N-(2-amino-5-(thiophen-2-yl)phenyl)benzamide (Cpd-60):** Following general method C, Boc deprotection of **12c** (48.2 mg, 0.107 mmol) was performed to afford **Cpd-60** (35.5 mg, 0.100 mmol, 93 % yield) as a pale yellow/green solid. HRMS (ESI) m/z: [M+H]⁺ calculated for C₁₉H₁₈N₃O₂S: 352.1120, found 352.1120.

### Synthesis of Linker

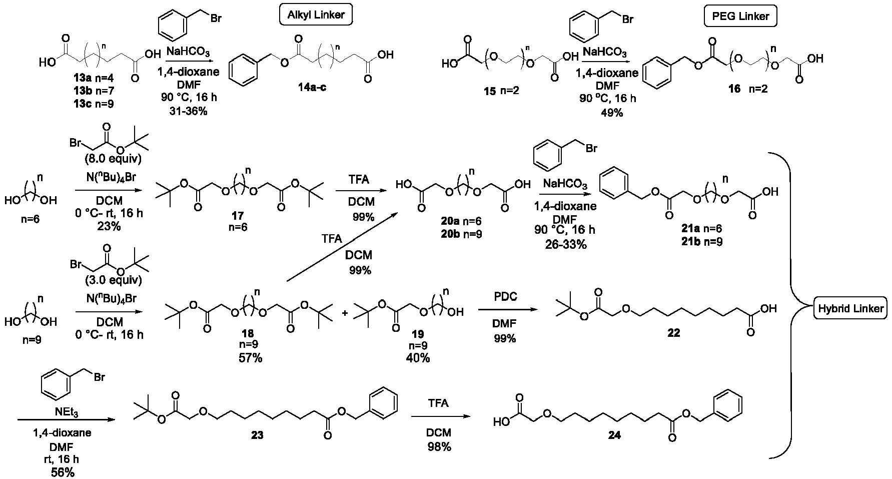

**9-(benzyloxy)-9-oxononanoic acid (14a):** Following general method D, **14a** was obtained from **13a** (2.00 g, 10.63 mmol) and benzyl bromide (1.26 mL, 10.63 mmol). The crude product was purified by column chromatography (50% EtOAc in hexane) to afford **14a** (1.083 g, 3.85 mmol, 36% yield) as a clear colourless oil. HRMS (ESI) m/z: [M+Na]⁺ calculated for C₁₆H₂₂O₄Na: 301.1416, found 301.1415.

**12-(benzyloxy)-12-oxododecanoic acid (14b):** Following general method D, **14b** was obtained from **13b** (2.00 g, 8.68 mmol) and benzyl bromide (1.03 mL, 8.68 mmol). The crude product was purified by column chromatography (50% EtOAc in hexane) to afford **14b** (0.998g, 2.99 mmol, 34% yield) as a clear colourless oil. HRMS (ESI) m/z: [M+Na]⁺ calculated for C₁₉H₂₈O₄Na : 343.1885, found 343.1887.

**14-(benzyloxy)-14-oxotetradecanoic acid (14c):** Following general method D, **14c** was obtained from **13c** (1.50 g, 5.81 mmol) and benzyl bromide (0.69 mL, 5.81 mmol). The crude product was purified by column chromatography (50% EtOAc in hexane) to afford **14c** (0.628 g, 1.78 mmol, 31% yield) as a clear colourless oil. HRMS (ESI) m/z: [M+H]⁺ calculated for C₂₁H₃₃O₄: 349.2379, found 349.2374.

**3-oxo-1-phenyl-2,5,8,11-tetraoxatridecan-13-oic acid (16):** Following general method D, **16** was obtained from **15** (1.00 g, 3.15 mmol) and benzyl bromide (0.37 mL, 3.15 mmol). The crude product was purified by column chromatography (0-10% MeOH in DCM) to afford **16** (0.484 g, 1.53 mmol, 49% yield) as a clear yellow oil. HRMS (ESI) m/z: [M+H]⁺ calculated for C₁₅H₂₁O_{7:}313.1287, found 313.1290.

**Bi-tert-butyl 2,2'-(hexane-1,6-diylbis(oxy))diacetate (17):** Hexane-1,6-diol (1.50 g, 12.69 mmol) was dissolved in DCM (52 ml) before tert-butyl 2-bromoacetate (15.0 ml, 102.28 mmol) was added dropwise, followed by the addition of tetra-n-butylammonium bromide (4.50 g, 13.96 mmol). The reaction mixture was then cooled to 0°C before NaOH (37% w/w, 52 ml) was added. The reaction mixture was then allowed to stir vigorously at room temperature for 16 hours. The crude reaction mixture was biphasic and the organic layer (top layer) was yellow in colour. The organic layer was separated then the aqueous layer was washed with DCM (3 x 15 ml). The organic layers were combined, dried over MgSO₄ before being filtered and concentrated in *vacuo* to yield a clear pale yellow oil (6.87 g), which slowly crystallised. The crude product was purified by column chromatography (1% MeOH in DCM) to afford **17** (1.015 g, 2.90 mmol, 23% yield) as a clear colourless oil. HRMS (ESI) m/z: [M+Na]⁺ calculated for C₁₈H₃₄O₆Na: 369.2253, found 369.2255.

**Di-tert-butyl 2,2'-(nonane-1,9-diylbis(oxy))diacetate (18) and tert-butyl 2-((9-hydroxynonyl) oxy)acetate (19):** Nonane-1,9-diol (2.00 g, 12.48 mmol) was dissolved in DCM (50 ml) before tert-butyl 2-bromoacetate (5.53 ml, 37.44 mmol) was added dropwise, followed by the addition of tetra-n-butylammonium bromide (4.43 g, 13.73 mmol). The reaction mixture was then cooled to 0°C before NaOH (37% w/w, 50 ml) was added. The reaction mixture was then allowed to stir vigorously at room temperature for 16 hours. The crude reaction mixture was biphasic and the organic layer (top layer) was pale yellow in colour. The organic layer was separated then the aqueous layer was washed with DCM (3 x 15 ml). The organic layers were combined, dried over MgSO₄ before being filtered and concentrated in *vacuo* to yield a clear pale yellow oil. The crude product was purified by column chromatography (10-30% EtOAc in hexane) to afford **18** (2.80 g, 7.14 mmol, 57% yield) and **19** (1.387 g, 5.01 mmol, 40% yield) as clear colourless oils. HRMS (ESI) m/z: [M+Na]⁺ calculated for C₁₅H₃₀O₄Na: 297.2042, found 297.2046.

**2,2'-(hexane-1,6-diylbis(oxy))diacetic acid (20a):** TFA (0.4 mL) was added to a stirring solution of **17** (0.460 g, 1.33 mmol) in DCM (2 mL) and the resulting reaction mixture stirred at room temperature for 4 hours. The reaction mixture was concentrated in *vacuo* to afford **20a** (319.0 mg, 1.36 mmol, 99% yield) as a white solid. HRMS (ESI) m/z: [M+H]⁺ calculated for C₁₀H₁₉O₆: 235.1182, found 235.1182.

**2,2'-(nonane-1,9-diylbis(oxy))diacetic acid (20b):** TFA (3 mL) was added to a stirring solution of **18** (1.55 g, 3.99 mmol) in DCM (3 mL) and the resulting reaction mixture stirred at room temperature for 4.5 hours. The reaction mixture was concentrated in *vacuo* to afford **20b** (1.09 g, mmol, 3.95 mmol, 99% yield) as a white solid. HRMS (ESI) m/z: [M+H]⁺ calculated for C₁₃H₂₅O₆: 277.1651, found 277.1656.

**2-((6-(2-(benzyloxy)-2-oxoethoxy)hexyl)oxy)acetic acid (21a):** Following general method D, **21a** was obtained from **20a** (0.304 g, 1.30 mmol) and benzyl bromide (0.15 mL, 1.30 mmol). The crude product was purified by column chromatography (0-100% EtOAc in hexane) to afford **21a** (0.110 g, 0.34 mmol, 26% yield) as a yellow oil. HRMS (ESI) m/z: [M+H]⁺ calculated for C₁₇H₂₅O₆: 325.1651, found 325.1659.

**2-((9-(2-(benzyloxy)-2-oxoethoxy)nonyl)oxy)acetic acid (21b):** Following general method D, **21b** was obtained from **20b** (1.047 g, 3.79 mmol) and benzyl bromide (0.45 mL, 3.79 mmol). The crude product was purified by column chromatography (0-50% EtOAc in hexane) to afford **21b** (0.463 g, 1.24 mmol, 33% yield) as a clear pale yellow oil. HRMS (ESI) m/z: [M+H]⁺ calculated for C₂₀H₃₁O₆: 367.2121, found 367.2121.

**9-(2-(tert-butoxy)-2-oxoethoxy)nonanoic acid (22):** Pyridinium dichromate (685.5 mg, 1.822 mmol) was added portionwise to a solution of **19** (100.0 mg, 0.364 mmol) in dry DMF (2 mL) and stirred at room temperature for 6 hours. The reaction mixture was diluted with 10% citric acid solution (30 mL) and extracted with EtOAc (4 x 20 mL). The combined organic layers were washed with sat. NaCl (3 x 30 mL), dried over Na₂SO₄, filtered and concentrated in *vacuo* to afford **22** (109.8 mg, 0.361 mmol, 99% yield) as a pale yellow oil. HRMS (ESI -ve, Direct Infusion) m/z: [M-H]-calculated for C₁₅H₂₇O₅: 287.1858, found 287.1862.

**Benzyl 9-(2-(tert-butoxy)-2-oxoethoxy)nonanoate (23):** To a solution of **22** (215.3 mg, 0.747 mmol) in 1,4-dioxane/DMF (1:1, 8 mL), was added benzyl bromide (0.10 mL, 0.821 mmol) followed by the addition of NEt₃ (0.12 mL, 0.885 mmol), then the resultant mixture was stirred at room temperature for 5 hours. The reaction mixture was concentrated in *vacuo* to afford a yellow oil, then the crude product was purified by column chromatography (0-40% EtOAc in hexane) to afford **23** (159.9 mg, 0.418 mmol, 56% yield) as a clear pale yellow oil. HRMS (ESI) m/z: [M+Na]⁺ calculated for C₂₂H₃₄O₅Na: 401.2304, found 401.2303.

**2-((9-(benzyloxy)-9-oxononyl)oxy)acetic acid (24):** TFA (1 mL) was added to a stirring solution of **23** (159.9 mg, 0.422) in DCM (2 mL) and the resulting reaction mixture stirred at room temperature for 4 hours. The reaction mixture was concentrated in *vacuo* to afford **24** (132.5 mg, 0.407 mmol, 98% yield) as a clear colourless oil. HRMS (ESI) m/z: [M+H]⁺ calculated for C₁₈H₂₇O₅: 323.1858, found 323.1862.

### Synthesis of HDACi-linker Conjugates with VHL Ligand

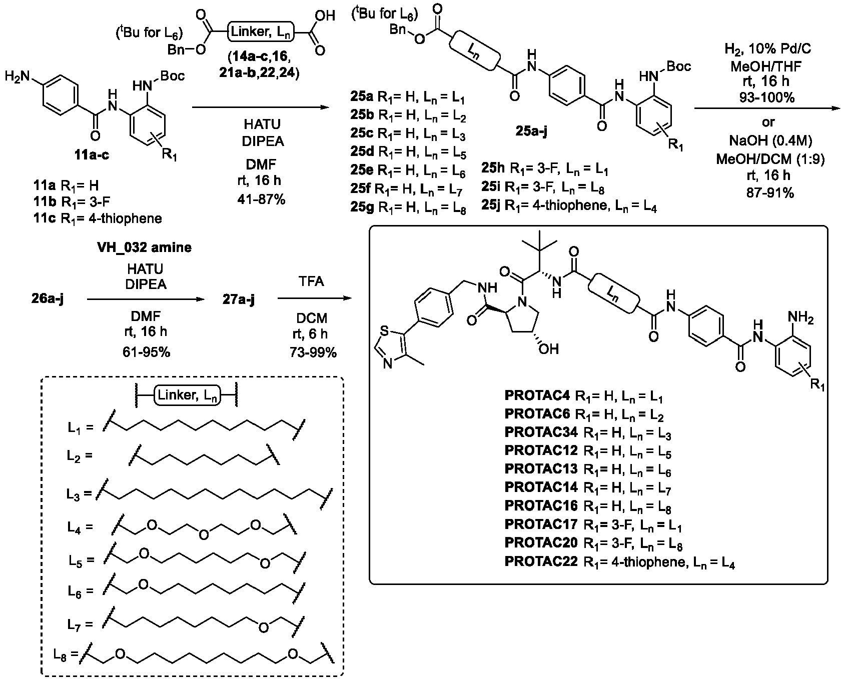

**Benzyl 12-((4-((2-((tert-butoxycarbonyl)amino)phenyl)carbamoyl)phenyl)amino)-12-oxododeca-noate (25a):** Following general method E, **25a** was obtained from **14a** (257 mg, 0.802 mmol) and **11a** (200 mg, 0.611 mmol). The crude product was purified by column chromatography (50% EtOAc in hexane) to give **25a** (274 mg, 0.430 mmol, 70% yield) as a pale yellow solid. HRMS (ESI) m/z: [M+Na]⁺ calculated for C₃₇H₄₇N₃O₆Na: 652.3363, found 652.3364.

### Benzyl 9-((4-((2-((tert-butoxycarbonyl)amino)phenyl)carbamoyl)phenyl)amino)-9-oxononanoate (25b):

Following general method E, **25b** was obtained from **14b** (0.475 g, 1.71 mmol) and **11a** (0.430 g, 1.31 mmol). The crude product was purified by column chromatography (0-50% EtOAc in hexane) to give **25b** (0.540 g, 0.94 mmol, 53% yield) as a yellow tar. HRMS (ESI) m/z: [M+H]⁺ calculated for C₃₄H₄₂N₃O₆: 588.3074, found 588.3067.

**Benzyl 14-((4-((2-((tert-butoxycarbonyl)amino)phenyl)carbamoyl)phenyl)amino)-14-oxotetradecanoate (25c):** Following general method E, **25c** was obtained from **14c** (421.4 mg, 1.21 mmol) and **11a** (300.0 mg, 0.92 mmol). The crude product was purified by column chromatography (0-50% EtOAc in hexane) to give **25c** (372.4 mg, 0.56 mmol, 61% yield) as a yellow solid. HRMS (ESI) m/z: [M+H]⁺ calculated for C₃₉H₅₂N₃O₆: 658.3856, found 658.3880.

**Benzyl 2-((6-(2-((4-((2-((tert-butoxycarbonyl)amino)phenyl)carbamoyl)phenyl)amino)-2-oxoethoxy)hexyl)oxy)acetate (25d):** Following general method E, **25d** was obtained from **21a** (78.2 mg, 0.241 mmol) and **11a** (71.8 mg, 0.219 mmol). The crude product was purified by column chromatography (0-100% EtOAc in hexane) to afford **25d** (76.5 mg, 0.120 mmol, 55% yield) as a pale yellow oil. HRMS (ESI) m/z: [M+H]⁺ calculated for C₃₅H₄₄N₃O₈: 634.3128, found 634.3125.

**Tert-butyl 2-((9-((4-((2-((tert-butoxycarbonyl)amino)phenyl)carbamoyl)phenyl)amino)-9-oxononyl)oxy)acetate (25e):** Following general method E, **25e** was obtained from **22** (85.0 mg, 0.295 mmol) and **11a** (88.4 mg, 0.270 mmol). The crude product was purified by column chromatography (0-100% EtOAc in hexane) to afford **25e** (66.3 mg, 0.110 mmol, 41% yield) as a yellow tar. HRMS (ESI) m/z: [M+H]⁺ calculated for C₃₃H₄₈N₃O₇: 598.3492, found 598.3484.

**Benzyl 9-(2-((4-((2-((tert-butoxycarbonyl)amino)phenyl)carbamoyl)phenyl)amino)-2-oxoethoxy)nonanoate (25f):** Following general method E, **25f** was obtained from **24** (130.0 mg, 0.403 mmol) and **11a** (110.0 mg, 0.336 mmol). The crude product was purified by column chromatography (0-100% EtOAc in hexane) to afford **25f** (157.4 mg, 0.247 mmol, 73% yield) as a clear colourless tar. HRMS (ESI) m/z: [M+H]⁺ calculated for C₃₆H₄₆N₃O₇: 632.3336, found 632.3335.

**Benzyl 2-((9-(2-((4-((2-((tert-butoxycarbonyl)amino)phenyl)carbamoyl)phenyl)amino)-2-oxoetho xy)nonyl)oxy)acetate (25g):** Following general method E, **25g** was obtained from **21b** (145.0 mg, 0.396 mmol) and **11a** (99.6 mg, 0.304 mmol). The crude product was purified by column chromatography (0-70% EtOAc in hexane) to afford **25g** (157.8 mg, 0.229 mmol, 75% yield) as a clear colourless tar. HRMS (ESI) m/z: [M+H]⁺ calculated for C₃₈H₅₀N₃O₈: 676.3598, found 676.3605.

**Benzyl 12-((4-((2-((tert-butoxycarbonyl)amino)-4-fluorophenyl)carbamoyl)phenyl)amino)-12-oxododecanoate (25b):**

Following general method E, **25h** was obtained from **14a** (246.4 mg, 0.767 mmol) in and **11b** (201.8 mg, 0.584 mmol). The crude product was purified by column chromatography (10-80% EtOAc in hexane) to give **25h** (332.4 mg, 0.508 mmol, 87% yield) as a dark orange tar. HRMS (ESI) m/z: [M+H]⁺calculated for C₃₇H₄₇FN₃O₆: 648.3449, found 648.3452.

**Benzyl 2-((9-(2-((4-((2-((tert-butoxycarbonyl)amino)-4-fluorophenyl)carbamoyl)phenyl)amino)-2-oxoethoxy)nonyl)oxy)acetate (25i):** Following general method E, **25i** was obtained from **21b** (167.7 mg, 0.458 mmol) and **11b** (120.0 mg, 0.347 mmol). The crude product was purified by column chromatography (25-30% EtOAc in hexane) to afford **25i** (114.8 mg, 0.164 mmol, 42% yield) as an orange tar. HRMS (ESI) m/z: [M+H]⁺calculated for C₃₈H₄₉FN₃O₈: 694-3504, found 694.3517.

**Benzyl 2-(2-(2-(2-((4-((2-((tert-butoxycarbonyl)amino)-5-(thiophen-2-yl)phenyl)carbamoyl)phenyl) amino)-2-oxoethoxy)ethoxy)ethoxy)acetate (25j):** Following general method E, **25j** was obtained from **16** (114.0 mg, 0.362 mmol) and **11c** (114.0 mg, 0.278 mmol). The crude product was purified by column chromatography (0-100% EtOAc in hexane, product eluted at 80-90% EtOAc) to afford **25i** (117.5 mg, 0.162 mmol, 58% yield) as a dark yellow tar. HRMS (ESI) m/z: [M+H]⁺ calculated for C₃-H₄₂N₃O₉S: 704.2642, found 704.2637.

**12-((4-((2-((Tert-butoxycarbonyl)amino)phenyl)carbamoyl)phenyl)amino)-12-oxododecanoic acid (26a)** : Following general method F, benzyl ester hydrogenolysis of **25a** (171 mg, 0.271 mmol) was performed to afford **26a** (146 mg, 0.266 mmol, 98% yield) as an off-white solid. HRMS (ESI) m/z: [M+Na]⁺ calculated for C₃₀H₄₁N₃O₆Na: 562.2893, found 562.2886.

**9-((4-((2-((tert-butoxycarbonyl)amino)phenyl)carbamoyl)phenyl)amino)-9-oxononanoic acid (26b):** Following general method F, benzyl ester hydrogenolysis of **25b** (370.8 mg, 0.63 mmol) was performed to afford **26b** (295.1 mg, 0.59 mmol, 93% yield) as a white crystalline solid. HRMS (ESI) m/z: [M+H]⁺ calculated for C₂₇H₃₆N₃O₆: 498.2604, found 498.2597.

**14-((4-((2-((tert-butoxycarbonyl)amino)phenyl)carbamoyl)phenyl)amino)-14-oxotetradecanoic acid (26c):** Following general method F, benzyl ester hydrogenolysis of **25c** (348.6 mg, 0.530 mmol) was performed to afford **26c** (302.0 mg, 0.527 mmol, 99% yield) as a white solid. HRMS (ESI) m/z: [M+H]⁺ calculated for C₃₂H₄₆N₃O₆: 568.3387, found 568.3391.

**2-((6-(2-((4-((2-((tert-butoxycarbonyl)amino)phenyl)carbamoyl)phenyl)amino)-2-oxoethoxy)hex yl)oxy)acetic acid (26d):** Following general method F, benzyl ester hydrogenolysis of **25d** (76.5 mg, 0.121 mmol) was performed to afford **26d** (65.8 mg, 0.120 mmol, 99% yield) as a clear colourless oil. HRMS (ESI) m/z: [M+H]⁺ calculated for C₂₈H₃₈N₃O₈: 544.2659, found 544.2645.

**2-((9-((4-((2-((tert-butoxycarbonyl)amino)phenyl)carbamoyl)phenyl)amino)-9-oxononyl)oxy)ace tic acid (26c):** To a solution of **25c** (62.7 mg, 0.111 mmol) in DCM (2.7 mL) a solution of NaOH in MeOH (4M, 0.3 mL) was added. The reaction was stirred at room temperature for 16 h. The reaction mixture was concentrated in *vacuo,* then redissolved in water (10 mL) and acified with HCl (3M, ca. 1 mL) to pH 2. The product was then extracted in EtOAc (2 x 20 mL), dried over Na₂SO₄, filtered and concentrated in *vacuo* to afford **26e** as a yellow solid (55.4 mg, 0.101 mmol, 91% yield). HRMS (ESI) m/z: [M+H]⁺ calculated for C₂₉H₄₀N₃O₇: 542.2866, found 542.2861.

**9-(2-((4-((2-((tert-butoxycarbonyl)amino)phenyl)carbamoyl)phenyl)amino)-2-oxoethoxy)nonanoic acid (26f):** Following general method F, benzyl ester hydrogenolysis of **25f** (120.2 mg, 0.190 mmol) was performed to afford **26f** (105.6 mg, 0.189 mmol, 99% yield) as a clear colourless tar. HRMS (ESI) m/z: [M+H]⁺ calculated for C₂₉H₄₀N₃O₇: 542.2866, found 542.2863.

**2-((9-(2-((4-((2-((tert-butoxycarbonyl)amino)phenyl)carbamoyl)phenyl)amino)-2-oxoethoxy)non yl)oxy)acetic acid (26g):** Following general method F, benzyl ester hydrogenolysis of **25g** (134.8 mg, 0.199 mmol) was performed to afford **26g** (118.7 mg, 0.199 mmol, 100% yield) as a clear colourless tar. HRMS (ESI) m/z: [M+H]⁺ calculated for C₃₁H₄₄N₃O₈: 586.3128, found 586.3128.

### 12-((4-((2-((tert-butoxycarbonyl)amino)-4-fluorophenyl)carbamoyl)phenyl)amino)-12-oxododecanoic acid (26h):

Following general method F, benzyl ester hydrogenolysis of **25h** (219.6 mg, 0.339 mmol) was performed to afford **26h** (192.9 mg, 0.266 mmol, 98% yield) as a pale purple/brown solid. HRMS (ESI) m/z: [M+H]⁺ calculated for C₃₀H₄₁FN₃O₆: 558.2979, found 558.2974.

**2-((9-(2-((4-((2-((tert-butoxycarbonyl)amino)-4-fluorophenyl)carbamoyl)phenyl)amino)-2-oxoethoxy)nonyl)oxy)acetic acid (26i):** Following general method F, benzyl ester hydrogenolysis of **25i** (114.0 mg, 0.164 mmol) was performed to afford **26i** (99.8 mg, 0.164 mmol, 100% yield) as a yellow/brown tar. HRMS (ESI) m/z: [M+H]⁺ calculated for C₃₁H₄₃FN₃O₈: 604.3034, found 604.3046.

**2-(2-(2-(2-((4-((2-((tert-butoxycarbonyl)amino)-5-(thiophen-2-yl)phenyl)carbamoyl)phenyl)amino)-2-oxoethoxy)ethoxy)ethoxy)acetic acid (26j):** To a solution of **25j** (111.6 mg, 0.159 mmol) in MeOH (10 mL) was added NaOH (160 mg) until solution reached pH 10 (0.4 M NaOH in MeOH), then the resultant solution stirred at room temperature for 4 hours. The reaction mixture was concentrated in *vacuo,* then dissovled in water (10 mL) and washed with EtOAC (20 mL). The aqueous layer was acidified to pH₂ with HCl (1M) and the product was extracted with EtOAc (2 x 20 mL), then the combined organic layers dried over Na₂SO₄, filtered and concentrated in *vacuo* to afford **26j** (96.7 mg, 0.154 mmol, 87% yield) as a white solid. HRMS (ESI) m/z: [M+H]⁺ calculated for C₃₀H₃₆N₃O₉S: 614.2172, found 614.2161.

**Tert-butyl(2-(4-(12-(((S)-1-((2S,4R)-4-hydroxy-2-((4-(4-methylthiazol-5-yl)benzyl)carbamoyl)pyrr olidin-1-yl)-3,3-dimethyl-1-oxobutan-2-yl)amino)-12-oxododecanamido)benzamido)phenyl)carbamate (27a):** Following general method G, **27a** was obtained from **26a** (65.5 mg, 0.121 mmol) and **VH_032 amine** (50.0 mg, 0.099 mmol). The crude product was purified by column chromatography (0-5% MeOH in DCM) to afford **27a** (61.9 mg, 0.064 mmol, 64% yield) as a white solid. HRMS (ESI) m/z: [M+H]⁺ calculated for C₅₂H₇₀N₇O₈S: 952.5007, found 952.5009.

**Tert-butyl (2-(4-(9-(((S)-1-((2S,4R)-4-hydroxy-2-((4-(4-methylthiazol-5-yl)benzyl)carbamoyl)pyrrolid in-1-yl)-3,3-dimethyl-1-oxobutan-2-yl)amino)-9-oxononanamido)benzamido)phenyl)carbamate (27b):** Following general method G, **27b** was obtained from **26b** (59.9 mg, 0.120 mmol) and **VH_032 amine** (50.0 mg, 0.099 mmol). The crude product was purified by column chromatography (0-5% MeOH in DCM) to afford **27b** (76.1 mg, 0.083 mmol, 84% yield) as a white solid. HRMS (ESI) m/z: [M+H]⁺ calculated for C₄₉H₆₄N₇O₈S: 910,4537, found 910.4529.

**Tert-butyl (2-(4-(14-(((S)-1-((2S,4R)-4-hydroxy-2-((4-(4-methylthiazol-5-yl)benzyl)carbamoyl)pyrrolidin-1-yl)-3,3-dimethyl-1-oxobutan-2-yl)amino)-14-oxotetradecanamido)benzamido)phenyl)carbamate (27c):** Following general method G, **27c** was obtained from **26c** (53.5 mg, 0.094 mmol) and **VH_032 amine** (40.0 mg, 0.080 mmol). The crude product was purified by column chromatography (0-5% MeOH in DCM) to afford **27c** (62.5 mg, 0.063 mmol, 79% yield) as a white solid. HRMS (ESI) m/z: [M+H]⁺ calculated for C₅₄H₇₄N₇O₈S: 980.5320, found 980.5303.

**Tert-butyl (2-(4-(2-((6-(2-(((S)-1-((2S,4R)-4-hydroxy-2-((4-(4-methylthiazol-5-yl)benzyl)carbamoyl)pyrrolidin-1-yl)-3,3-dimethyl-1-oxobutan-2-yl)amino)-2-oxoethoxy)hexyl)oxy)acetamido)benz amido)phenyl)carbamate (27d):** Following general method G, **27d** was obtained from **26d** (67.2 mg, 0.124 mmol) and **VH_032 amine** (50.0 mg, 0.099 mmol). The crude product was purified by column chromatography (0-10% MeOH in DCM) to afford **27d** (92.4 mg, 0.093 mmol, 94% yield) as a white solid. HRMS (ESI) m/z: [M+H]⁺ calculated for C₅₀H₆₆N₇O₁₀S: 956.4592, found 956.4590.

**Tert-butyl (2-(4-(9-(2-(((S)-1-((2S,4R)-4-hydroxy-2-((4-(4-methylthiazol-5-yl)benzyl)carbamoyl) pyrrolidin-1-yl)-3,3-dimethyl-1-oxobutan-2-yl)amino)-2-oxoethoxy)nonanamido)benzamido)phe nyl)carbamate (27e):** Following general method G, **27e** was obtained from **26e** (52.3 mg, 0.099 mmol) and **VH_032 amine** (40.0 mg, 0.080 mmol). The crude product was purified by column chromatography (0-10% MeOH in DCM) to afford **27e** (68.4 mg, 0.071 mmol, 89% yield) as a white solid. HRMS (ESI) m/z: [M+H]⁺ calculated for C₅₁H₆₈N₇O₉S: 954-4799, found 954.4798.

**Tert-butyl (2-(4-(2-((9-(((S)-1-((2S,4R)-4-hydroxy-2-((4-(4-methylthiazol-5-yl)benzyl)carbamoyl) pyrrolidin-1-yl)-3,3-dimethyl-1-oxobutan-2-yl)amino)-9-oxononyl)oxy)acetamido)benzamido)ph enyl)carbamate (27f):** Following general method G, **27f** was obtained from **26f** (52.8 mg, 0.097 mmol) and **VH_032 amine** (40.0 mg, 0.080 mmol). The crude product was purified by column chromatography (0-10% MeOH in DCM) to afford **27f** (72.9, 0.076 mmol, 95% yield) as a pale yellow/white solid. HRMS (ESI) m/z: [M+H]⁺ calculated for C₅₁H₆₈N₇O₉S: 954-4799, found 954.4792.

**Tert-butyl (2-(4-(2-((9-(2-(((S)-1-((2S,4R)-4-hydroxy-2-((4-(4-methylthiazol-5-yl)benzyl)carbamoyl)pyrrolidin-1-yl)-3,3-dimethyl-1-oxobutan-2-yl)amino)-2-oxoethoxy)nonyl)oxy)acetamido)benz amido)phenyl)carbamate (27g):** Following general method G, **27g** was obtained from **26g** (56.2 mg, 0.096 mmol) and **VH_032 amine** (40.0 mg, 0.080 mmol). The crude product was purified by column chromatography (0-8% MeOH in DCM) to afford **27g** (68.4 mg, 0.068 mmol, 85% yield) as a white solid. HRMS (ESI) m/z: [M+H]⁺ calculated for C₅₃H₇₂N₇O₁₀S: 998.5061, found 998.5048.

**Tert-butyl (5-fluoro-2-(4-(12-(((S)-1-((2S,4R)-4-hydroxy-2-((4-(4-methylthiazol-5-yl)benzyl)carbamoyl)pyrrolidin-1-yl)-3,3-dimethyl-1-oxobutan-2-yl)amino)-12-oxododecanamido)benzamido)phenyl) carbamate (27h):** Following general method G, 27h was obtained from 26h (53.2 mg, 0.095 mmol) and VH_032 **amine** (40.0 mg, 0.080 mmol). The crude product was purified by column chromatography (0-10% MeOH in DCM) to afford 27h (58.7 mg, 0.060 mmol, 75% yield) as a white solid. HRMS (ESI) m/z: [M+H]⁺ calculated for C₅₂H₆₉FN₇O₈S: 970.4912, found 970.4913.

**Tert-butyl (5-fluoro-2-(4-(2-((9-(2-(((S)-1-((2S,4R)-4-hydroxy-2-((4-(4-methylthiazol-5-yl)benzyl) carbamoyl)pyrrolidin-1-yl)-3,3-dimethyl-1-oxobutan-2-yl)amino)-2-oxoethoxy)nonyl)oxy)acetamido) benzamido)phenyl)carbamate (27i):** Following general method G, **27i** was obtained from **26i** (57.6 mg, 0.096 mmol) and **VH_032 amine** (40.0 mg, 0.080 mmol). The crude product was purified by column chromatography (0-8% MeOH in DCM) to afford **27i** (75.7, 0.072 mmol, 90% yield) as a pale yellow/white solid. HRMS (ESI) m/z: [M+H]⁺ calculated for C₅₃H₇₁FN₇O₁₀S: 1016.4967, found 1016.4928.

**Tert-butyl (2-(4-((S)-13-((2S,4R)-4-hydroxy-2-((4-(4-methylthiazol-5-yl)benzyl)carbamoyl)pyrrolidine-1-carbonyl)-14,14-dimethyl-11-oxo-3,6,9-trioxa-12-azapentadecanamido)benzamido)-4-(thiophen-2-yl)phenyl)carbamate (27j):** Following general method G, **27j** was obtained from **26j** (35.1 mg, 0.057 mmol) and **VH_032 amine** (24.0 mg, 0.048 mmol). The crude product was purified by column chromatography (0-10% MeOH in DCM) to afford **27j** (30.3 mg, 0.029 mmol, 61% yield) as a yellow tar. HRMS (ESI) m/z: [M+H]+ calculated for C₅₂H₆₄N₇O₁₁S₂: 1026.4105, found 1026.4066.

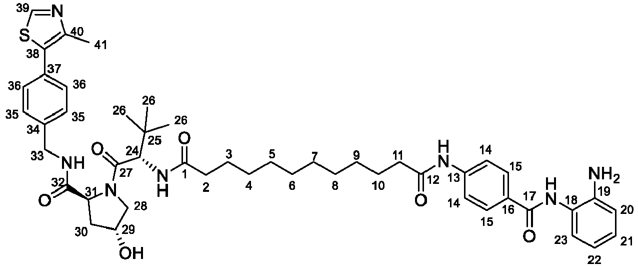

**N1-(4-((2-aminophenyl)carbamoyl)phenyl)-N12-((S)-1-((2S,4R)-4-hydroxy-2-((4-(4-methylthiazol-5-yl)benzyl)carbamoyl)pyrrolidin-1-yl)-3,3-dimethyl-1-oxobutan-2-yl)dodecanediamide (PROTAC4):** Following general method H, Boc deprotection of **27a** (37.6 mg, 0.0395 mmol) was performed to afford **PROTAC4** (25.8 mg, 0.0288 mmol, 73% yield) as a pale yellow solid. Prior to biological evaluation the PROTAC was further purified by semi-preparative HPLC (5-95% MeCN in H₂O, 260 nm, 45 min gradient). ¹H NMR (400 MHz, Methanol-d₄) δ_{H} ppm 8.86 (s, 1 H, 39-CH), 7.95 (d, *J*=8.8 Hz, 2 H, 15-CH), 7.72 (d, *J*=8.8 Hz, 2 H, 14-CH), 7.43 - 7.48 (m, 2 H, 36-CH), 7.38 - 7.42 (m, 2 H, 35-CH), 7.18 (dd, *J*=7.8, 1.3 Hz, 1 H, 23-CH), 7.07 (app. td, *J*=7.8, 1.3 Hz, 1 H, 21-CH), 6.90 (dd, *J*=7.8, 1.3 Hz, 1 H, 20-CH), 6.76 (app. td, *J*=7.8, 1.3 Hz, 1 H, 22-CH), 4.60 - 4.66 (m, 1 H, 24-CH), 4.55 - 4.60 (m, 1 H, 31-CH), 4.50 - 4.55 (m, 1 H, 33-CH), 4.47 - 4.50 (m, 1 H, 29-CH), 4.31 - 4.39 (m, 1 H, 33-CH), 3.86 - 3.93 (m, 1H, 28-CH), 3.76 - 3.83 (m, 1H, 28-CH), 2.47 (s, 3 H, 41-CH₃), 2.40 (t, *J*=7.5 Hz, 2 H, 11-CH₂), 2.18 - 2.33 (m, 3 H, 2-CH₂,30-CH), 2.03 - 2.12 (m, 1 H, 30-CH), 1.70 (quin, *J*=7.5 Hz, 2 H, 10-CH₂), 1.53 - 1.64 (m, 2 H, 3-CH₂), 1.28 - 1.41 (m, 12 H, (4-9)-CH₂), 1.03 (s, 9 H, 26-CH₃). HRMS (ESI) m/z: [M+H]⁺ calculated for C₄₇H₆₂N₇O₆S: 852.4476, found 852.4482.

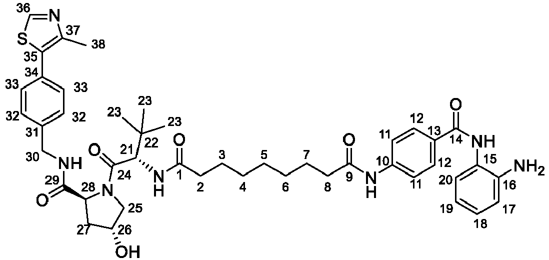

**N1-(4-((2-aminophenyl)carbamoyl)phenyl)-N9-((S)-1-((2S,4R)-4-hydroxy-2-((4-(4-methylthiazol-5-yl)benzyl)carbamoyl)pyrrolidin-1-yl)-3,3-dimethyl-1-oxobutan-2-yl)nonanediamide (PROTAC6):** Following general method H, Boc deprotection of **27b** (37.6 mg, 0.0395 mmol) was performed to afford **PROTAC6** (37.0 mg, 0.044 mmol, 99% yield) as an off-white solid. Prior to biological evaluation the PROTAC was further purified by semi-preparative HPLC (5-95% MeCN in H₂O, 260 nm, 45 min gradient). ¹H NMR (400 MHz, Methanol-d₄) δ_{H} ppm 8.86 (s, 1 H, 36-CH), 7.95 (d, *J*=8.7 Hz, 2 H, 12-CH), 7.71 (d, *J*=8.7 Hz, 2 H, 11-CH), 7.45 (d, *J*=8.4 Hz, 2 H, 33-CH), 7.40 (d, *J*=8.4 Hz, 2 H, 32-CH), 7.18 (app. dd, *J*=7.8, 1.3 Hz, 1 H, 20-CH), 7.07 (app. td, *J*=7.8, 1.3 Hz, 1 H, 18-CH), 6.90 (app. dd, *J*=7.8, 1.3 Hz, 1 H, 17-CH), 6.76 (app. td, *J*=7.8, 1.3 Hz, 1H, 19-CH), 4.63 (s, 1 H, 21-CH), 4.55 - 4.61 (m, 1 H, 28-CH), 4.50 - 4.55 (m, 1 H, 30-CH), 4.47 - 4.50 (m, 1 H, 29-CH), 4.31- 4.38 (m, 1 H, 30-CH), 3.87 - 3.94 (m, 1 H, 25-CH), 3.76 - 3.83 (m, 1 H, 25-CH), 2.46 (s, 3 H, 38-CH₃), 2.39 (t, *J*=7.5 Hz, 2 H, 8-CH₃), 2.17 - 2.33 (m, 3 H, 2-CH₂,27-CH), 2.03 - 2.11 (m, 1 H, 27-CH), 1.70 (quin, *J*=7.5 Hz, 2 H, 7-CH₂), 1.61 (quin, *J*=6.9 Hz, 2 H, 3-CH₂), 1.27 - 1.44 (m, 6 H, (4-6)-CH₂), 1.03 (s, 9 H, 23-CH₃). HRMS (ESI) m/z: [M+H]⁺ calculated for C₄₄H₅₆N₇O₆S: 810.4013, found 810.4005.

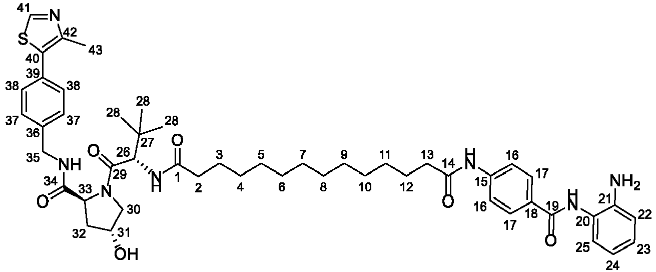

**N1-(4-((2-aminophenyl)carbamoyl)phenyl)-N14-((S)-1-((2S,4R)-4-hydroxy-2-((4-(4-methylthiazol-5-yl)benzyl)carbamoyl)pyrrolidin-1-yl)-3,3-dimethyl-1-oxobutan-2-yl)tetradecanediamide (PROTAC34):** Following general method H, Boc deprotection of **27c** (62.5 mg, 0.064 mmol) was performed to afford **PROTAC34** (51.6 mg, 0.058 mmol, 91% yield) as a white solid. ¹H NMR (400 MHz, Methanol-*d₄*) δ_{H} ppm 8.86 (s, 1 H, 41, CH), 7.95 (d, *J*=8.7 Hz, 2 H, 17-CH), 7.72 (d, *J*=8.7 Hz, 2 H, 16-CH), 7.43 - 7.47 (m, 2 H, 38-CH), 7.38 - 7.43 (m, 2 H, 37-CH), 7.18 (dd, *J*=7.8, 1.3 Hz, 1H, 25-CH), 7.06 (app. td, *J*=7.8, 1.3 Hz, 1 H, 23-CH), 6.90 (dd, *J*=7.8, 1.3 Hz, 1 H, 22-CH), 6.76 (app. td, *J*=7.8, 1.3 Hz, 1 H, 24-CH), 4.63 (s, 1 H, 26-CH), 4.55 - 4.60 (m, 1 H, 33-CH), 4.52 (d, *J*=15.5 Hz, 1 H, 35-CH), 4.46 - 4.50 (m, 1 H, 31-CH), 4.34 (d, *J*=15.5 Hz, 1 H, 35-CH), 3.86 - 3.93 (m, 1 H, 30-CH), 3.75 - 3.82 (m, 1 H, 30-CH), 2.46 (s, 3 H, 43-CH₃), 2.39 (t, *J*=7.4 Hz, 2 H, 13-CH₂), 2.17 - 2.32 (m, 3 H, 2-CH₂,32-CH), 2.03 - 2.11 (m, 1 H, 32-CH), 1.70 (quin, *J*=7.4 Hz, 2 H, 12-CH₂), 1.52 - 1.65 (m, 2 H, 3-CH₂), 1.27 - 1.39 (m, 16 H, (4-11)-CH₂), 1.03 (s, 9 H, 28-CH₃). HRMS (ESI) m/z: [M+H]⁺ calculated for C₄₉H₆₆N₇O₆S: 880.4795, found 880.4762.

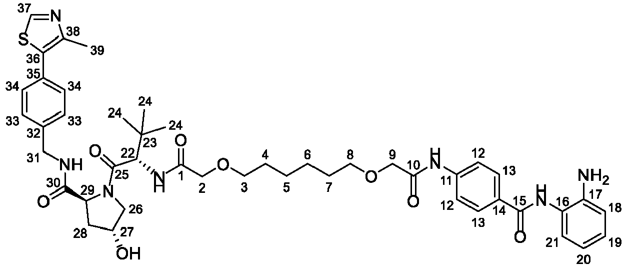

**(2S,4R)-1-((S)-2-(2-((6-(2-((4-((2-aminophenyl)carbamoyl)phenyl)amino)-2-oxoethoxy)hexyl) oxy)acetamido)-3,3-dimethylbutanoyl)-4-hydroxy-N-(4-(4-methylthiazol-5-yl)benzyl)pyrrolidine -2-carboxamide (PROTAC12):** Following general method H, Boc deprotection **of 27d** (86.3 mg, 0.090 mmol) was performed to afford **PROTAC12** (71.7 mg, 0.083 mmol, 90% yield) as a pale yellow solid. Prior to biological evaluation the PROTAC was further purified by semi-preparative HPLC (5-95% MeCN in H₂O, 260 nm, 45 min gradient). ¹H NMR (400 MHz, Methanol-*d₄*) δ_{H} ppm 8.85 (s, 1H, 37-CH), 7.96 (d, *J*=8.6 Hz, 2 H, 13-CH), 7.75 (d, *J*=8.6 Hz, 2 H, 12-CH), 7.42 - 7.45 (m, 2 H, 34-CH), 7.37 - 7.41 (m, 2 H, 33-CH), 7.18 (dd, *J*=7.7, 1.1 Hz, 1 H, 21-CH), 7.07 (td, *J*=7.7, 1.1 Hz, 1 H, 19-CH), 6.89 (dd, *J*=7.7, 1.1 Hz, 1 H, 18-CH), 6.76 (td, *J*=7.7, 1.1 Hz, 1 H, 20-CH), 4.69 (s, 1 H, 22-CH), 4.55 - 4.62 (m, 1 H, 29-CH), 4.45 - 4.55 (m, 2 H, 27,31-CH), 4.31 - 4.38 (m, 1 H, 31-CH), 4.05 - 4.11 (m, 2 H, 9-CH₂), 3.95 - 4.00 (m, 1H, 2-CH), 3.89 - 3.94 (m, 1 H, 2-CH), 3.83 - 3.89 (m, 1 H, 26-CH), 3.75 - 3.82 (m, 1 H, 26-CH), 3.53 - 3.62 (m, 4 H, 3,8-CH₂), 2.46 (s, 3 H, 39-CH₃), 2.18 - 2.27 (m, 1H, 28-CH), 2.03 - 2.13 (m, 1 H, 28-CH), 1.62 - 1.73 (m, 4 H, 4,7-CH₂), 1.42 - 1.52 (m, 4 H, 5,6-CH₂), 1.03 (s, 9 H, 24-CH₃). HRMS (ESI) m/z: [M+H]⁺ calculated for C₄₅H₅₈N₇O₈S: 856.4068, found 856.4064.

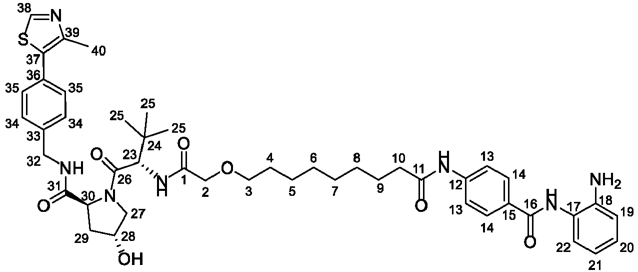

### (2S,4R)-1-((S)-2-(2-((9-((4-((2-aminophenyl)carbamoyl)phenyl)amino)-9-oxononyl)oxy)acetamid o)-3,3-dimethylbutanoyl)-4-hydroxy-N-(4-(4-methylthiazol-5-yl)benzyl)pyrrolidine-2-carboxamide (PROTAC13):

Following general method H, Boc deprotection of **27e** (66.9 mg, 0.070 mmol) was performed to afford **PROTAC13** (52.5 mg, 0.060 mmol, 86% yield) as a pale yellow solid. Prior to biological evaluation the PROTAC was further purified by semi-preparative HPLC (5-95% MeCN in H₂O, 260 nm, 45 min gradient). ¹H NMR (400 MHz, Methanol-*d₄)* δ_{H} ppm 8.85 (s, 1H, 38-CH), 7.94 (d, *J*=8.7 Hz, 2 H, 14-CH), 7.72 (d, *J*=8.7 Hz, 2 H, 13-CH), 7.43 - 7.47 (m, 2 H, 35-CH), 7.37 - 7.42 (m, 2 H, 34-CH), 7.17 (dd, *J*=7.7, 1.1 Hz, 1 H, 22-CH), 7.07 (app. td, *J*=7.7, 1.1 Hz, 1H, 20-CH), 6.89 (dd, *J*=7.7, 1.1 Hz, 1 H, 19-CH), 6.76 (app. td, *J*=7.7, 1.1 Hz, 1 H, 21-CH), 4.69 (s, 1 H, 23-CH), 4.56 - 4.63 (m, 1H, 30-CH), 4.47 - 4.55 (m, 2 H, 28,32-CH), 4.35 (d, *J*=15.5 Hz, 1 H, 32-CH), 3.94 - 3.99 (m, 1 H, 2-CH), 3.89 - 3.94 (m, 1 H, 2-CH), 3.84 - 3.89 (m, 1 H, 27-CH), 3.76 - 3.82 (m, 1H, 27-CH), 3.53 (t, *J*=6.3 Hz, 2 H, 3-CH₂), 2.46 (s, 3 H, 40-CH₃), 2.37 (t, *J*=7.5 Hz, 2 H, 10-CH₂), 2.19 - 2.27 (m, 1 H, 29-CH), 2.04 - 2.12 (m, 1 H, 29-CH), 1.59 - 1.71 (m, 4 H, 4,9-CH₂), 1.33 - 1.43 (m, 8 H, (5-8)-CH₂), 1.03 (s, 9 H, 25-CH₃). HRMS (ESI) m/z: [M+H]⁺ calculated for C₄₆H₆₀N₇O₇S: 854.4275, found 854.4277.

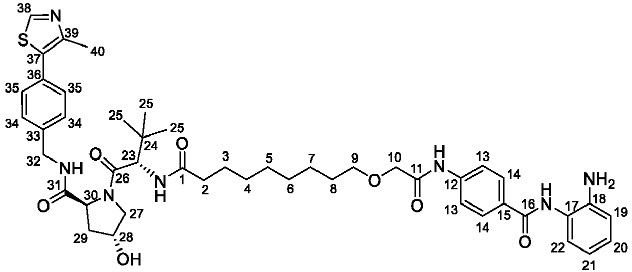

### (2S,4R)-1-((S)-2-(9-(2-((4-((2-aminophenyl)carbamoyl)phenyl)amino)-2-oxoethoxy)nonanamido) -3,3-dimethylbutanoyl)-4-hydroxy-N-(4-(4-methylthiazol-5-yl)benzyl)pyrrolidine-2-carboxamide (PROTAC14):

Following general method H, Boc deprotection of **27f** (60.2 mg, 0.063 mmol) was performed to afford **PROTAC14** (50.6 mg, 0.059 mmol, 93% yield) as a pale yellow solid. Prior to biological evaluation the PROTAC was further purified by semi-preparative HPLC (5-95% MeCN in H₂O, 260 nm, 45 min gradient). ¹H NMR (400 MHz, Methanol-*d₄*) δ_{H} ppm 8.86 (s, 1 H, 38-CH), 7.98 (d, *J*=8.7 Hz, 2 H, 14-CH), 7.77 (d, *J*=8.7 Hz, 2 H, 13-CH), 7.45 (d, *J*=8.3 Hz, 2 H, 35-CH), 7.41 (d, *J*=8.3 Hz, 2 H, 34-CH), 7.18 (dd, *J*=7.6, 1.3 Hz, 1 H, 22-CH), 7.07 (app. td, *J*=7.6, 1.3 Hz, 1 H, 20-CH), 6.90 (dd, *J*=7.6, 1.3 Hz, 1H, 19-CH), 6.77 (app. td, *J*=7.6, 1.3 Hz, 1 H, 21-CH), 4.63 (s, 1 H, 23-CH), 4.56 - 4.59 (m, 1 H, 30-CH), 4.52 (d, *J*=15.5 Hz, 1 H, 32-CH), 4.46 - 4.50 (m, 1 H, 28-CH), 4.35 (d, *J*=15.5 Hz, 1 H, 32-CH), 4.09 (s, 2 H, 10-CH₂), 3.86 - 3.92 (m, 1 H, 27-CH), 3.76 - 3.82 (m, 1 H, 27-CH), 3.60 (t, *J*=6.6 Hz, 2 H, 9-CH₂), 2.47 (s, 3 H, 40-CH₃), 2.18 - 2.32 (m, 3 H, 2-CH₂,29-CH), 2.03 - 2.12 (m, 1 H, 29-CH), 1.68 (quin, *J*=6.6 Hz, 2 H, 8-CH₂), 1.56 - 1.64 (m, 2 H, 3-CH₂), 1.32 - 1.45 (m, 8 H, (4-7)-CH₂), 1.03 (s, 9 H, 25-CH₃). HRMS (ESI) m/z: [M+H]⁺ calculated for C₄₆H₆₀N₇O₇S: 854.4275, found 854.4268.

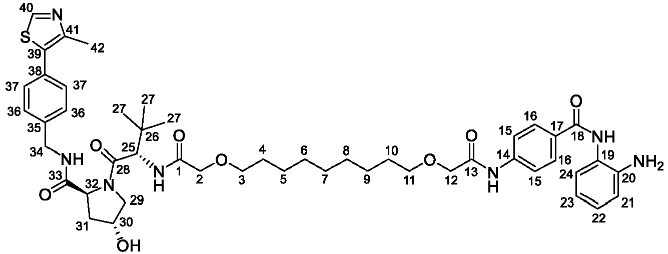

### (2S,4R)-1-((S)-2-(2-((9-(2-((4-((2-aminophenyl)carbamoyl)phenyl)amino)-2-oxoethoxy)nonyl)oxy )acetamido)-3,3-dimethylbutanoyl)-4-hydroxy-N-(4-(4-methylthiazol-5-yl)benzyl)pyrrolidine-2-carboxamide (PROTAC16):

Following general method H, Boc deprotection **of 27g** (66.7 mg, 0.067 mmol) was performed to afford **PROTAC16** (58.2 mg, 0.064 mmol, 96% yield) as a pale yellow solid. Prior to biological evaluation the PROTAC was further purified by semi-preparative HPLC (5-95% MeCN in H₂O, 260 nm, 45 min gradient). ¹H NMR (400 MHz, Methanol-*d₄*) δ_{H} ppm 8.86 (s, 1 H, 40-CH), 7.97 (d, *J*=8.6 Hz, 2 H, 16-CH), 7.76 (d, *J*=8.6 Hz, 2 H, 15-CH), 7.45 (d, *J*=8.3 Hz, 2 H, 37-CH), 7.39 (d, J=8.3 Hz, 2 H, 36-CH), 7.18 (dd, *J*=7.7, 1.1 Hz, 1 H, 24-CH), 7.07 (app. td, *J*=7.7, 1.1 Hz, 1H, 22-CH), 6.90 (dd, *J*=7.7, 1.1 Hz, 1 H, 21-CH), 6.76 (app. td, *J*=7.7, 1.1 Hz, 1 H, 23-CH), 4.69 (s, 1 H, 25-CH), 4.56 - 4.61 (m, 1H, 32-CH), 4.53 (d, *J*=15.5 Hz, 1 H, 34-CH), 4.46 - 4.50 (m, 1 H, 30-CH), 4.34 (d, *J*=15.5 Hz, 1 H, 34-CH), 4.07 (s, 2 H, 12-CH₂), 3.97 (d, *J*=15.4 Hz, 1 H, 2-CH₂), 3.92 (d, *J*=15.4 Hz, 1H, 2-CH₂), 3.83 - 3.90 (m, 1 H, 29-CH), 3.75 - 3.82 (m, 1 H, 29-CH), 3.50 - 3.59 (m, 4 H, 3,11-CH₂), 2.46 (s, 3 H, 42-CH₃), 2.17 - 2.26 (m, 1 H, 31-CH), 2.03 - 2.11 (m, 1 H, 31-CH), 1.59 - 1.69 (m, 4 H, 4,10-CH₂), 1.32 - 1.44 (m, 10 H, (5-9)-CH₂), 1.03 (s, 9 H, 27-CH₃). HRMS (ESI) m/z: [M+H]⁺ calculated for C₄₈H₆₄N₇O₈S: 898.4537, found 898.4531.

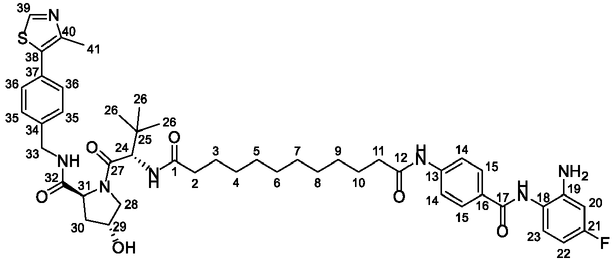

**N1-(4-((2-amino-4-fluorophenyl)carbamoyl)phenyl)-N12-((S)-1-((2S,4R)-4-hydroxy-2-((4-(4-methylthiazol-5-yl)benzyl)carbamoyl)pyrrolidin-1-yl)-3,3-dimethyl-1-oxobutan-2-yl)dodecanediamide (PROTAC17):** Following general method H, Boc deprotection of **27h** (58.7 mg, 0.061 mmol) was performed to afford **PROTAC17** (49.2 mg, 0.056 mmol, 92% yield) as a pale yellow solid. ¹H NMR (400 MHz, Methanol-d₄) δ_{H} ppm 8.86 (s, 1H, 39-CH), 7.94 (d, *J*=8.7 Hz, 2 H, 15-CH), 7.72 (d, *J*=8.7 Hz, 2 H, 14-CH), 7.43 - 7.47 (m, 2 H, 36-CH), 7.37 - 7.42 (m, 2 H, 35-CH), 7.11 (dd, *J_{HH}*=8.6, *J_{HF}*=6.0 Hz, 1 H, 23-CH), 6.58 (dd, *J_{HF}*=10.7*, J_{HH}*=2.8 Hz, 1 H, 20-CH), 6.41 (app. td, *J_{HF}*=8.6, *J_{HH}* =8.6, 2.8 Hz, 1 H, 22-CH), 4.63 (s, 1 H, 24-CH), 4.55 - 4.60 (m, 1 H, 31-CH), 4.52 (d, *J*=15.5 Hz, 1 H, 33-CH), 4.47 - 4.50 (m, 1 H, 29-CH), 4.35 (d, *J*=15.5 Hz, 1 H, 33-CH), 3.86 - 3.93 (m, 1 H, 28-CH), 3.76 - 3.82 (m, 1 H, 28-CH), 2.46 (s, 3 H, 41-CH₃), 2.39 (t, *J*=7.5 Hz, 2 H, 11-CH₂), 2.17 - 2.31 (m, 3 H, 2-CH₂,30-CH), 2.03 - 2.12 (m, 1 H, 30-CH), 1.69 (quin, *J*=7.5 Hz, 2 H, 10-CH₂), 1.53 - 1.63 (m, 2 H, 3-CH₂), 1.29 - 1.37 (m, 12 H, (4-9)-CH₂), 1.03 (s, 9 H, 26-CH₃). HRMS (ESI) m/z: [M+H]⁺ calculated for C₄₇H₆₁FN₇O₆S: 870.4388, found 870.4376.

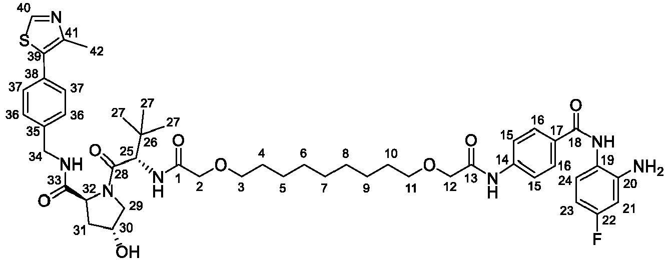

**(2S,4R)-1-((S)-2-(2-((9-(2-((4-((2-amino-4-fluorophenyl)carbamoyl)phenyl)amino)-2-oxoethoxy)nonyl) oxy)acetamido)-3,3-dimethylbutanoyl)-4-hydroxy-N-(4-(4-methylthiazol-5-yl)benzyl)pyrrolidine-2-carboxamide (PROTAC20):** Following general method H, Boc deprotection of **27i** (70.8 mg, 0.070 mmol) was performed to afford **PROTAC20** (63.3 mg, 0.068, 98% yield) as a pale brown solid. ¹H NMR (400 MHz, Methanol-*d₄*) δ_{H} ppm 8.86 (s, 1 H, 40-CH), 7.96 (d, *J*=8.7 Hz, 2 H, 16-CH), 7.75 (d, *J*=8.7 Hz, 2 H, 15-CH), 7.42 - 7.47 (m, 2 H, 37-CH), 7.37 - 7.42 (m, 2 H, 36-CH), 7.11 (dd, *J_{HH}*=8.6, *J_{HF}*=6.1 Hz, 1 H, 24-CH), 6.58 (dd, *J_{HF}*=10.7*, J_{HH}*=2.8 Hz, 1 H, 20-CH), 6.41 (td, *J_{HF}*=8.6, *J_{HH}*=8.6, 2.8 Hz, 1 H, 23-CH), 4.68 (s, 1H, 25-CH), 4.59 (dd, *J*=9.0, 7.8 Hz, 1 H, 32-CH), 4.52 (d, *J*=15.6 Hz, 1 H, 34-CH), 4.47 - 4.50 (m, 1 H, 30-CH), 4.34 (d, *J*=15.6 Hz, 1 H, 34-CH), 4.07 (s, 2 H, 12-CH₂), 3.95 (d, *J*=15.4 Hz, 1H, 2-CH₂), 3.94 (d, *J*=15.4 Hz, 1 H, 2-CH₂), 3.84 - 3.89 (m, 1 H, 29-CH), 3.74 - 3.82 (m, 1 H, 29-CH), 3.50 - 3.59 (m, 4 H, (3,11)-CH₂), 2.46 (s, 3 H, 42-CH₃), 2.18 - 2.27 (m, 1 H, 31-CH), 2.02 - 2.12 (m, 1H, 31-CH), 1.58 - 1.68 (m, 4 H, (4,10)-CH₂), 1.32 - 1.43 (m, 12 H, (5-9)-CH₂), 1.03 (s, 9 H, 27-CH₃). HRMS (ESI) m/z: [M+H]⁺ calculated for C₄₈H₆₃FN₇O₈S: 916.4443, found 916.4426.

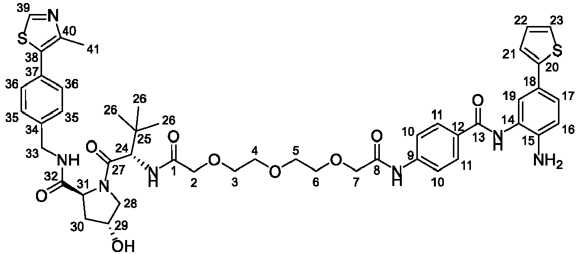

**(2S,4R)-1-((S)-14-((4-((2-amino-5-(thiophen-2-yl)phenyl)carbamoyl)phenyl)amino)-2-(tert-butyl)-4,14-dioxo-6,9,12-trioxa-3-azatetradecanoyl)-4-hydroxy-N-(4-(4-methylthiazol-5-yl)benzyl)pyrrolidine-2-carboxamide (PROTAC22):** Following general method H, Boc deprotection of **27j** (30.3 mg, 0.029 mmol) was performed to afford **PROTAC22** (26.8 mg, 0.029 mmol, 99% yield) as a pale yellow solid. ¹H NMR (400 MHz, Methanol-*d₄*) δ_{H} ppm 8.84 (s, 1 H, 39-CH), 7.98 (d, *J*=8.7 Hz, 2 H, 11-CH), 7.75 (d, *J*=8.7 Hz, 2 H, 10-CH), 7.49 (d, *J*=2.1 Hz, 1 H, 19-CH), 7.40 - 7.44 (m, 2 H, 36-CH), 7.36 - 7.39 (m, 2 H, 35-CH), 7.34 (dd, *J*=8.3, 2.1 Hz, 1H, 17-CH), 7.22 (d, *J*=5.0 Hz, 1 H, 23-CH), 7.19 (d, *J*=3.7 Hz, 1 H, 21-CH), 7.01 (dd, *J*=5.0, 3.7 Hz, 1H, 22-CH), 6.89 (d, *J*=8.3 Hz, 1 H, 16-CH), 4.68 (s, 1 H, 24-CH), 4.54 - 4.60 (m, 1 H, 31-CH), 4.51 (d, *J*=15.5 Hz, 1 H, 33-CH), 4.46 - 4.49 (m, 1 H, 29-CH), 4.31 (d, *J*=15.5 Hz, 1 H, 33-CH), 4.15 (d, *J*=15.8 Hz, 1 H, 7-CH), 4.09 (d, *J*=15.8 Hz, 1 H, 7-CH), 4.02 (d, *J*=15.6 Hz, 1 H, 2-CH), 3.91 (d, *J*=15.6 Hz, 1H, 2-CH), 3.82 - 3.87 (m, 1H, 28-CH), 3.69 - 3.81 (m, 9 H, 28-CH,(3-6)-CH₂), 2.45 (s, 3 H, 41-CH₃), 2.16 - 2.24 (m, 1 H, 30-CH), 2.03 - 2.11 (m, 1 H, 30-CH), 1.02 (s, 9 H, 26-CH3). HRMS (ESI) m/z: [M+H]⁺ calculated for C₄₇H₅₆N₇O₉S₂: 926.3581, found 926.3556.

### Synthesis of HDAC PROTACs Containing Alternative VHL Ligands

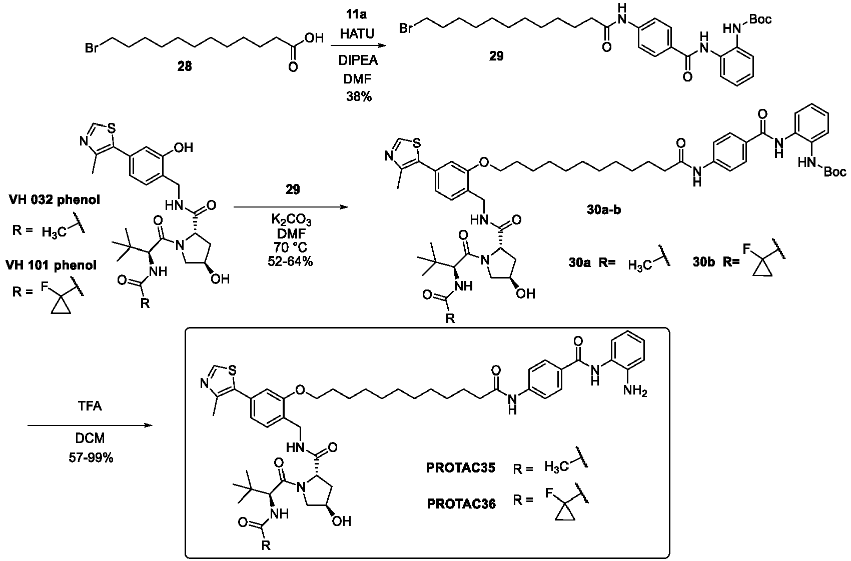

**Tert-butyl (2-(4-(12-bromododecanamido)benzamido)phenyl)carbamate (29):** Following general method E, **29** was obtained from **28** (332.6 mg, 1.19 mmol) and **11a** (300.0 mg, 0.92 mmol). The crude product was purified by column chromatography (0-50% EtOAc in hexane) to give **29** (207.0 mg, 0.35 mmol, 38% yield) as a white solid. HRMS (ESI) m/z: [M+H]⁺ calculated for C₃₀H₄₃⁸¹BrN₃O₄: 590.2416, found 590.2411.

**Tert-butyl (2-(4-(12-(2-(((2S,4R)-1-((S)-2-acetamido-3,3-dimethylbutanoyl)-4-hydroxypyrrolidine-2-carboxamido)methyl)-5-(4-methylthiazol-5-yl)phenoxy)dodecanamido)benzamido)phenyl)carbamate (30a):** Following general method I, **30a** was obtained from (2S,4R)-1-((S)-2-acetamido-3,3-dimethylbutanoyl)-4-hydroxy-N-(2-hydroxy-4-(4-methylthiazol-5-yl)benzyl)pyrrolidine-2-carboxamide (**VH 032 phenol,** 17.2 mg, 0.034 mmol) and **29** (20.0 mg, 0.034 mmol). The crude product was purified by column chromatography (0-10% MeOH in DCM) to afford **30a** (17.8 mg, 0.018 mmol, 52% yield) as a white solid. HRMS (ESI) m/z: [M+H]⁺ calculated for C₅₄H₇₄N₇O₉S: 996.5269 , found 996.5239.

**Tert-butyl (2-(4-(12-(2-(((2S,4R)-1-((S)-2-(1-fluorocyclopropane-1-carboxamido)-3,3-dimethylbuta noyl)-4-hydroxypyrrolidine-2-carboxamido)methyl)-5-(4-methylthiazol-5-yl)phenoxy)dodecanamido) benzamido)phenyl)carbamate (30b):** Following general method I, **30b** was obtained from (2S,4R)-1-((S)-2-(1-fluorocyclopropane-1-carboxamido)-3,3-dimethylbutanoyl)-4-hydroxy-N-(2-hydroxy-4-(4-methylthiazol-5-yl)benzyl)pyrrolidine-2-carboxamide (**VH 101 phenol,** 18.6 mg, 0.034 mmol) and **29** (20.0 mg, 0.034 mmol). The crude product was purified by column chromatography (1-10% MeOH in DCM) to afford **30b** (24.2 mg, 0.022 mmol, 64% yield) as a white solid. HRMS (ESI) m/z: [M+H]⁺ calculated for C₅₆H₇₅FN₇O₉S: 1040.5331, found 1040.5304.

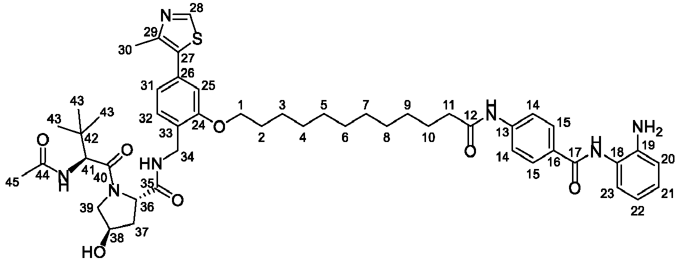

**(2S,4R)-1-((S)-2-acetamido-3,3-dimethylbutanoyl)-N-(2-((12-((4-((2-aminophenyl)carbamoyl) phenyl)amino)-12-oxododecyl)oxy)-4-(4-methylthiazol-5-yl)benzyl)-4-hydroxypyrrolidine-2-carboxamide (PROTAC35):** Following general method H, Boc deprotection of 30a (17.8 mg, 0.018 mmol) was performed to afford **PROTAC35** (16.1 mg, 0.018 mmol, 99% yield) as a white solid. ¹H NMR (400 MHz, Methanol-*d₄*) δ_{H} ppm 8.86 (s, 1 H, 28-CH), 7.95 (d, *J*=8.7 Hz, 2 H, 15-CH), 7.72 (d, *J*=8.7 Hz, 2 H, 14-CH), 7.47 (d, *J*=8.2 Hz, 1 H, 32-CH), 7.17 (dd, *J*=7.7*,* 1.3 Hz, 1 H, 23-CH), 7.07 (app. td, *J*=7.7, 1.3 Hz, 1 H, 21-CH), 6.94 - 7.00 (m, 2 H, 25,31-CH), 6.90 (dd, *J*=7.7*,* 1.3 Hz, 1 H, 20-CH), 6.76 (app. td, *J*=7.7*,* 1.3 Hz, 1 H, 22-CH), 4.57 - 4.65 (m, 2 H, 36,41-CH), 4.48 - 4.52 (m, 1 H, 38-CH), 4.46 (d, *J*=16.1 Hz, 1 H, 34-CH), 4.39 (d, *J*=16.1 Hz, 1 H, 34-CH), 4.05 (t, *J*=6.3 Hz, 2 H, 1-CH2), 3.86 - 3.92 (m, 1 H, 39-CH2), 3.75 - 3.81 (m, 1 H, 39-CH2), 2.48 (s, 3 H, 30-CH₃), 2.40 (t, J=7.5 Hz, 2 H, 11-CH₂), 2.17 - 2.25 (m, 1 H, 37-CH), 2.07 - 2.15 (m, 1 H, 37-CH), 1.99 (s, 3 H, 45-CH₃), 1.78 - 1.87 (m, 2 H, 2-CH₂), 1.71 (quin, *J*=7.3 Hz, 2 H, 10-CH₂), 1.46 - 1.56 (m, 2 H, 3-CH₂), 1.32 - 1.42 (m, 12 H, (4-9)-CH₂), 1.02 (s, 9 H, 43-CH₃). HRMS (ESI) m/z: [M+H]⁺ calculated for C₄₉H₆₆N₇O₇S: 896.4744, found 896.4744.

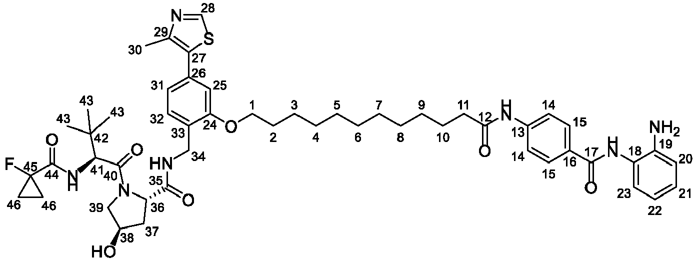

**(2S,4R)-N-(2-((12-((4-((2-aminophenyl)carbamoyl)phenyl)amino)-12-oxododecyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (PROTAC36):** Following general method H, Boc deprotection of **30b** (24.2 mg, 0.022 mmol) was performed followed by purification by column chromatography (2-5% MeOH in DCM) to afford **PROTAC36** (11.7 mg, 0.012 mmol, 57% yield) as a white solid. ¹H NMR (400 MHz, Methanol-*d₄*) δ_{H} ppm 8.86 (s, 1 H, 28-CH), 7.95 (d, *J*=8.7 Hz, 2 H, 15-CH), 7.72 (d, *J*=8.7 Hz, 2 H, 14-CH), 7.47 (d, *J*=7.7 Hz, 1 H, 32-CH), 7.17 (dd, *J*=7.8, 1.3 Hz, 1 H, 23-CH), 7.06 (app. td, *J*=7.8, 1.3 Hz, 1 H, 21-CH), 6.96 - 7.02 (m, 2 H, 25,31-CH), 6.90 (dd, *J*=7.8, 1.3 Hz, 1 H, 20-CH), 6.76 (app. td, *J*=7.8, 1.3 Hz, 1 H, 22-CH), 4.74 (d, *J*_{HF}=0.8 Hz, 1 H, 41-CH), 4.60 - 4.67 (m, 1 H, 36-CH), 4.49 - 4.53 (m, 1 H, 38-CH), ₄.₄₇ (d, *J*=16.0 Hz, 1 H, 34-CH), 4.39 (d, *J*=16.0 Hz, 1 H, 34-CH), 4.06 (t, *J*=6.3 Hz, 2 H, 1-CH2), 3.82 - 3.88 (m, 1 H, 39-CH), 3.76 - 3.81 (m, 1 H, 39-CH), 2.48 (s, 3 H, 30-CH₃), 2.40 (t, *J*=7.5 Hz, 2 H, 11-CH₂), 2.19 - 2.27 (m, 1 H, 37-CH), 2.09 - 2.16 (m, 1 H, 37-CH), 1.78 - 1.89 (m, 2 H, 2-CH₂), 1.64 - 1.76 (m, 2 H, 10-CH₂), 1.48 - 1.57 (m, 2 H, 3-CH₂), 1.28 - 1.40 (m, 16 H, (4-9)-CH₂,46-CH₂), 1.04 (s, 9 H, 43-CH₃). HRMS (ESI) m/z: [M+H]+ calculated for C₅₁H₆₇FN₇O₇S: 940.4807, found 940.4781.

### Synthesis of HDAC PROTACs Containing IAP Ligands

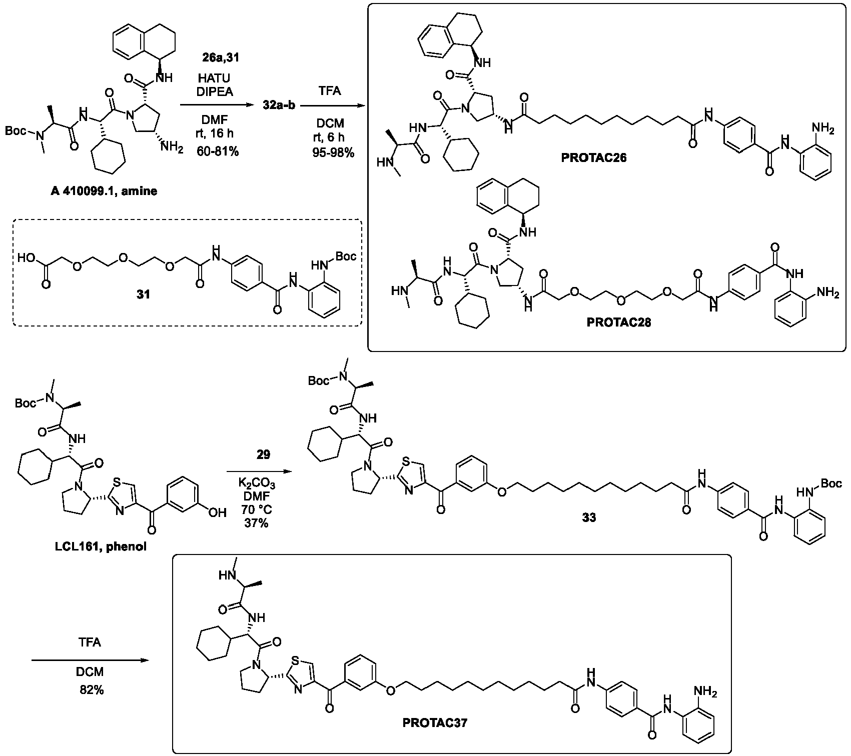

**Tert-butyl ((S)-1-(((S)-2-((2S,4S)-4-1(12-((4-((2-((tert-butoxycarbonyl)amino)phenyl)carbamoyl)phenyl) amino)-12-oxododecanamido)-2-(((R)-1,2,3,4-tetrahydronaphthalen-1-yl)carbamoyl)pyrrolidin-1-yl)-1-cyclohexyl-2-oxoethyl)amino)-1-oxopropan-2-yl)(methyl)carbamate (32a):** Following general method K, **32a** was obtained from **26a** (24.3 mg, 0.045 mmol) and A **410099.1 amine** (24.5 mg, 0.038 mmol). The crude product was purified by column chromatography (0-10% MeOH in DCM) to afford **32a** (26.1 mg, 0.023 mmol, 60% yield) as a pale yellow solid. HRMS (ESI) m/z: [M+H]⁺ calculated for C₆₂H₈₉N₈O₁₀: 1105.6702, found 1105.6704.

**Tert-butyl ((S)-1-(((S)-2-((2S,4S)-4-(2-(2-(2-(2-((4-((2-((tert-butoxycarbonyl)amino)phenyl)carbamoyl) phenyl)amino)-2-oxoethoxy)ethoxy)ethoxy)acetamido)-2-(((R)-1,2,3,4-tetrahydronaphthalen-1-yl)carbamoyl)pyrrolidin-1-yl)-1-cyclohexyl-2-oxoethyl)amino)-1-oxopropan-2-yl)(methyl)carbamate (32b):** Following general method K, **32b** was obtained from **31** (24.0 mg, 0.045 mmol) and **A 410099.1 amine** (24.5 mg, 0.038 mmol). The crude product was purified by column chromatography (0-10% MeOH in DCM) to afford **32b** (34.0 mg, 0.031 mmol, 81% yield) as a white solid. HRMS (ESI) m/z: [M+H]⁺ calculated for C₅₈H₈₁N₈O₁₃: 1097.5923, found 1097.5881.

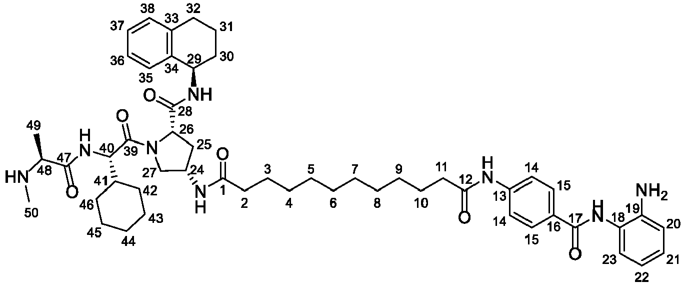

**N¹-(4-((2-aminophenyl)carbamoyl)phenyl)-N¹²-((3S,5S)-1-((S)-2-cyclohexyl-2-((S)-2-(methylamino) propanamido)acetyl)-5-(((R)-1,2,3,4-tetrahydronaphthalen-1-yl)carbamoyl)pyrrolidin-3-yl) dodecanediamide (PROTAC26):** Following general method H, dual Boc deprotection **of 32a** (26.1 mg, 0.0227 mmol) was performed to afford **PROTAC26** (21.0 mg, 0.0223 mmol, 98% yield) as a pale yellow solid. ¹H NMR (400 MHz, Methanol-*d₄*) δ_{H} ppm 7.95 (d, *J*=8.7 Hz, 2 H, 15-CH), 7.73 (d, *J*=8.7 Hz, 2 H, 14-CH), 7.35 - 7.42 (m, 1 H, 35-CH), 7.18 (dd, *J*=7.8, 1.2 Hz, 1 H, 23-CH), 7.11 - 7.15 (m, 2 H, 36,37-CH), 7.05 - 7.10 (m, 2 H, 21,38-CH), 6.90 (dd, *J*=7.8, 1.2 Hz, 1 H, 20-CH), 6.77 (app. td, *J*=7.8, 1.2 Hz, 1 H, 22-CH), 5.02 - 5.09 (m, 1 H, 29-CH), 4.41 - 4.53 (m, 3 H, 24,26,40-CH), 4.16 (dd, *J*=10.3, 6.2 Hz, 1 H, 27-CH), 3.57 (dd, *J*=10.3, 5.4 Hz, 1 H, 27-CH), 3.13 (q, *J*=6.8 Hz, 1 H, 48-CH), 2.72 - 2.84 (m, 2 H, 32-CH₂), 2.46 - 2.55 (m, 1 H, 25-CH), 2.40 (t, *J*=7.4 Hz, 2 H, 11-CH₂), 2.29 (s, 3 H, 50-CH₃), 2.19 (t, *J*=7.5 Hz, 2 H, 2-CH₂), 1.67 - 1.96 (m, 13 H, 10,30,31-CH₂,25,41,(42-46)-CH), 1.57 - 1.64 (m, 2 H, 3-CH₂), 1.32 - 1.42 (m, 12 H, (4-9)-CH₂), 1.23-1.29 (m, 3 H, (43-45)-CH), 1.20 (d, *J*=6.8 Hz, 3 H, 49-CH₃), 1.03 - 1.05 (m, 2 H, 42,46-CH). HRMS (ESI) m/z: [M+H]⁺ calculated for C₅₂H₇₃N₈O₆: 905.5653, found 905.5648.

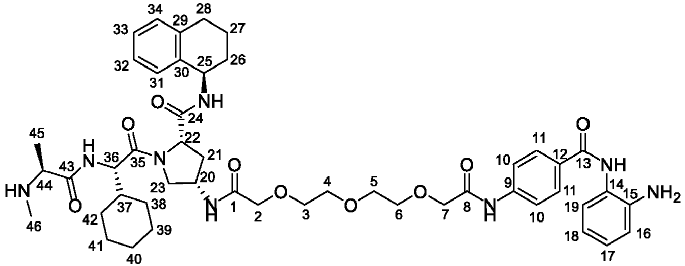

**(2S,4S)-4-(2-(2-(2-(2-((4-((2-aminophenyl)carbamoyl)phenyl)amino)-2-oxoethoxy)ethoxy)ethoxy) acetamido)-1-((S)-2-cyclohexyl-2-((S)-2-(methylamino)propanamido)acetyl)-N-((R)-1,2,3,4-tetrahydronaphthalen-1-yl)pyrrolidine-2-carboxamide (PROTAC28):** Following general method H, dual Boc deprotection **of 32b** (34.0 mg, 0.031 mmol) was performed to afford **PROTAC28** (26.8 mg, 0.029 mmol, 95% yield) as a white solid. ¹H NMR (400 MHz, Methanol-*d₄*) δ_{H} ppm 7.98 (d, *J*=8.7 Hz, 2 H, 11-CH), 7.80 (d, *J*=8.7 Hz, 2 H, 10-CH), 7.33 - 7.39 (m, 1 H, 31-CH), 7.19 (dd, *J*=7.8, 1.3 Hz, 1 H, 19-CH), 7.03 - 7.15 (m, 4 H, 17,32,33,34-CH), 6.90 (dd, *J*=7.8, 1.3 Hz, 1 H, 16-CH), 6.75 (app. td, *J*=7.8, 1.3 Hz, 1 H, 18-CH), 5.00 - 5.08 (m, 1 H, 25-CH), 4.58 - 4.65 (m, 1 H, 20-CH), 4.46 (dd, *J*=8.9, 4.7 Hz, 1 H, 22-CH), 4.43 (d, *J*=8.4 Hz, 1 H, 36-CH), 4.08 - 4.18 (m, 3 H, 7-CH₂,23-CH), 4.01 (s, 2 H, 2-CH₂), 3.79 - 3.87 (m, 2 H, PEG-CH₂), 3.70 - 3.79 (m, 6 H, PEG-CH₂ × 3), 3.62 - 3.68 (m, 1 H, 23-CH), 3.09 (q, J=6.8 Hz, 1 H, 44-CH), 2.68 - 2.85 (m, 2 H, 28-CH₂), 2.44 - 2.53 (m, 1 H, 21-CH₂), 2.26 (s, 3 H, 46-CH₃), 1.62 - 1.98 (m, 11 H, 26,27-CH₂,21,37,(38-42)-CH), 1.17 - 1.27 (m, 3 H, (39-41)-CH), 1.16 (d, J=6.9 Hz, 3 H, 45-CH₃), 0.98 - 1.13 (m, 2 H, 38,42-CH). HRMS (ESI) m/z: [M+H]+ calculated for C₄₈H₆₅N₈O₉: 897.4875, found 897.4860.

**Tert-butyl ((S)-1-(((S)-2-((S)-2-(4-(3-((12-((4-((2-((tert-butoxycarbonyl)amino)phenyl)carbamoyl) phenyl)amino)-12-oxododecyl)oxy)benzoyl)thiazol-2-yl)pyrrolidin-1-yl)-1-cyclohexyl-2-oxoethyl) amino)-1-oxopropan-2-yl)(methyl)carbamate (33):** Following general method I, **33** was obtained from tert-butyl ((S)-1-(((S)-1-cyclohexyl-2-((S)-2-(4-(3-hydroxybenzoyl)thiazol-2-yl)pyrrolidin-1-yl)-2-oxoethyl) amino)-1-oxopropan-2-yl)(methyl)carbamate (**LCL161 phenol**, 20.5 mg, 0.034 mmol) and **29** (20.0 mg, 0.034 mmol). The crude product was purified by column chromatography (1-8% MeOH in DCM) to afford **33** (14.7 mg, 0.013 mmol, 37% yield) as a white solid. HRMS (ESI) m/z: [M+H]⁺ calculated for C₆₁H₈₄N₇O₁₀S: 1106.6000, found 1106.5963.

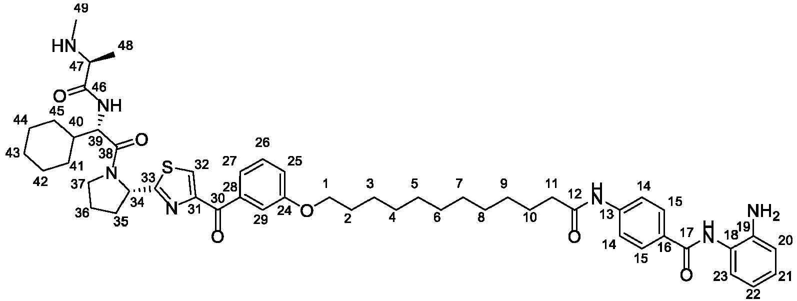

**N-(2-aminophenyl)-4-(12-(3-(2-((S)-1-((S)-2-cyclohexyl-2-((S)-2-(methylamino)propanamido)acetyl) pyrrolidin-2-yl)thiazole-4-carbonyl)phenoxy)dodecanamido)benzamide (PROTAC37):** Following general method H, dual Boc deprotection of **33** (14.7 mg, 0.013 mmol) was performed followed by purification by column chromatography (0-10% MeOH in DCM) to afford **PROTAC37** (9.4 mg, 0.0103 mmol, 82% yield) as a white solid. ¹H NMR (400 MHz, Methanol-*d₄*) δ_{H} ppm 8.32 (s, 1 H, 32-CH), 7.95 (d, *J*=8.7 Hz, 2 H, 15-CH), 7.65 - 7.76 (m, 4 H, 14,27,2₉-CH), 7.42 (app. t, *J*=8.0 Hz, 1 H, 26-CH), 7.15 - 7.22 (m, 2 H, 23,25-CH), 7.07 (app. td, *J*=7.8, 1.3 Hz, 1 H, 21-CH), 6.90 (dd, *J*=7.8, 1.3 Hz, 1 H, 20-CH), 6.77 (app. td, *J*=7.8, 1.3 Hz, 1 H, 22-CH), 5.48 (dd, *J*=7.8, 3.1 Hz, 1 H, 34-CH), 4.52 - 4.60 (m, 1 H, 39-CH), 4.04 (t, *J*=6.4 Hz, 2 H, 1-CH2), 3.95 - 4.01 (m, 1 H, 37-CH), 3.86 - 3.93 (m, 1 H, 37-CH), 3.20 (q, *J*=6.8 Hz, 1 H, 47-CH), 2.40 (t, *J*=7.5 Hz, 2 H, 11-CH₂), 2.29 - 2.37 (m, 4 H, 35-CH,49-CH₃), 2.18 - 2.28 (m, 2 H, 35,36-CH), 2.08 - 2.17 (m, 1 H, 36-CH), 1.77 - 1.85 (m, 2 H, 2-CH₂), 1.54 - 1.77 (m, 8 H, 10-CH₂,40-CH,(41-45)-CH),1.46 - 1.53 (m, 2 H, 3-CH₂), 1.32 - 1.43 (m, 12 H, (4-9)-CH₂), 1.24 (d, *J*=6.8 Hz, 3 H, 48-CH₃), 1.00 - 1.18 (m, 5 H, (41-45)-CH). HRMS (ESI) m/z: [M+H]+ calculated for C₅₁H₆₈N₇O₆S: 906.4952, found 906.4925.

### Synthesis of HDAC PROTACs Containing a CRBN Ligand

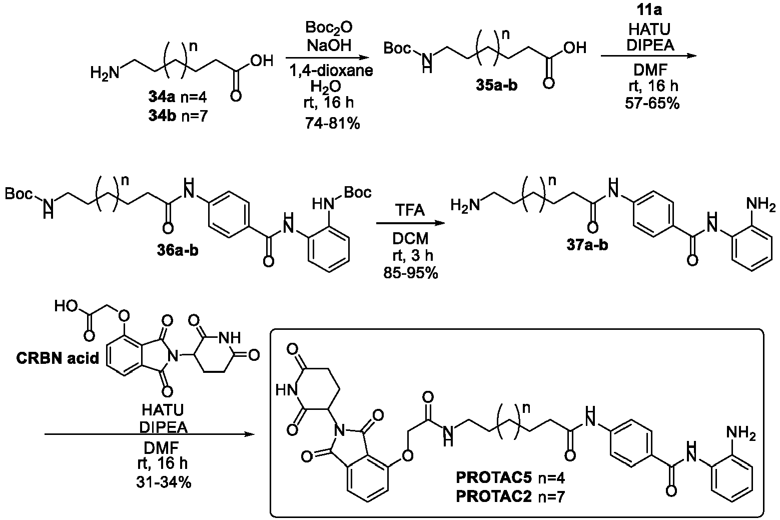

**9-((tert-butoxycarbonyl)amino)nonanoic acid (35a):** A solution of di-tert-butyldicarbonate (0.305 g, 1.40 mmol) in 1,4-dioxane/water (2:1, 2 mL) was added slowly to a solution of **34a** (0.220 g, 1.27 mmol) and NaOH (0.051 g, 1.27 mmol) in 1,4-dioxane/water (2:1, 7 mL) at 0 °C, and then the mixture was stirred at room temperature for 64 hours. The reaction mixture was concentrated in *vacuo* to afford an off-yelllow solid (0.391 g), then the basic residue redissolved in water (20 mL) and washed with EtOAc (2 × 20 mL). The aqueous phase was then acidified with 1 M HCl (ca. 2 mL) to pH 1 and extracted with EtOAc (3 × 20 mL). The organic phases were combined, dried over Na₂SO₄, filtered and concentrated *in vacuo* to afford **35a** (0.260 g, 0.94 mmol, 74% yield) as a clear yellow tar (slowly crystallised). HRMS (ESI) m/z: [M+H]⁺ calculated for C₁₄H₂₈NO₄: 274.2018, found 274.2017.

**12-((Tert-butoxycarbonyl)amino)dodecanoic acid (35b):** A solution of Boc₂O (2.23 g, 10.22 mmol) in 1,4-dioxane/water (2:1, 10 mL) was added slowly to a solution of **34b** (2.00 g, 9.29 mmol) and NaOH (0.37 g, 9.29 mmol) in 1,4-dioxane/water (2:1, 50 mL) at 0 °C, and then the mixture was stirred at room temperature for 18 hours. The reaction mixture was concentrated *in vacuo,* then the basic residue redissolved in water (100 mL) and washed with EtOAc (2 × 50 mL). The aqueous phase was then acidified with 1 M HCl (ca. 15 mL) to pH 1 and extracted with EtOAc (3 × 100 mL). The organic phases were combined, dried over Na₂SO₄, filtered and concentrated in *vacuo* to afford **35b** (2.41 g, 7.56 mmol, 81% yield) as a fluffy white solid. HRMS (ESI) m/z: [M+Na]⁺ calculated for C₁₇H₃₃NO₄Na: 338.2307, found 338.2307.

### Tert-butyl (2-(4-(9-((tert-butoxycarbonyl)amino)nonanamido)benzamido)phenyl)carbamate (36a):

Following general method E, **36a** was obtained from **35a** (247.5 mg, 0.91 mmol) and **11a** (250.0 mg, 0.76 mmol). The crude product was purified by column chromatography (0-80% EtOAc in hexane) to afford **36a** (255.8 mg, 0.43 mmol, 57% yield). HRMS (ESI) m/z: [M+H]⁺ calculated for C₃₂H₄₇N₄O₆: 583.3496, found 583.3492.

### Tert-butyl (2-(4-(12-((tert-butoxycarbonyl)amino)dodecanamido)benzamido)phenyl)carbamate (36b):

Following general method E, **36b** was obtained from **35b** (318 mg, 1.01 mmol) and **11a** (300 mg, 0.92 mmol).

The crude product was purified by column chromatography (50% EtOAc in hexane) to afford **36b** (374 mg, 0.59 mmol, 65% yield) as a pale yellow crystalline solid. HRMS (ESI) m/z: [M+Na]⁺ calculated for C₃₅H₅₂N₄O₆Na: 647.3785, found 647.3792.

**4-(9-aminononanamido)-N-(2-aminophenyl)benzamide (37a):** Following general method C, Boc deprotection of **36a** (96.0 mg, 0.165 mmol) was performed to afford **37a** (54.0 mg, 0.140 mmol, 85% yield) as a pale yellow solid. HRMS (ESI) m/z: [M+H]⁺ calculated for C₂₂H₃₁N₄O₂: 383.2447, found 383.2440.

**4-(12-Aminododecanamido)-N-(2-aminophenyl)benzamide (37b):** Following general method C, Boc deprotection of **36b** (159 mg, 0.255 mmol) was performed to afford **37b** (104 mg, 0.242 mmol, 95% yield) as a pale yellow solid. HRMS (ESI) m/z: [M+H]⁺ calculated for C₂₅H₃₇N₄O₂: 425.2917, found 425.2918.

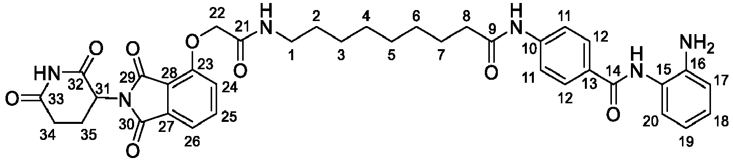

### N-(2-aminophenyl)-4-(9-(2-((2-(2,6-dioxopiperidin-3-yl)-1,3-dioxoisoindolin-4-yl)oxy)acetamido)nonan amido)benzamide (PROTAC5):

Following general method J, **PROTAC5** was obtained from **CRBN acid** (33.9 mg, 0.102 mmol) and **37a** (39.0 mg, 0.102 mmol). The crude product was purified by column chromatography (5% MeOH in DCM) to afford **PROTAC5** (22.5 mg, 0.032 mmol, 31% yield) as a pale yellow solid. ¹H NMR (400 MHz, Methanol-*d₄*) δ_{H} ppm 7.94 (d, *J*=8.7 Hz, 2 H, 12-CH), 7.79 (dd, *J*=8.4, 7.3 Hz, 1 H, 25-CH), 7.71 (d, *J*=8.7 Hz, 2 H, 11-CH), 7.52 (dd, *J*=7.3, 0.5 Hz, 1 H, 26-CH), 7.41 (dd, *J*=8.4, 0.5 Hz, 1 H, 24-CH), 7.18 (app. dd, *J*=7.7*,* 1.4 Hz, 1 H, 20-CH), 7.08 (app. td, *J*= 7.7, 1.4 Hz, 1 H, 18-CH), 6.90 (app. dd, *J*=7.7*,* 1.4 Hz, 1 H, 17-CH), 6.77 (app. td, *J*=7.7*,* 1.4 Hz, 1 H, 19-CH), 5.13 (dd, *J*=12.5, 5.5 Hz, 1 H, 31-CH), 4.75 (s, 2 H, 22-CH₂), 3.29 - 3.33 (m, 2 H, 1-CH2), 2.82 - 2.93 (m, 1 H, 34-CH), 2.66 - 2.79 (m, 2 H, 34-CH,35-CH), 2.39 (t, *J*=7.5 Hz, 2 H, 8-CH₂), 2.10 - 2.18 (m, 1 H, 35-CH), 1.70 (quin, *J*=7.1 Hz, 2 H, 7-CH₂), 1.58 (quin, *J*=7.1 Hz, 2 H, 2-CH₂), 1.35 - 1.40 (m, 8 H, (3-6)-CH₂). HRMS (ESI) m/z: [M+H]⁺ calculated for C₃₇H₄₁N₆O₈; 697.2986, found 697.2981.

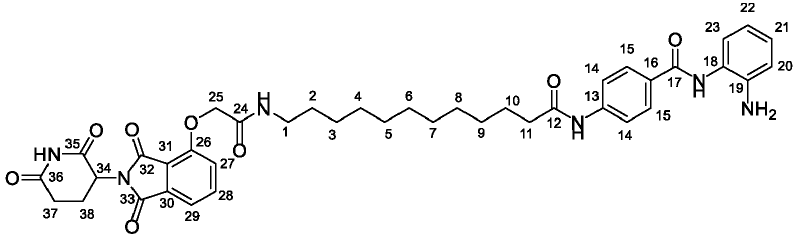

**N-(2-aminophenyl)-4-(12-(2-((2-(2,6-dioxopiperidin-3-yl)-1,3-dioxoisoindolin-4-yl)oxy)acetamido) dodecanamido)benzamide (PROTAC2):** Following general method J, **PROTAC2** was obtained from **CRBN acid** (31.6 mg, 0.095 mmol) and **37b** (40.4 mg, 0.095 mmol). The crude product was purified by column chromatography (5% MeOH in DCM) to afford **PROTAC2** (25.4 mg, 0.033 mmol, 34% yield) as a pale yellow solid. ¹H NMR (400 MHz, DMSO-*d₆*) δ_{H} ppm 11.11 (br s, 1 H, NH), 10.11 (s, 1 H, NH), 9.54 (s, 1 H, NH), 7.93 (d, *J*=8.8 Hz, 2 H, 15-CH), 7.91 (s, 1 H, NH), 7.81 (dd, *J*=8.4, 7.2 Hz, 1 H, 28-CH), 7.70 (d, *J*=8.8 Hz, 2 H, 14-CH), 7.50 (d, *J*=7.2 Hz, 1 H, 29-CH), 7.39 (d, *J*=8.4 Hz, 1 H, 27-CH), 7.15 (dd, *J*=7.7*,* 1.4 Hz, 1 H, 23-CH), 6.96 (app. td, *J*=7.7*,* 1.4 Hz, 1 H, 21-CH), 6.78 (dd, *J*=7.7*,* 1.4 Hz, 1 H, 20-CH), 6.59 (app. td, *J*=7.7*,* 1.4 Hz, 1 H, 22-CH), 5.12 (dd, *J*=12.9, 5.4 Hz, 1 H, 34-CH), 4.87 (s, 2 H, NH₂), 4.76 (s, 2 H, 25-CH₂), 3.13 (q, *J*=6.5 Hz, 2 H, 1-CH2), 2.85 - 2.95 (m, 1 H, 37-CH), 2.53 - 2.63 (m, 2 H, 37-CH,38-CH), 2.33 (t, *J*=7.4 Hz, 2 H, 11-CH₂), 2.00 - 2.07 (m, 1 H, 38-CH), 1.59 (quin, *J*=7.0 Hz, 2 H, 10-CH₂), 1.39 - 1.47 (m, 2 H, 2-CH₂), 1.23 -1.31 (m, 14 H, (3-9)-CH₂). HRMS (ESI) m/z: [M+H]⁺ calculated for C₄₀H₄₇N₆O₈: 739.3455, found 739.3455.

### Utilising Click Chemistry to Synthesize a HDAC PROTAC containing a VHL Ligand

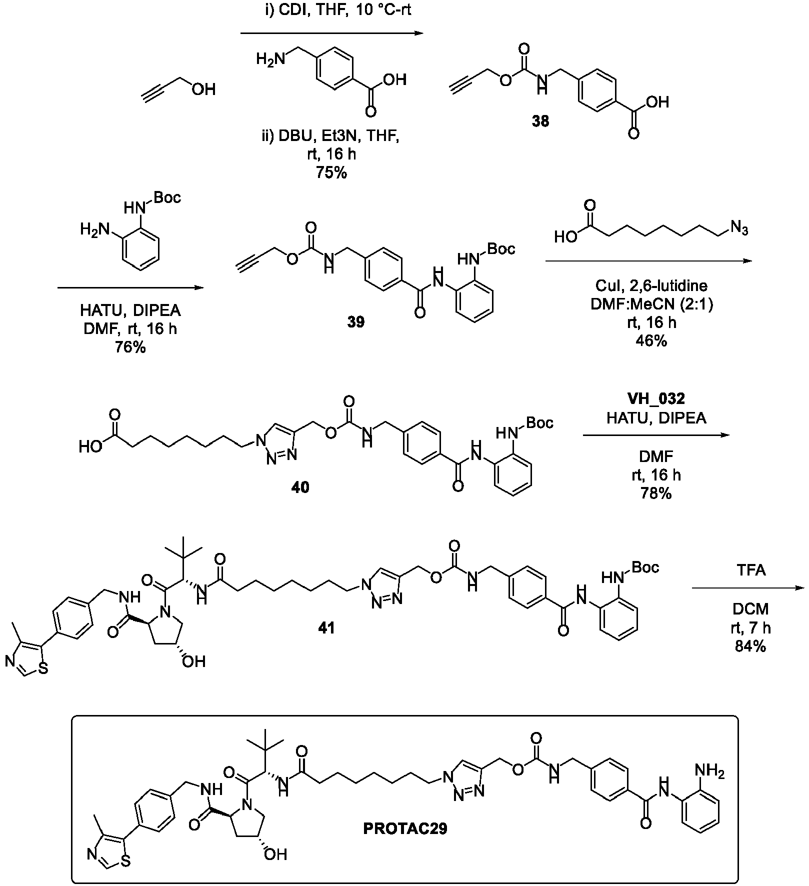

**4-({[(Prop-2-yn-1-yloxy)carbonyl]amino}methyl)benzoic acid (38):** Propargyl alcohol (0.12 mL, 1.98 mmol) and carbonyldiimidazole (0.32 g, 1.98 mmol) were dissolved in THF (5 mL) and stirred at 10 °C for 1 h. 4-(Aminomethyl)benzoic acid (0.30 g, 1.98 mmol), DBU (0.29 mL, 1.98 mmol) and triethylamine (0.27 mL, 1.98 mmol) were suspended in THF (5 mL) and combined with the CDI-intermediate solution and the reaction stirred at room temperature for 16 h. The solvent was removed under reduced pressure, suspended in H₂O (10 mL) and the solution was acidified with 1M HCl solution to pH 5. The resulting precipitate was collected by gravity filtration and air dried to provide **38** (0.35 g, 75% yield) as a white powder. HRMS (ESI) m/z: [M+H]⁺ calculated for C₁₂H₁₂NO₄: 234.0766, found 234.0764.

**Tert-butyl *N*-{2-[4-({[(prop-2-yn-1-yloxy)carbonyl]amino}methyl)benzamid o]phenyl} carbamate (39): 38** and HATU (0.49 g, 1.28 mmol) were dissolved in anhydrous DMF (5 mL) and stirred at room temperature for 1 h. Tert-butyl N-(2-aminophenyl)carbamate (0.18 g, 0.85 mmol) and diisopropylethylamine (0.30 mL, 1.71 mmol) were added and the reaction stirred at room temperature for 16 h. The solvent was removed under reduced pressure and suspended in EtOAc (20 mL). The organic solution was washed with sat. NaHCO₃ (2 × 20 mL), brine (20 mL), the organic layers combined, dried over MgSO₄, filtered and the solvent removed under reduced pressure. The crude oil was purified by flash chromatography on silica (Hexane:EtOAc 30:70-50:50 gradient) to provide **39** (0.28 g, 76% yield) as a pale pink oil. HRMS (ESI) m/z: [M+H]⁺ calculated for C₂₃H₂₆N₃O₅: 424.1872, found 424.1871.

**8-(4-((((4-((2-((tert-butoxycarbonyl)amino)phenyl)carbamoyl)benzyl)carbamoyl)oxy)methyl)-1H-1,2,3-triazol-1-yl)octanoic acid (40): 39** (0.13 g, 0.29 mmol), 8-azido-octanoic acid (0.05 g, 0.29 mmol) and CuI (0.01 g, 0.10 mmol) were suspended in degassed DMF:MeCN (5 mL, 2:1) under an inert atmosphere. 2,6-Lutidine (0.06 g, 0.52 mmol) was added and the reaction stirred at room temperature for 16 h. The solvent was removed under reduced pressure and suspended in EtOAc (10 mL), washed with sat. NaHCO₃ (2 × 10 mL), brine (2 × 10 mL), the organic layers combined, dried over MgSO₄, filtered and the solvent removed under reduced pressure. The crude oil was purified by flash chromatography on silica (3-5% MeOH:DCM solvent gradient) to provide **40** (0.08 g, 46%) as an off white solid. HRMS (ESI) m/z: [M+Na]⁺ calculated for C₃₁H₄₀N₆O₇Na: 631.2856, found 631.2856.

**(1-(8-(((S)-1-((2S,4R)-4-hydroxy-2-((4-(4-methylthiazol-5-yl)benzyl)carbamoyl)pyrrolidin-1-yl)-3,3-dimethyl-1-oxobutan-2-yl)amino)-8-oxooctyl)-1H-1,2,3-triazol-4-yl)methyl(4-((2-((tert-butoxycarbonyl)amino)phenyl)carbamoyl)benzyl)carbamate (41):** Following general method G, **41** was obtained from **40** (0.075 g, 0.12 mmol) and **VH_032 amine** (0.05 g, 0.10 mmol). The crude product was purified by flash chromatography on silica (2-7% MeOH:DCM solvent gradient) to provide **41** as an off-white crystalline solid (0.08 g, 78%). HRMS (ESI) m/z: [M+Na]⁺ calculated for C₅₃H₆₈N₁₀O₉SNa: 1043.4789, found 1043.4758.

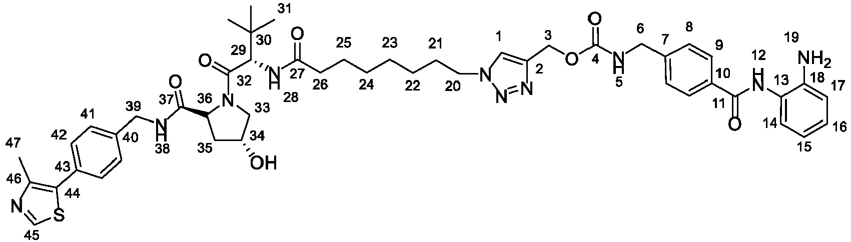

**(1-(8-(((S)-1-((2S,4R)-4-hydroxy-2-((4-(4-methylthiazol-5-yl)benzyl)carbamoyl)pyrrolidin-1-yl)-3,3-dimethyl-1-oxobutan-2-yl)amino)-8-oxooctyl)-1H-1,2,3-triazol-4-yl)methyl(4-((2-amino phenyl)carbamoyl)benzyl)carbamate (PROTAC29):** Following general method H, Boc deprotection of **41** (0.08 g, 0.08 mmol) was performed to afford **PROTAC29** (0.06 g, 84%) as an off-white solid. ¹H NMR (400 MHz, Methanol-*d₄*) δH ppm 8.75 (s, 1H, 45-CH), 7.87 (s, 1H, 1-CH), 7.82 (m, 2H, 9-CH), 7.35-7.33 (m, 2H, 41-CH), 7.31-7.27 (m, 4H, 8-CH, 42-CH), 7.08-7.06 (m, 1H, 14-CH), 6.98-6.94 (m, 1H, 16-CH), 6.80-6.77 (m, 1H, 17-CH), 6.67-6.63 (m, 1H, 15-CH), 5.05 (s, 2H, 3-CH2), 4.51 (s, 1H, 29-CH), 4.47-4.39 (m, 2H, 36-CH, 39-CH), 4.38-4.36 (m, 1H, 34-CH), 4.28-4.19 (m, 3H, 20-CH₂, 39-CH), 4.25 (s, 2H, 6-CH₂), 3.79-3.76 (m, 1H, 33-CH), 3.69-3.65 (m, 1H, 33-CH), 2.35 (s, 3H, 47-CH₃), 2.17-2.06 (m, 3H, 26-CH₂, 35-CH), 1.99-1.92 (m, 1H, 35-CH), 1.80-1.73 (m, 2H, 21-CH₂), 1.49-1.42 (m, 2H, 25-CH₂), 1.23-1.14 (m, 6H, 22-CH₂, 23-CH₂, 24-CH₂). 0.91 (s, 9H, 31-CH₃). HRMS (ESI) m/z: [M+Na]⁺ calculated for C₄₈H₆₀N₁₀O₇SNa: 943.4265, found 943.4246.

### Synthesis of PROTAC4 Negative Control using an Inactive Isomer of the VHL Ligand

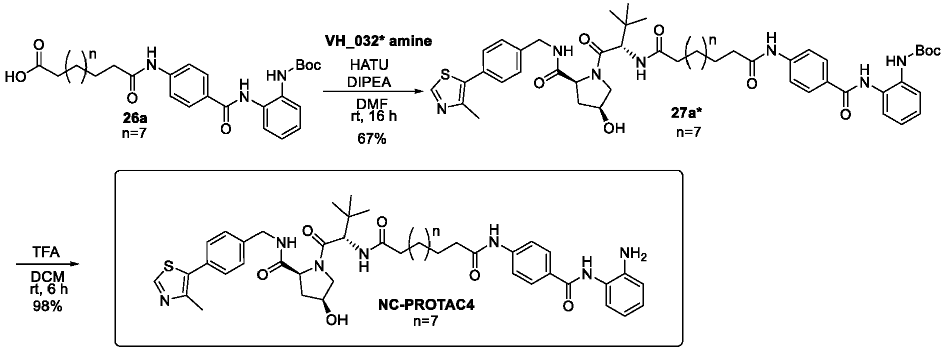

**Tert-butyl (2-(4-(12-(((S)-1-((2S,4S)-4-hydroxy-2-((4-(4-methylthiazol-5-yl)benzyl)carbamoyl)pyrroli din-1-yl)-3,3-dimethyl-1-oxobutan-2-yl)amino)-12-oxododecanamido)benzamido)phenyl)carbamate (27a*):** To a solution of **26a** (51.4 mg, 0.095 mmol) in dry DMF (1 mL) at 0 °C, DIPEA (0.04 mL, 0.238 mmol) and HATU (39.3 mg, 0.103 mmol) were added. The reaction mixture was stirred for 15 minutes, after which a solution of (2S,4S)-1-[(2S)-2-Amino-3,3-dimethyl-butanoyl]-4-hydroxy-N-[[4-(4-methylthiazol-5-yl)phenyl]methyl]pyrrolidine-2-carboxamide dihydrochloride **(VH_032* amine,** 40.0 mg, 0.079 mmol) in DMF (1 mL) was added slowly and the resultant solution stirred at room temperature for 16 hours. The reaction mixture was diluted in EtOAc (10 mL), then washed with sat. NaHCO₃ (2×5 mL) and sat. NaCl (2×5 mL). The organic layer was dried over MgSO₄, filtered and concentrated in *vacuo* to afford a dark yellow tar (104 mg). The crude product was purified by column chromatography (0-5% MeOH in DCM) to afford **27a*** (50.8 mg, 0.053 mmol, 67% yield) as a white solid. HRMS (ESI) m/z: [M+H]⁺ calculated for C₅₂H₇₀N₇O₈S: 952.5007, found 952.4999.

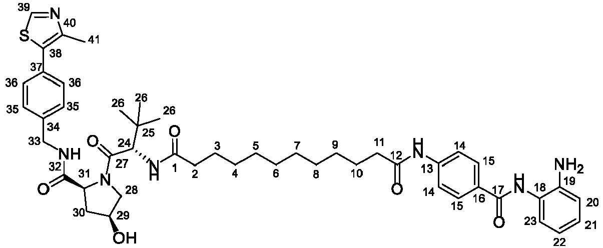

**N1-(4-((2-aminophenyl)carbamoyl)phenyl)-N12-((S)-1-((2S,4S)-4-hydroxy-2-((4-(4-methylthiazol-5-yl)benzyl)carbamoyl)pyrrolidin-1-yl)-3,3-dimethyl-1-oxobutan-2-yl)dodecanediamide (NC-PROTAC4):** Following general method H, Boc deprotection of **27a*** (29.0 mg, 0.030 mmol) was performed to afford **NC-PROTAC4** (25.6 mg, 0.029 mmol, 98% yield) as a pale yellow solid. Prior to biological evaluation the product was further purified by semi-preparative HPLC (5-95% MeCN in H₂O, 260 nm, 45 min gradient). ). ¹H NMR (400 MHz, Methanol-*d₄*) δ_{H} ppm 8.86 (s, 1 H, 39-CH) 7.95 (d, *J*=8.7 Hz, 2 H, 15-CH) 7.72 (d, *J*=8.7 Hz, 2 H, 14-CH) 7.44 (d, *J*=8.5 Hz, 2 H, 36-CH) 7.40 (d, *J*=8.5 Hz, 2 H, 35-CH) 7.18 (dd, *J*=7.8, 1.3 Hz, 1 H, 23-CH) 7.07 (app. td, *J*=7.8, 1.3 Hz, 1 H, 21-CH) 6.90 (dd, *J*=7.8, 1.3 Hz, 1 H, 20-CH) 6.76 (app. td, *J*=7.8, 1.3 Hz, 1 H, 22-CH), 4.47 - 4.58 (m, 3 H, 24-CH,31-CH,33-CH) 4.32 - 4.40 (m, 2 H, 29-CH,33-CH) 4.03 (dd, *J*=10.5, 5.1 Hz, 1 H, 28-CH) 3.69 (dd, *J*=10.5, 3.5 Hz, 1 H, 28-CH) 2.47 (s, 3 H, 41-CH₃) 2.42 - 2.45 (m, 1 H, 30-CH) 2.40 (t, *J*=7.4 Hz, 2 H, 11-CH₂) 2.18 - 2.32 (m, 2 H, 2-CH₂) 1.97 (dt, *J*=13.3, 4.3 Hz, 1 H, 30-CH) 1.70 (quin, *J*=7.4 Hz, 2 H, 10-CH₂) 1.53 - 1.63 (m, 2 H, 3-CH₂) 1.33 - 1.39 (m, 4 H, 4-CH₂,9-CH₂) 1.29 - 1.33 (m, 8 H, (5-8)-CH₂) 1.03 (s, 9 H, 26-CH₃). HRMS (ESI) m/z: [M+H]⁺ calculated for C₄₇H₆₂N₇O₆S: 852.4482, found 852.4483.

### In vitro HDAC Assay with CoREST Complex

Inhibition tests against LSD1-HDAC-C0REST complex were performed using a two-enzyme fluorescence-based HDAC assay. The inhibitor/PROTACs were dissolved at 50 mM in DMSO, then 1:2 serial dilutions performed using HDAC assay buffer (50 mM Tris pH 7.5, 150 mM NaCl) to afford range of concentrations. 10 uL of these solutions were added to individual wells, followed by 20 µL of HDAC complex dissolved in HDAC assay buffer (18 nM) and 20 µL of the substrate Boc-(Ac)Lys-AMC dissolved in HDAC assay buffer.

The assays were performed in black 96-well plates with a reaction volume of 50 µL per well. All determinations were performed in triplicate. Control wells containing no inhibitor were used to determine when inhibition had ceased. After an incubation of 20 minutes at 37 ° C and 150 rpm, deacetylation was stopped by the addition of 50 µL of a developing solution containing trypsin (50 mM Tris pH 7.5, 100 mM NaCl, 10 mg/mL trypsin). Fluorescence intensity was measured with a plate reader (PerkinElmer, 2030 multilabel reader, VICTOR X₅, λₑₓ 335 nm, λₑₘ 460 nm). HDAC Activity was calculated by subtracting the average blank fluorescence from the well fluorescence. Graphpad Prism software was utilised to determine IC₅₀ values.

### Cell Lines and Materials

E14 wild type (WT) mouse embryonic stem (mES) cells were maintained on gelatinised plates in standard mES media consisting of Knockout Dulbecco's Modified Eagle Medium (KO DMEM) (GIBCO, 10829-018) supplemented with 15% Fetal Bovine Serum (FBS) (Sigma, F9665), 1X glutamine/penicillin/streptomycin (GIBCO, 10378-016), 100 µM β-mercaptoethanol (Sigma) and Leukaemia Inhibitory Factor (synthesised in house). HCT116 human colon carcinoma cells were grown in Dulbecco's Modified Eagle Medium (DMEM) (GIBCO, 41965-039) supplemented with 10% Fetal Bovine Serum (FBS) (Sigma) and 1X glutamine/ penicillin/streptomycin (GIBCO, 10378-016). Both cell lines were incubated at 37 °C with 5% CO₂. Cells were treated with PROTACs (0.01-40 µM) alongside HDAC inhibitors CI-994 (10/40 µM) and Panobinostat (30 nM) as controls.

### Western Blotting

HCT116 or mES cells were treated 24 hours after seeding. 24 hours post treatment, cells were harvested, lysed in lysis buffer (50mM Tris-HCl, 150 mM NaCl, 0.5% NP-40, 0.5% Triton X-100) with protease inhibitor (Sigma, P8340), then incubated on ice for 30 minutes, before being centrifuged (18,000 rcf, 15 minutes, 4 ° C). The supernatant was collected, and protein concentrations quantified via Bradford Assay using Protein Assay Dye Reagent Concentrate (BIO-RAD). For histone extraction, an equal volume of 0.4 N H₂SO₄ was added to the pellets and the extracts placed at 4 °C overnight. Following overnight incubation, the tubes were centrifuged (18,000 ref, 15 minutes, 4 ° C) and then the supernatant (histone extract) collected.

Western blots were run on NuPAGE^{™} 4-12% Bis-Tris gels with 20-30 µg of protein or 10 µL of acid-extracted histone loaded per lane, using NuPAGE^{™} LDS Sample Buffer (4X). PageRuler^{™} Plus Prestained Ladder was used for size standards. After gel electrophoresis at 140V for 75-90 minutes the separated proteins were transferrred onto nitrocellulose membrane at 30V for 60 minutes. The membranes were probed with primary antibodies (listed below) for 60-90 minutes. Blots were developed with complimentary IRDye conjugated secondary antibodies and the bands visualised using the Odyssey Infrared Imaging System. Image processing and band intensity quantification was performed using Image Studio Lite.

### Antibody Information

Primary Antibodies;
α-tubulin - Sigma, t5168 (1:10,000 dilution)
HDAC1 - Abcam, 109411 (1:2,000 dilution)
HDAC2 - Merck Millipore, 05-814 (1:2,000 dilution)
HDAC3 - Abcam, 32369 (1:2,000 dilution)
H3 - Merck Millipore, 05-499 (1:1,000 dilution)
H3K9Ac - Upstate, 06-942 (1:1,000 dilution)
H3K27Ac - Merck Millipore, 07-360 (1:1,000 dilution)
H3K56Ac - Active Motif, 39281 (1:1,000 dilution)

### Secondary Antibodies;

IRDye^{®} 680LT - LI-COR Biosciences, 926-68023 (1:10,000 dilution)
IRDye^{®} 800CW - LI-COR Biosciences, 926-32210 (1:10,000 dilution)

### Cell Viability Assay

To analyse cell death, cells were treated with DMSO, CI-994 (40µM), or PROTAC 4 (1-40µM) 24 hours after seeding. 24 hours post treatment, cells were harvested and fixed with 70% (vol/vol) ethanol at -20°C overnight. Cells were washed in PBS prior to incubation with 50 µg of propidium iodide and RNase A (10 µg/mL) for 30 min at room temperature in the dark. Samples were analysed using the BD FACSCanto II flow cytometer (BD Biosciences) in the PE_A channel with BD FACSDiva software.

## Claims

1. A compound of formula (I): 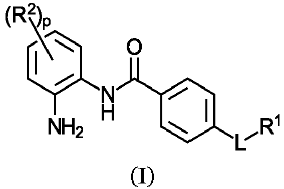 wherein L is a linker with a backbone comprising at least one group, the or each group being independently selected from the list consisting of an optionally substituted C₁-C₃₀ alkylene, an optionally substituted C₂-C₃₀ alkenylene, an optionally substituted C₂-C₃₀ alkynylene, NRs, O, S, SO, SO₂, an optionally substituted C₆-C₁₂ arylene and an optionally substituted 5 to 10 membered heteroarylene, wherein the backbone of the linker is between 7 and 50 atoms in length;
R¹ is an E3 ligand for an E3 ligase, which is: 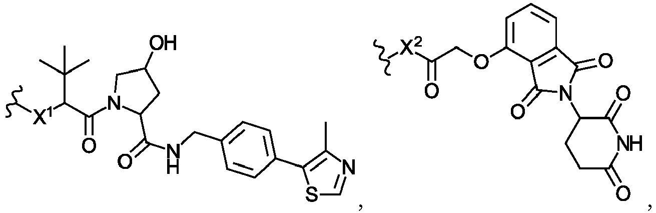 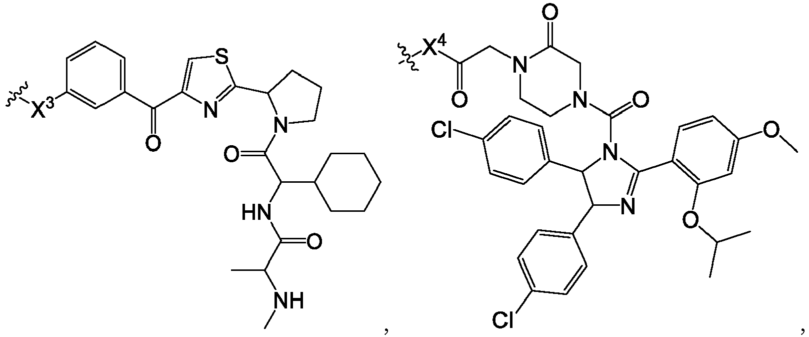 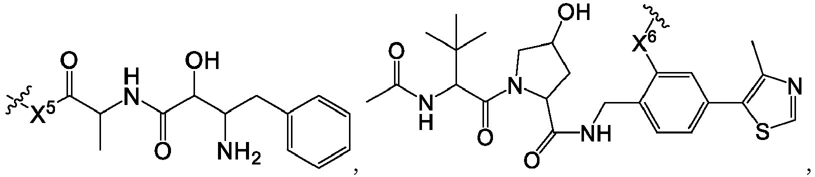 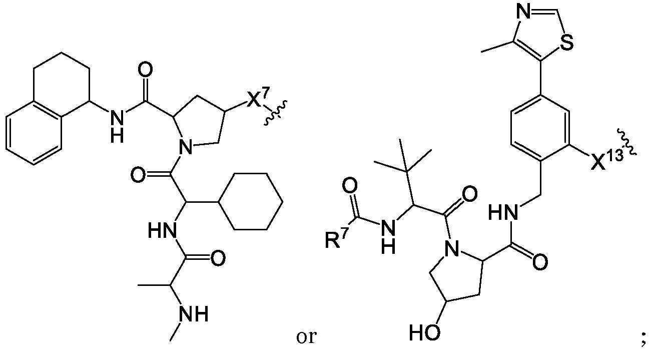
each R² is independently a halogen, OR3, NR³R⁴, C₁-C₆ alkyl, C₂-C₆ alkenyl, C₂-C₆ alkynyl, a C₆-C₁₂ aryl group or a 5 to 10 membered heteroaryl group;
R3 and R4 are independently H, C₁-C₆ alkyl, C₂-C₆ alkenyl or C₂-C₆ alkynyl;
p is 0 or an integer between 1 and 4;
X¹ to X⁷ and X¹³ are each NH or O and R⁷ is H, an optionally substituted C₁-C₆ alkyl, an optionally substituted C₂-C₆ alkenyl, an optionally substituted C₂-C₆ alkynyl, an optionally substituted C₃-C₆ cycloalkyl, an optionally substituted 3 to 6 membered heterocycle, an optionally substituted phenyl or an optionally substituted 5 or 6 membered heteroaryl;
wherein the or each optionally substituted alkylene, optionally substituted alkenylene, optionally substituted alkynylene, optionally substituted heteroaryl and optionally substituted heteroarylene may be unsubstituted or substituted with one or more of halogen, oxo, OH, NH₂, C₁-C₆ alkyl, C₂-C₆ alkenyl, C₂-C₆ alkynyl, a C₃-C₆ cycloalkyl, a 3 to 10 membered heterocycle, a C₆-C₁₂ aryl and/or a 5 to 10 membered heterocycle; and
the or each optionally substituted arylene may be unsubstituted or substituted with one or more of halogen, OH, NH₂, C₁-C₆ alkyl, C₂-C₆ alkenyl, C₂-C₆ alkynyl, a C₃-C₆ cycloalkyl, a 3 to 10 membered heterocycle, a C₆-C₁₂ aryl and/or a 5 to 10 membered heterocycle;
or a pharmaceutically acceptable salt, solvate or tautomeric form thereof.

2. The compound according to claim 1, wherein p is 0 or p is 1 and the compound of formula (I) is a compound of formula (Iaiii) or (Iaiv):

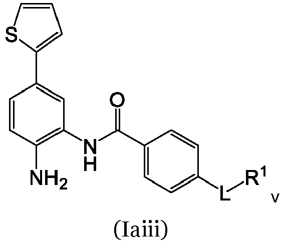

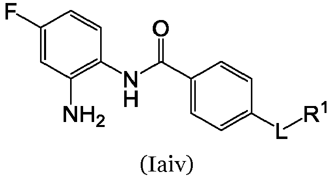

3. The compound according to claim 1 or claim 2, wherein R¹ is 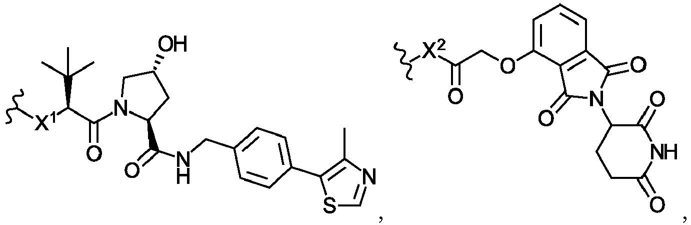 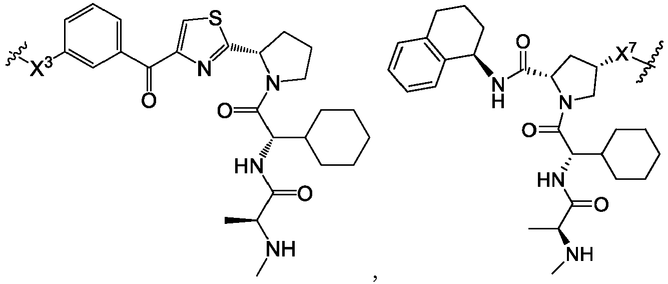 or 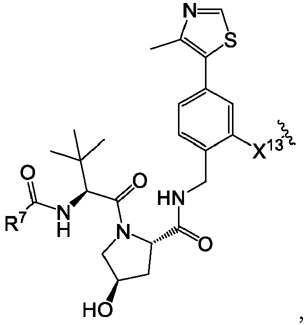 optionally wherein X¹, X² and X⁷ are NH and X3 and X¹³ are O and R⁷ is methyl or

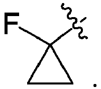

4. The compound according to any preceding claim, wherein the backbone of the linker is between 7 and 40, between 8 and 30, between 9 and 25, between 10 and 20 or between 11 and 15 atoms in length.

5. The compound according to any preceding claim, wherein L is -L¹-L²-, wherein:
L¹ is absent or is -L³-L⁴-L⁵-L⁶- and L² is -L⁷-L⁸-L⁹-L¹⁰-, wherein:
L³ is an optionally substituted C₁-C₆ alkylene, an optionally substituted C₂-C₆ alkenylene or an optionally substituted C₂-C₆ alkynylene;
L⁴ is NR⁵, O, S, 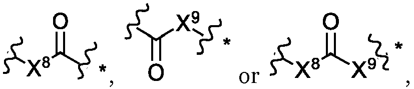 where an asterisk indicates a point of bonding to L5 or, if L5 is absent, L⁶;
L⁵ is absent, an optionally substituted C₁-C₆ alkylene, an optionally substituted C₂-C₆ alkenylene or an optionally substituted C₂-C₆ alkynylene, an optionally substituted C₆-C₁₂ arylene or an optionally substituted 5 to 10 membered heteroarylene;
L⁶ is an optionally substituted C₆-C₁₂ arylene or an optionally substituted 5 to 10 membered heteroarylene;
L⁷ is absent, an optionally substituted C₁-C₆ alkylene, an optionally substituted C₂-C₆ alkenylene or an optionally substituted C₂-C₆ alkynylene;
L⁸ is absent, 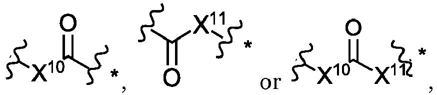 where an asterisk indicates a point of bonding to L⁹;
L⁹ is an optionally substituted C₁-C₂₀ alkylene, an optionally substituted C₂-C₂₀ alkenylene or an optionally substituted C₂-C₂₀ alkynylene, wherein the backbone of the alkylene, alkenylene or alkynylene group is optionally interrupted by one or more heteroatoms selected from O or NR⁶, or L⁹ is 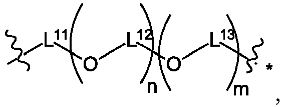 where an asterisk indicates a point of bonding to L¹⁰ or, if L¹⁰ is absent, R¹;
L¹⁰ is absent, C(O) or 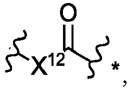 where an asterisk indicates a point of bonding to R¹;
L¹¹ is independently absent, an optionally substituted C₁-C₅ alkylene, an optionally substituted C₂-C₅ alkenylene or an optionally substituted C₂-C₅ alkynylene;
L¹² and L¹³ are independently an optionally substituted C₁-C₅ alkylene, an optionally substituted C₂-C₅ alkenylene or an optionally substituted C₂-C₅ alkynylene;
X⁸ to X¹² are independently O or NR⁶;
R5 and R⁶ are independently H, a C₁-C₆ alkyl, a C₂-C₆ alkenyl or a C₂-C₆ alkynyl;
m is o an integer between 1 and 10; and
n is an integer between 1 and 10.

6. The compound according to claim 5, wherein L¹ is 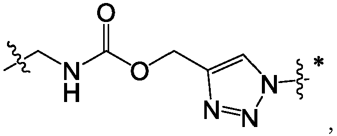 where an asterisk indicates a point of bonding to L², or L¹ is absent.

7. The compound according to claim 5 or 6, wherein L² is 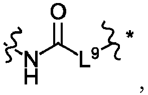 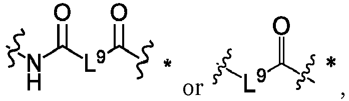 where an asterisk indicates a point of bonding to R¹, preferably wherein L² is 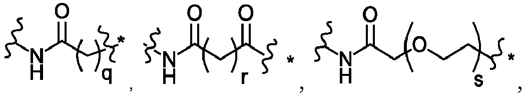 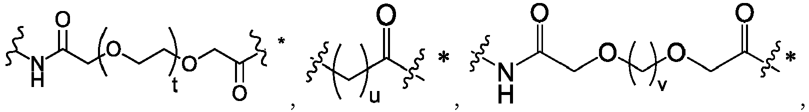 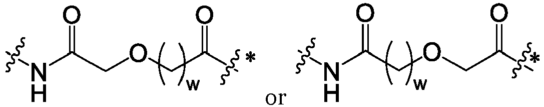 wherein:
an asterisk indicates a point of bonding to R¹;
q is an integer of at least 5;
r is an integer of at least 4;
s is an integer of at least 2;
t is an integer of at least 1;
u is an integer of at least 3;
v is an integer of at least 1; and
w is an integer of at least 2,
more preferably wherein q is an integer between 5 and 20, between 7 and 15 or between 10 and 12;
r is an integer between 4 and 20, between 7 and 15 or between 9 and 11;
s is an integer between 2 and 10 or between 2 and 5;
t is an integer between 1 and 10 or between 1 and 5;
u is an integer between 2 and 15, between 3 and 12, between 4 and 10 or between 6 and 8; and
w is an integer between 2 and 14, between 3 and 12, between 5 and 10 or between 7 and 9.

8. The compound according to any preceding claim, wherein L is 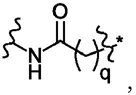 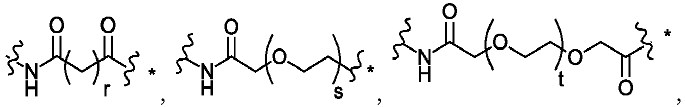 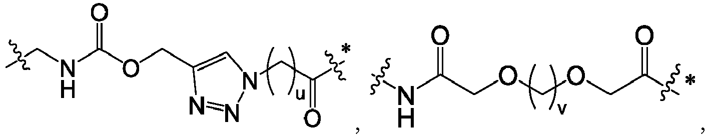 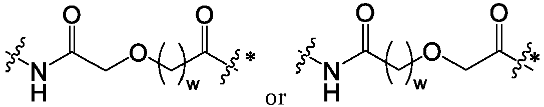 wherein:
an asterisk indicates a point of bonding to R¹;
q is an integer of at least 5;
r is an integer of at least 4;
s is an integer of at least 2;
t is an integer of at least 1;
u is an integer of at least 3;
v is an integer of at least 1; and
w is an integer of at least 2,
preferably wherein:
q is an integer between 5 and 20, between 7 and 15 or between 10 and 12;
r is an integer between 4 and 20, between 7 and 15 or between 9 and 11;
s is an integer between 2 and 10 or between 2 and 5;
t is an integer between 1 and 10 or between 1 and 5;
u is an integer between 2 and 15, between 3 and 12, between 4 and 10 or between 6 and 8; and
w is an integer between 2 and 14, between 3 and 12, between 5 and 10 or between 7 and 9.

9. The compound according to claim 1, wherein the compound of formula (I) is a compound of formula (Ic), (Id), (Ie) or (If): 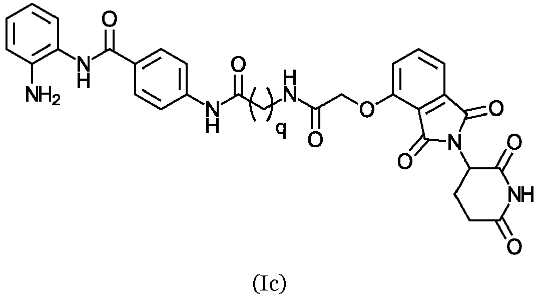 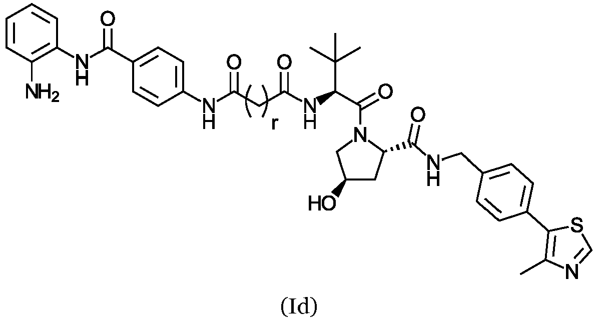 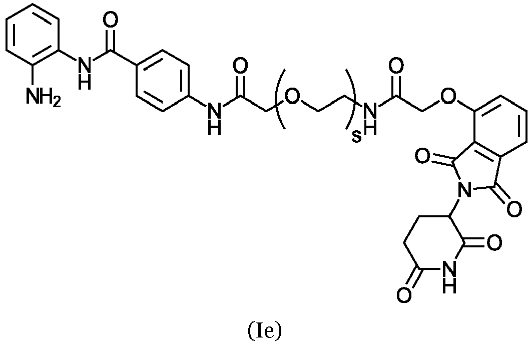 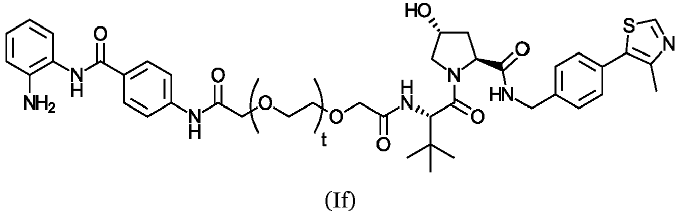 preferably wherein the compound is a compound of formula (Ic) and q is 11 or the compound is a compound of formula (Id) and r is 10.

10. The compound according to claim 1, wherein the compound is a compound of formula (101) to (116):

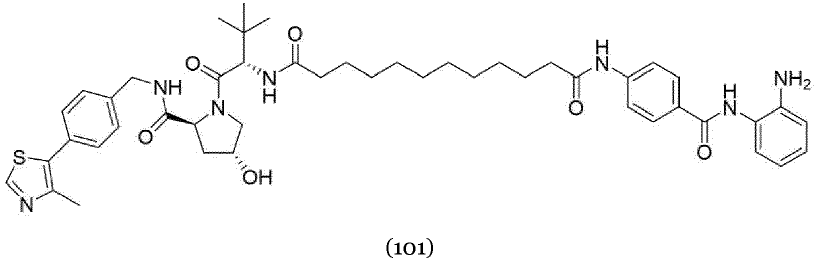

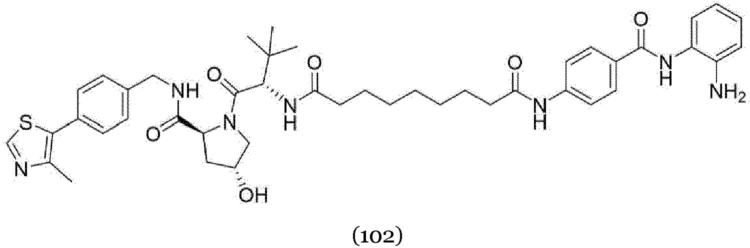

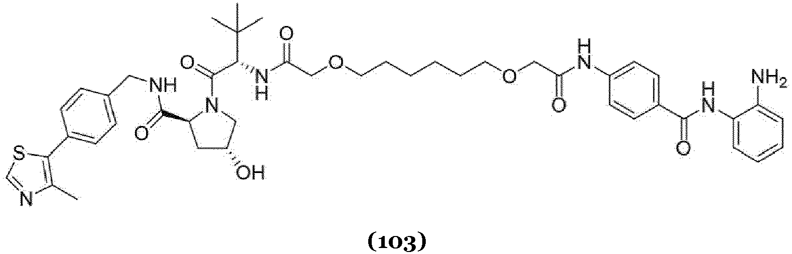

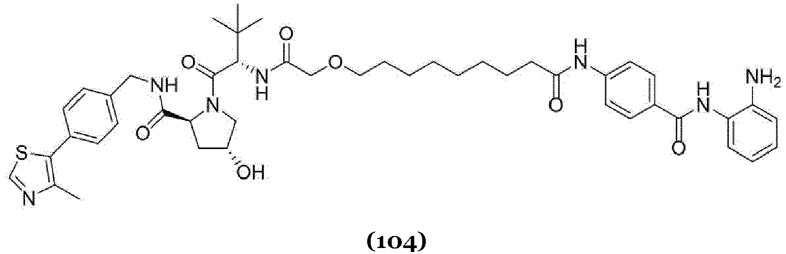

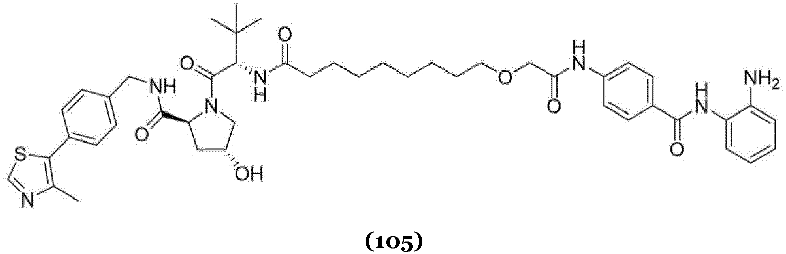

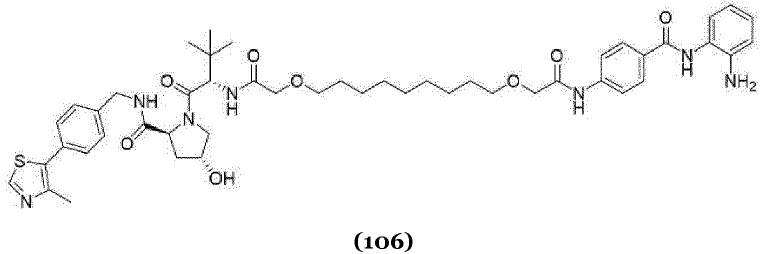

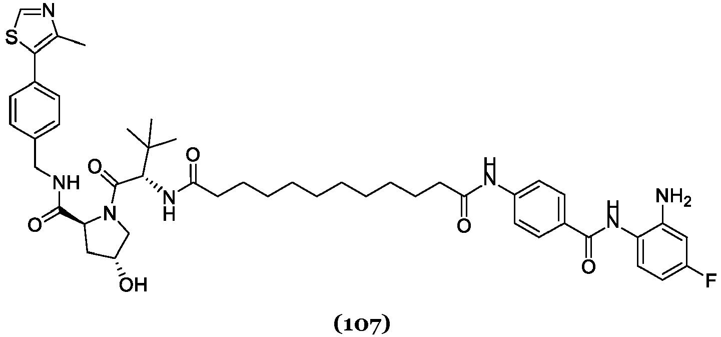

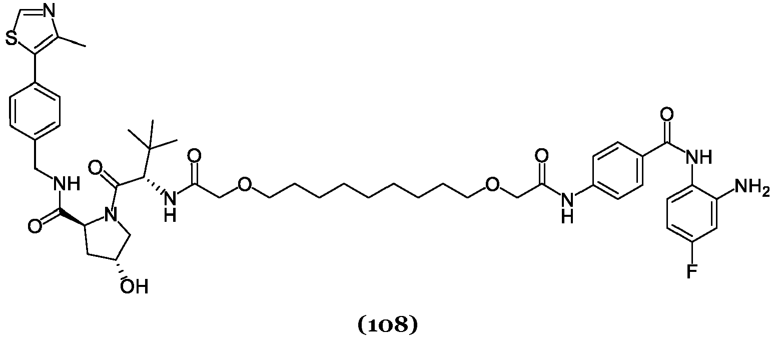

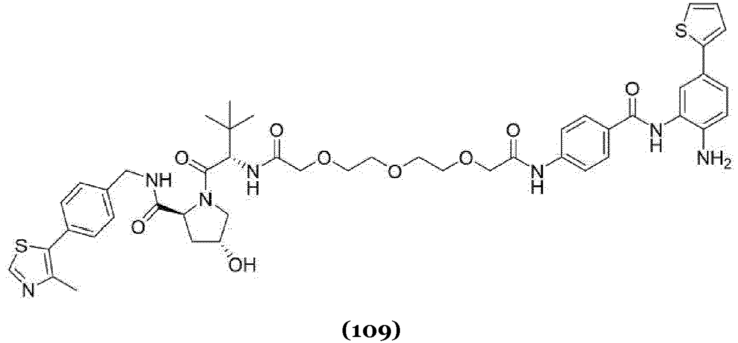

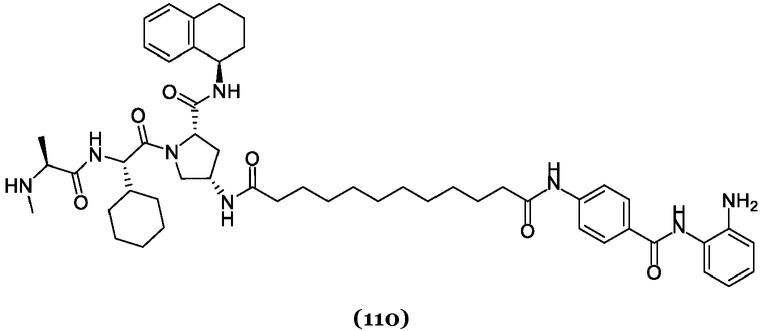

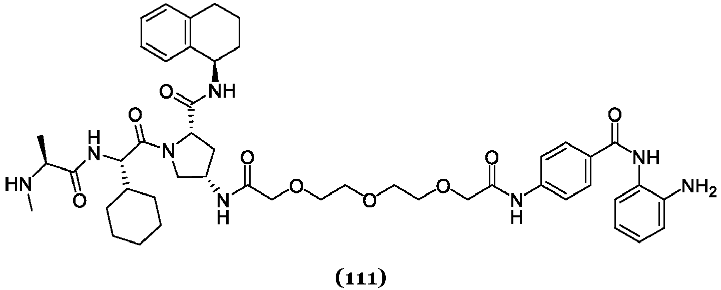

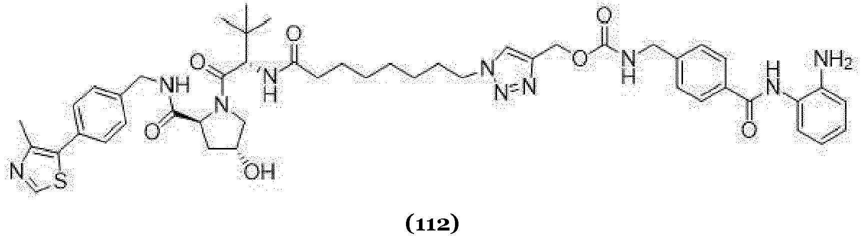

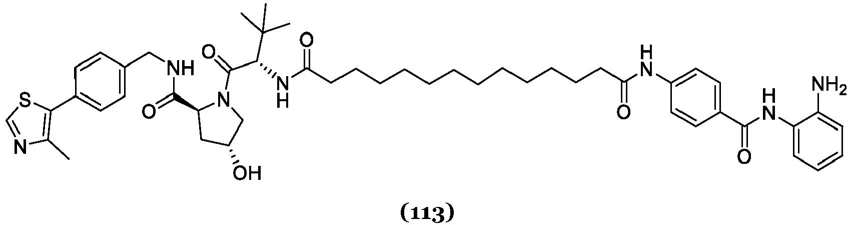

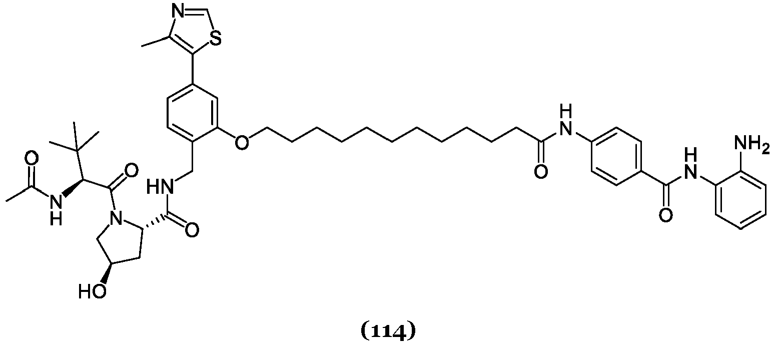

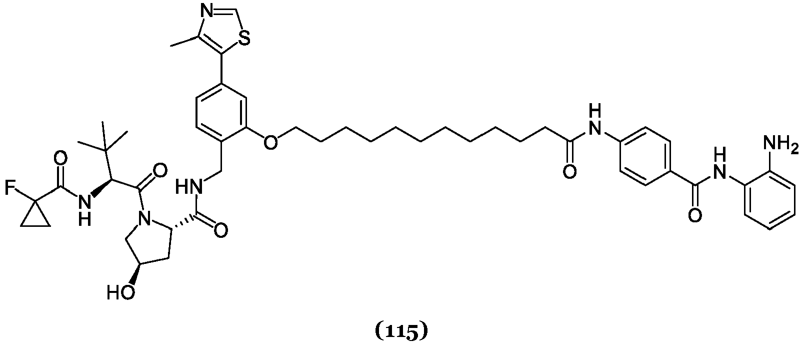

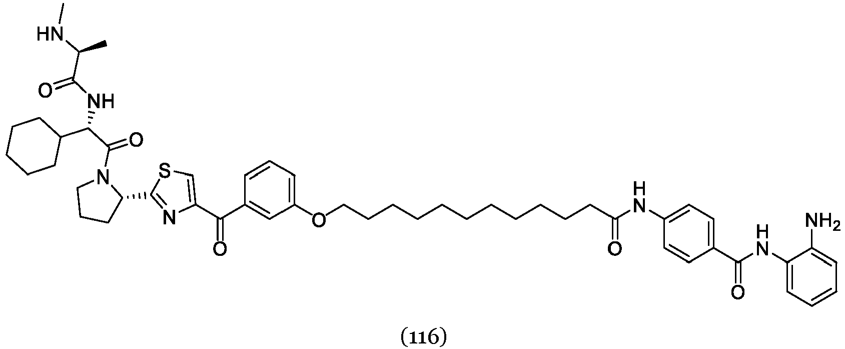

11. A pharmaceutical composition, the composition comprising a compound of formula (I), as defined in any one of claims 1 to 10, or a pharmaceutically acceptable complex, salt, solvate or tautomeric form thereof and a pharmaceutically acceptable vehicle.

12. A compound of formula (I), as defined in any one of claims 1 to 10, or a pharmaceutically acceptable complex, salt, solvate or tautomeric form thereof, or a pharmaceutical composition, as defined in claim 11, for use in therapy.

13. A compound of formula (I), as defined in any one of claims 1 to 10, or a pharmaceutically acceptable complex, salt, solvate or tautomeric form thereof, or a pharmaceutical compositions, as defined in claim 11, for use in treating cancer, preferably wherein the cancer is blood cancer, bowel cancer, brain cancer, breast cancer, cervical cancer, endometrial cancer, gastric cancer, liver cancer, lung cancer, ovarian cancer, pancreatic cancer, prostate cancer or skin cancer.

## Patentansprüche

1. Verbindung von Formel (I): 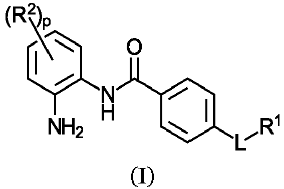 wobei L ein Linker mit einem Gerüst ist, umfassend mindestens eine Gruppe, wobei die oder jede Gruppe unabhängig ausgewählt ist aus der Liste, bestehend aus einem optional substituierten C₁-C₃₀-Alkylen, einem optional substituierten C₂-C₃₀-Alkenylen, einem optional substituierten C₂-C₃₀-Alkinylen, NR³, O, S, SO, SO₂, einem optional substituierten C₆-C₁₂-Arylen und einem optional substituierten 5- bis 10-gliedrigen Heteroarylen, wobei die Hauptkette des Linkers zwischen 7 und 50 Atome lang ist;
R¹ ein E3-Ligand für eine E3-Ligase ist, die wie folgt ist: 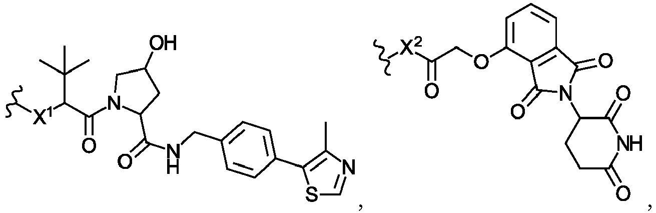 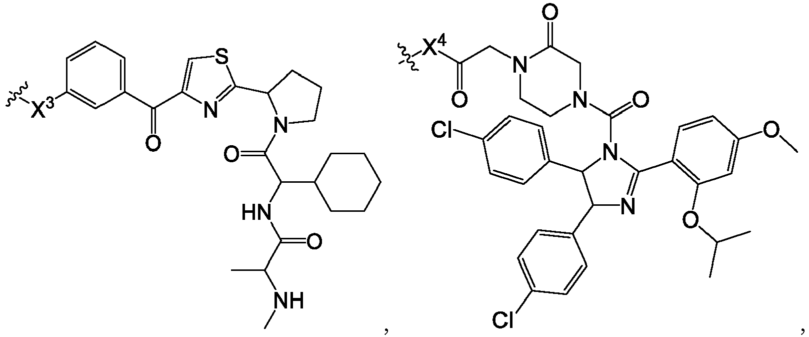 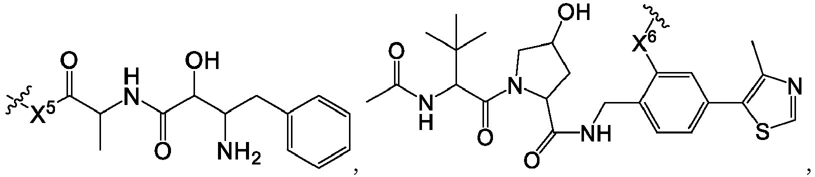 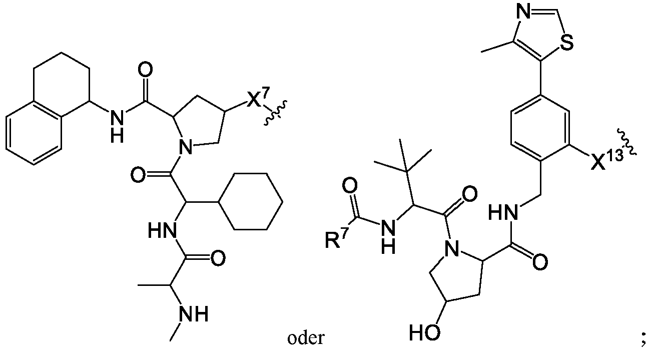
jedes R² unabhängig ein Halogen, OR³, NR³R⁴, C₁-C₆-Alkyl, C₂-C₆-Alkenyl, C₂-C₆-Alkinyl, eine C₆-C₁₂-Arylgruppe oder eine 5- bis 10-gliedrige Heteroarylgruppe ist;
R³ und R⁴ unabhängig H, C₁-C₆-Alkyl, C₂-C₆-Alkenyl oder C₂-C₆-Alkinyl sind;
p 0 oder eine ganze Zahl zwischen 1 und 4 ist;
X¹ bis X⁷ und X¹³ jeweils NH oder O sind und R⁷ H, ein optional substituiertes C₁-C₆-Alkyl, ein optional substituiertes C₂-C₆-Alkenyl, ein optional substituiertes C₂-C₆-Alkinyl, ein optional substituiertes C₃-C₆-Cycloalkyl, ein optional substituierter 3- bis 6-gliedriger Heterocyclus, ein optional substituiertes Phenyl oder ein optional substituiertes 5- oder 6-gliedriges Heteroaryl ist; wobei das oder jedes optional substituierte Alkylen, optional substituierte Alkenylen, optional substituierte Alkinylen, optional substituierte Heteroaryl und optional substituierte Heteroarylen unsubstituiert oder mit einem oder mehreren von Halogen, Oxo, OH, NH₂, C₁-C₆-Alkyl, C₂-C₆-Alkenyl, C₂-C₆-Alkinyl, einem C₃-C₆-Cycloalkyl, einem 3- bis 10-gliedrigen Heterocyclus, einem C₆-C₁₂-Aryl und/oder einem 5- bis 10-gliedrigen Heterocyclus substituiert sein kann; und
das oder jedes optional substituierte Arylen unsubstituiert oder mit einem oder mehreren von Halogen, OH, NH₂, C₁-C₆-Alkyl, C₂-C₆-Alkenyl, C₂-C₆-Alkinyl, einem C₃-C₆-Cycloalkyl, einem 3- bis 10-gliedrigen Heterocyclus, einem C₆-C₁₂-Aryl und/oder einem 5- bis 10-gliedrigen Heterocyclus substituiert sein kann;
oder ein pharmazeutisch annehmbares Salz, Solvat oder eine tautomere Form davon.

2. Verbindung nach Anspruch 1, wobei p 0 ist oder p 1 ist und die Verbindung von Formel (I) eine Verbindung von Formel (Iaiii) oder (Iaiv) ist:

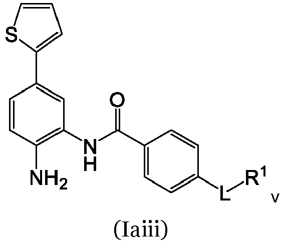

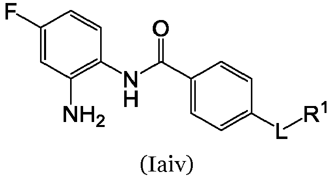

3. Verbindung nach Anspruch 1 oder Anspruch 2, wobei R¹ 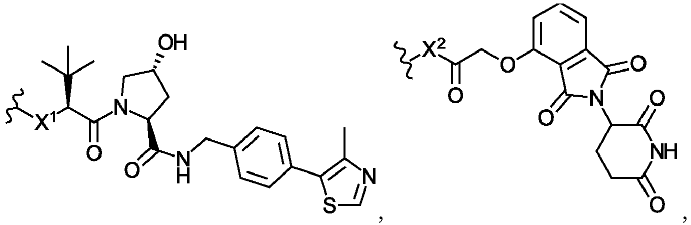 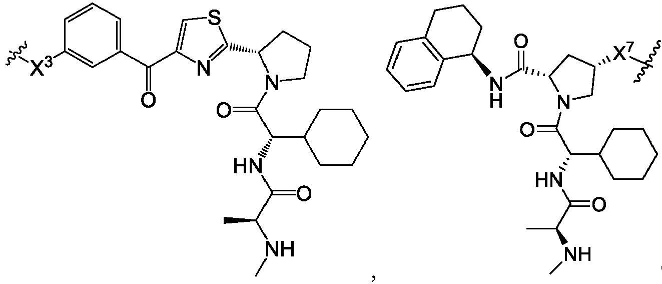 oder 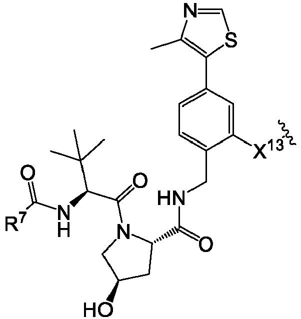 ist, optional wobei X¹, X² und X⁷ NH sind und X³ und X¹³ O sind und R⁷ Methyl oder

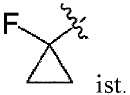

4. Verbindung nach einem vorhergehenden Anspruch, wobei das Gerüst des Linkers zwischen 7 und 40, zwischen 8 und 30, zwischen 9 und 25, zwischen 10 und 20 oder zwischen 11 und 15 Atome lang ist.

5. Verbindung nach einem vorhergehenden Anspruch, wobei L -L¹-L²- ist, wobei:
L¹ nicht vorhanden ist oder -L³-L⁴-L⁵-L⁶- ist und L² -L⁷-L⁸-L⁹-L¹⁰- ist, wobei:
L³ ein optional substituiertes C₁-C₆-Alkylen, ein optional substituiertes C₂-C₆-Alkenylen oder ein optional substituiertes C₂-C₆-Alkinylen ist;
L⁴ NR⁵, O, S, 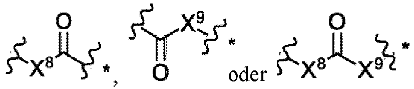 ist, wobei ein Sternchen einen Bindungspunkt an L⁵ oder, wenn L⁵ nicht vorhanden ist, an L⁶ angibt;
L⁵ nicht vorhanden, ein optional substituiertes C₁-C₆-Alkylen, ein optional substituiertes C₂-C₆-Alkenylen oder ein optional substituiertes C₂-C₆-Alkinylen, ein optional substituiertes C₆-C₁₂-Arylen oder ein optional substituiertes 5- bis 10-gliedriges Heteroarylen ist;
L⁶ ein optional substituiertes C₆-C₁₂-Arylen oder ein optional substituiertes 5- bis 10-gliedriges Heteroarylen ist;
L⁷ nicht vorhanden, ein optional substituiertes C₁-C₆-Alkylen, ein optional substituiertes C₂-C₆-Alkenylen oder ein optional substituiertes C₂-C₆-Alkinylen ist;
L⁸ nicht vorhanden, 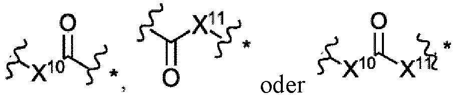 ist, wobei ein Sternchen einen Bindungspunkt an L⁹ angibt;
L⁹ ein optional substituiertes C₁-C₂₀-Alkylen, ein optional substituiertes C₂-C₂₀-Alkenylen oder ein optional substituiertes C₂-C₂₀-Alkinylen ist, wobei das Gerüst der Alkylen-, Alkenylen- oder Alkinylengruppe optional durch ein oder mehrere Heteroatome unterbrochen ist, die ausgewählt sind aus O oder NR⁶, oder L⁹ 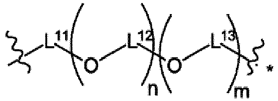 ist; wobei ein Sternchen einen Bindungspunkt an L¹⁰ oder, wenn L¹⁰ nicht vorhanden ist, an R¹ angibt;
L¹⁰ nicht vorhanden, C(O) oder 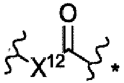 ist, wobei ein Sternchen einen Bindungspunkt an R¹ angibt;
L¹¹ unabhängig nicht vorhanden, ein optional substituiertes C₁-C₅-Alkylen, ein optional substituiertes C₂-C₅-Alkenylen oder ein optional substituiertes C₂-C₅-Alkinylen ist;
L¹² und L¹³ unabhängig ein optional substituiertes C₁-C₅-Alkylen, ein optional substituiertes C₂-Cs-Alkenylen oder ein optional substituiertes C₂-C₅-Alkinylen sind;
X⁸ bis X¹² unabhängig O oder NR⁶ sind;
R⁵ und R⁶ unabhängig H, ein C₁-C₆-Alkyl, ein C₂-C₆-Alkenyl oder ein C₂-C₆-Alkinyl sind;
m o eine ganze Zahl zwischen 1 und 10 ist; und
n eine ganze Zahl zwischen 1 und 10 ist.

6. Verbindung nach Anspruch 5, wobei L¹ 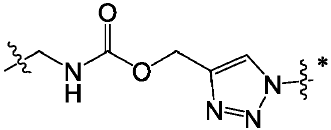 ist, wobei ein Sternchen einen Bindungspunkt an L² angibt oder L¹ nicht vorhanden ist.

7. Verbindung nach Anspruch 5 oder 6, wobei L² 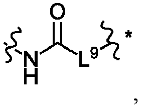 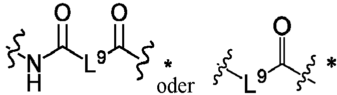 ist, wobei ein Sternchen einen Bindungspunkt an R¹ angibt, vorzugsweise wobei L² 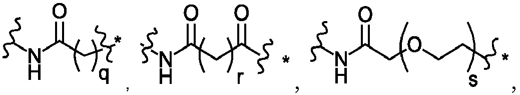 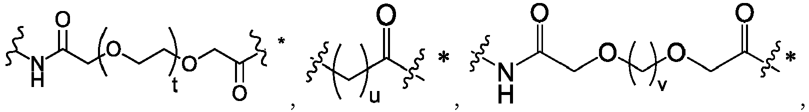 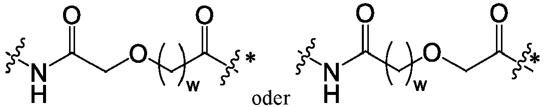 ist, wobei:
ein Sternchen einen Bindungspunkt an R¹ angibt;
q eine ganze Zahl von mindestens 5 ist;
r eine ganze Zahl von mindestens 4 ist;
s eine ganze Zahl von mindestens 2 ist;
t eine ganze Zahl von mindestens 1 ist;
u eine ganze Zahl von mindestens 3 ist;
v eine ganze Zahl von mindestens 1 ist; und
w eine ganze Zahl von mindestens 2 ist,
bevorzugter wobei q eine ganze Zahl zwischen 5 und 20, zwischen 7 und 15 oder zwischen 10 und 12 ist;
r eine ganze Zahl zwischen 4 und 20, zwischen 7 und 15 oder zwischen 9 und 11 ist;
s eine ganze Zahl zwischen 2 und 10 oder zwischen 2 und 5 ist;
t eine ganze Zahl zwischen 1 und 10 oder zwischen 1 und 5 ist;
u eine ganze Zahl zwischen 2 und 15, zwischen 3 und 12, zwischen 4 und 10 oder zwischen 6 und 8 ist; und
w eine ganze Zahl zwischen 2 und 14, zwischen 3 und 12, zwischen 5 und 10 oder zwischen 7 und 9 ist.

8. Verbindung nach einem vorhergehenden Anspruch, wobei L 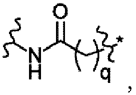 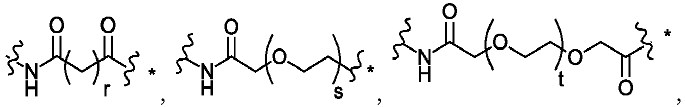 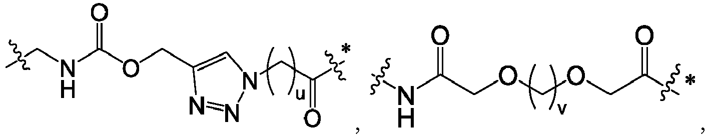 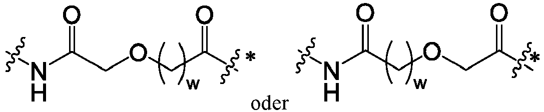 ist, wobei:
ein Sternchen einen Bindungspunkt an R¹ angibt;
q eine ganze Zahl von mindestens 5 ist;
r eine ganze Zahl von mindestens 4 ist;
s eine ganze Zahl von mindestens 2 ist;
t eine ganze Zahl von mindestens 1 ist;
u eine ganze Zahl von mindestens 3 ist;
v eine ganze Zahl von mindestens 1 ist; und
w eine ganze Zahl von mindestens 2 ist,
vorzugsweise wobei:
q eine ganze Zahl zwischen 5 und 20, zwischen 7 und 15 oder zwischen 10 und 12 ist;
r eine ganze Zahl zwischen 4 und 20, zwischen 7 und 15 oder zwischen 9 und 11 ist;
s eine ganze Zahl zwischen 2 und 10 oder zwischen 2 und 5 ist;
t eine ganze Zahl zwischen 1 und 10 oder zwischen 1 und 5 ist;
u eine ganze Zahl zwischen 2 und 15, zwischen 3 und 12, zwischen 4 und 10 oder zwischen 6 und 8 ist; und
w eine ganze Zahl zwischen 2 und 14, zwischen 3 und 12, zwischen 5 und 10 oder zwischen 7 und 9 ist.

9. Verbindung nach Anspruch 1, wobei die Verbindung von Formel (I) eine Verbindung von Formel (Ic), (Id), (Ie) oder (If) ist: 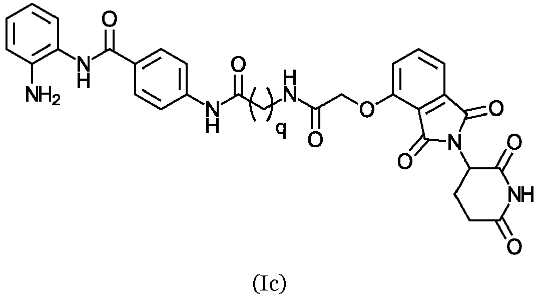 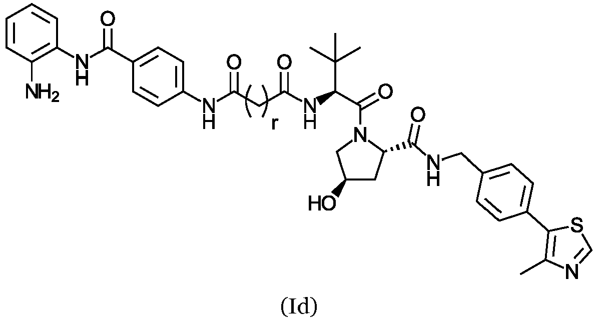 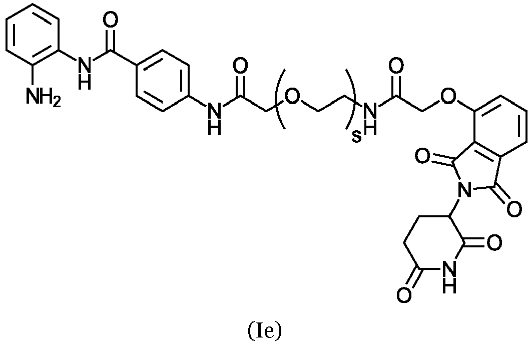 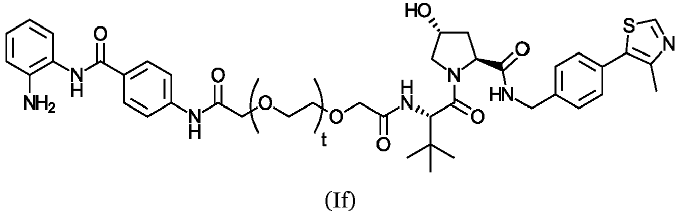 vorzugsweise wobei die Verbindung eine Verbindung von Formel (Ic) ist und q 11 ist oder die Verbindung eine Verbindung von Formel (Id) ist und r 10 ist.

10. Verbindung nach Anspruch 1, wobei die Verbindung eine Verbindung von Formel (101) bis (116) ist:

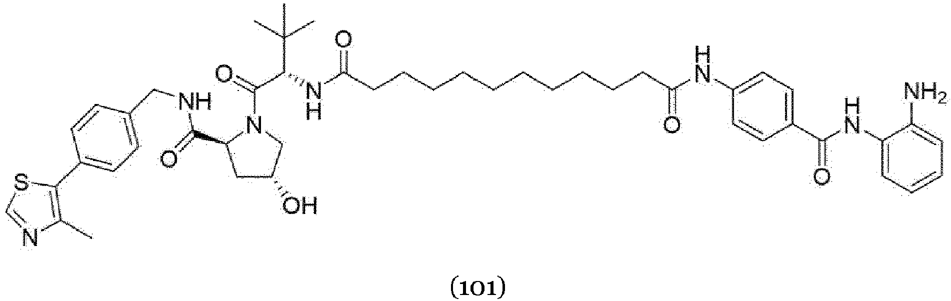

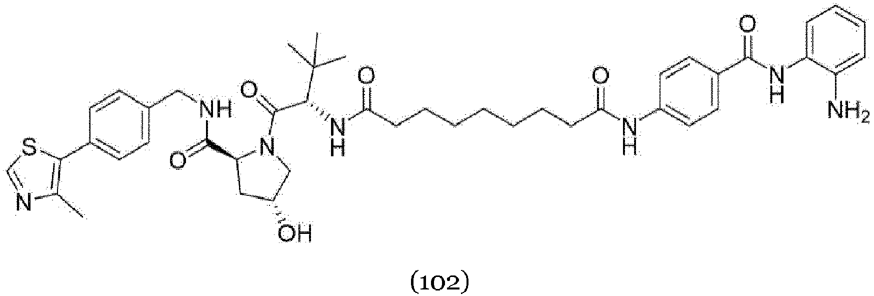

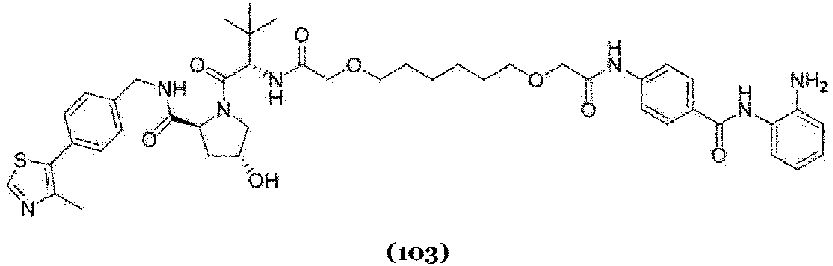

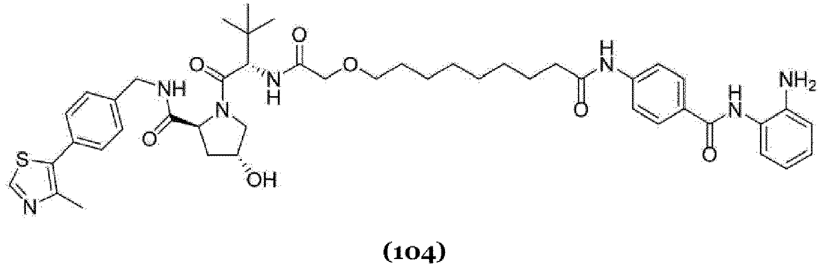

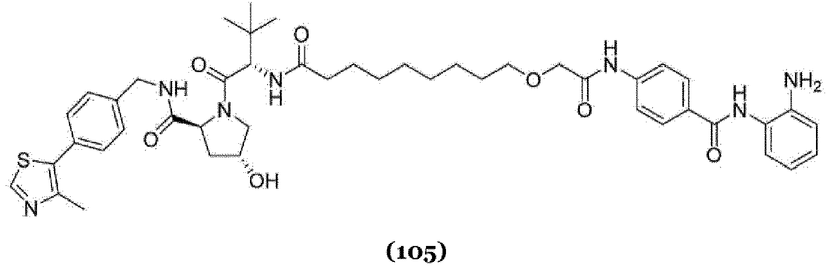

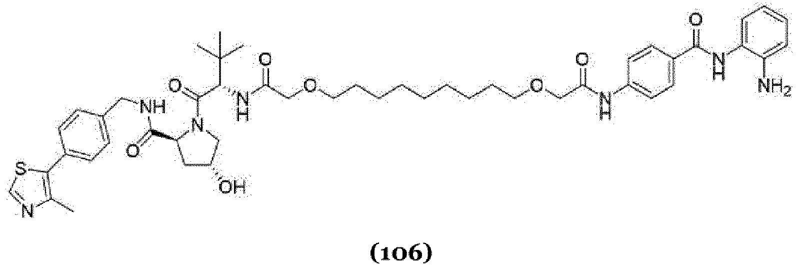

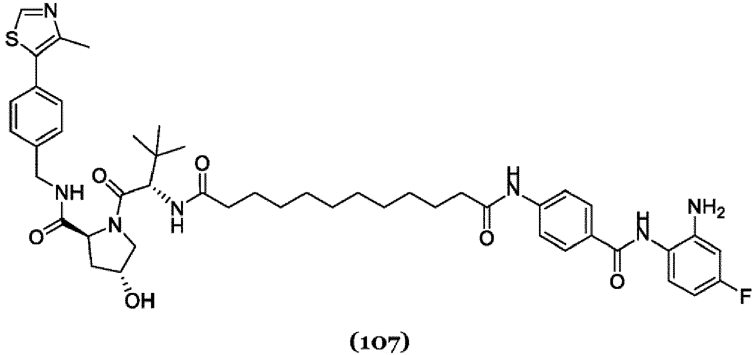

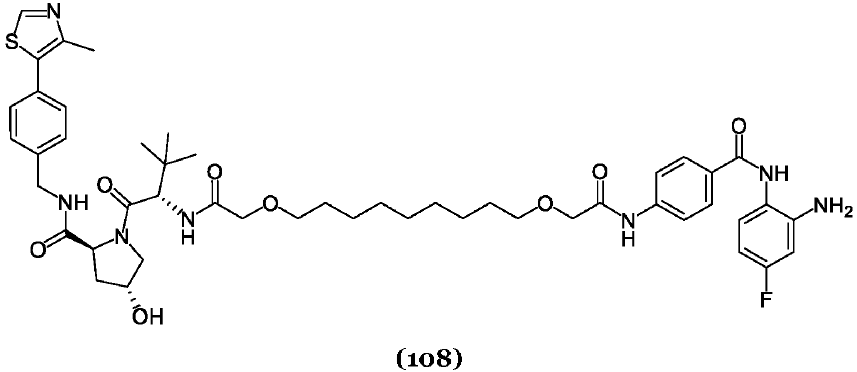

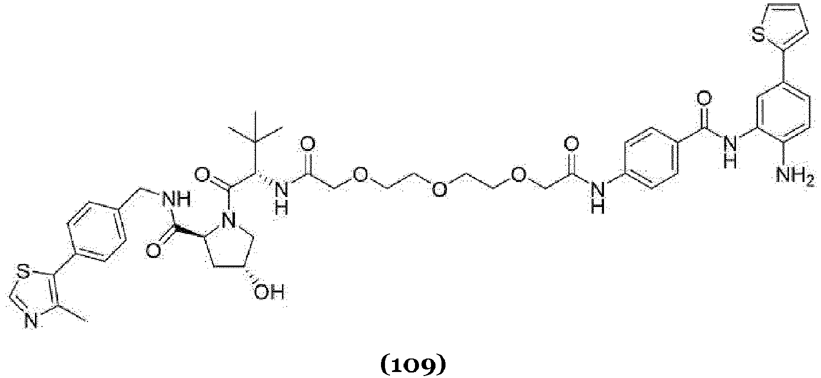

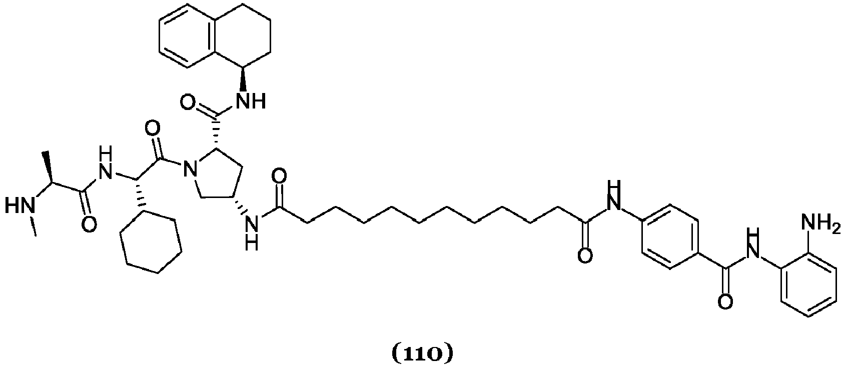

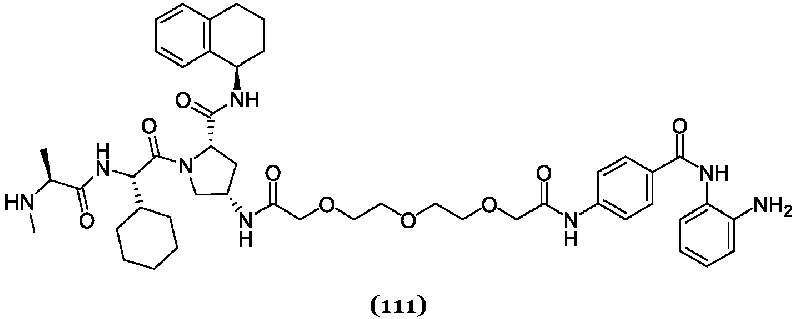

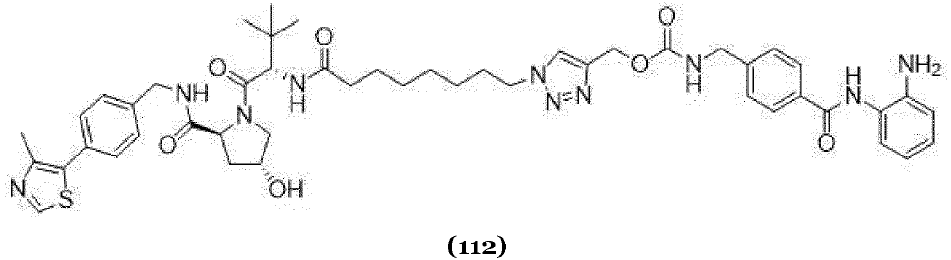

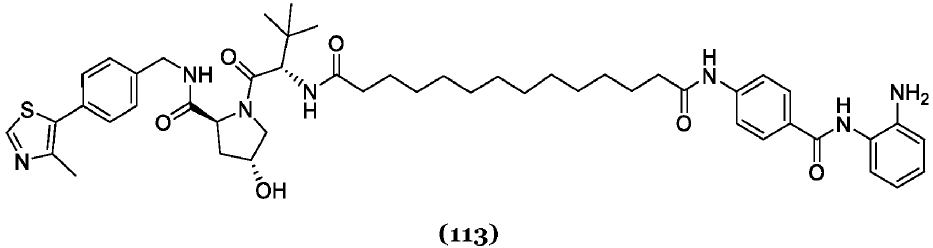

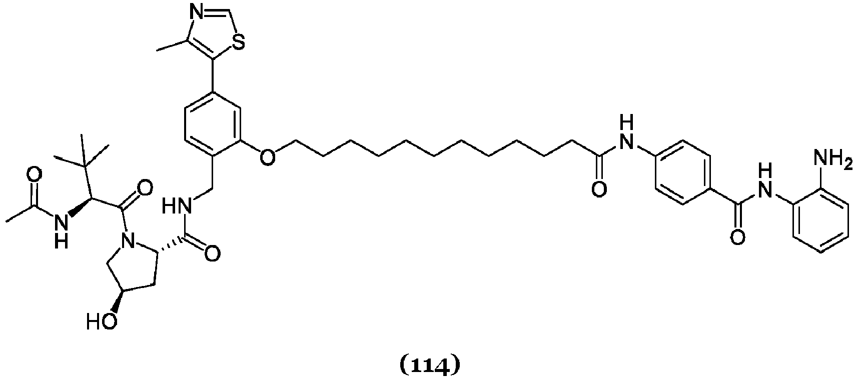

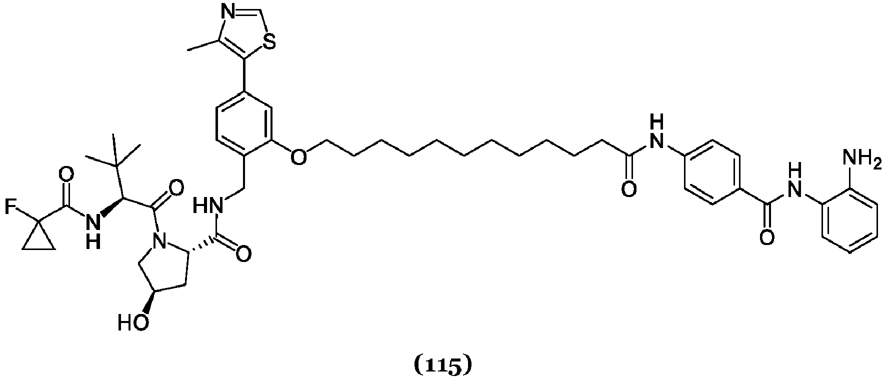

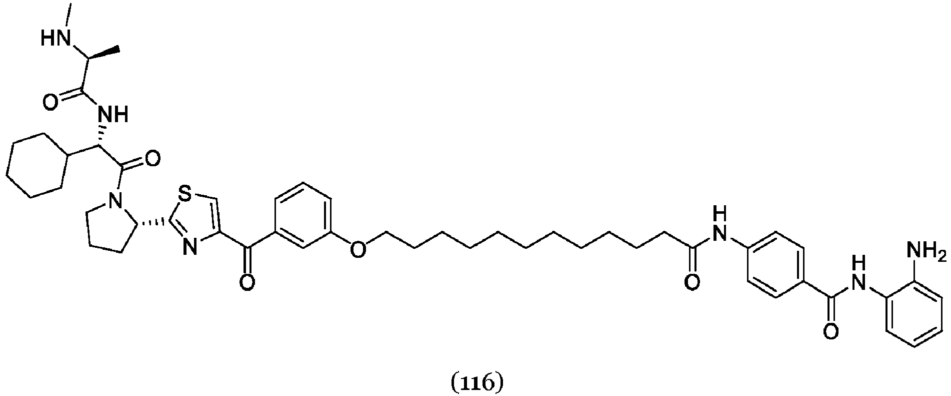

11. Pharmazeutische Zusammensetzung, die Zusammensetzung umfassend eine Verbindung von Formel (I), wie definiert in einem der Ansprüche 1 bis 10, oder einen pharmazeutisch annehmbaren Komplex, ein Salz, ein Solvat oder eine tautomere Form davon und einen pharmazeutisch annehmbaren Träger.

12. Verbindung von Formel (I), wie definiert in einem der Ansprüche 1 bis 10, oder pharmazeutisch annehmbarer Komplex, Salz, Solvat oder tautomere Form davon, oder pharmazeutische Zusammensetzung, wie definiert in Anspruch 11, zur Verwendung bei einer Therapie.

13. Verbindung von Formel (I), wie definiert in einem der Ansprüche 1 bis 10, oder pharmazeutisch annehmbarer Komplex, Salz, Solvat oder tautomere Form davon, oder pharmazeutische Zusammensetzung, wie definiert in Anspruch 11, zur Verwendung beim Behandeln von Krebs, vorzugsweise wobei der Krebs Blutkrebs, Darmkrebs, Gehirnkrebs, Brustkrebs, Gebärmutterhalskrebs, Endometriumkrebs, Magenkrebs, Leberkrebs, Lungenkrebs, Eierstockkrebs, Bauchspeicheldrüsenkrebs, Prostatakrebs oder Hautkrebs ist.

## Revendications

1. Composé de formule (I) : 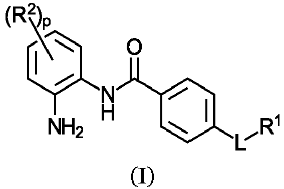 dans laquelle L représente un groupe de liaison avec une squelette comprenant au moins un groupe, le ou chaque groupe étant indépendamment choisi dans la liste constituée par un groupe C₁-C₃₀ alkylène éventuellement substitué, un groupe C₂-C₃₀ alcénylène éventuellement substitué, un groupe C₂-C₃₀ alcynylène éventuellement substitué, NR³, un atome O, S, un groupe SO, SO₂, un groupe C₆-C₁₂ arylène éventuellement substitué et un groupe hétéroarylène de 5 à 10 chaînons éventuellement substitué, ledit squelette du groupe de liaison faisant entre 7 et 50 atomes de long ;
R¹ représente un ligand E3 pour une ligase E3, qui est : 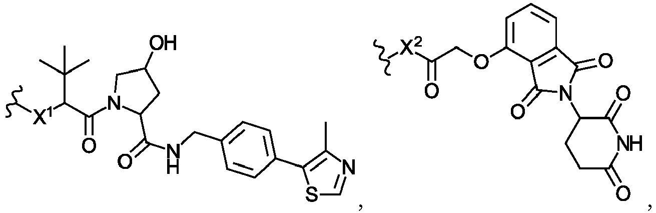 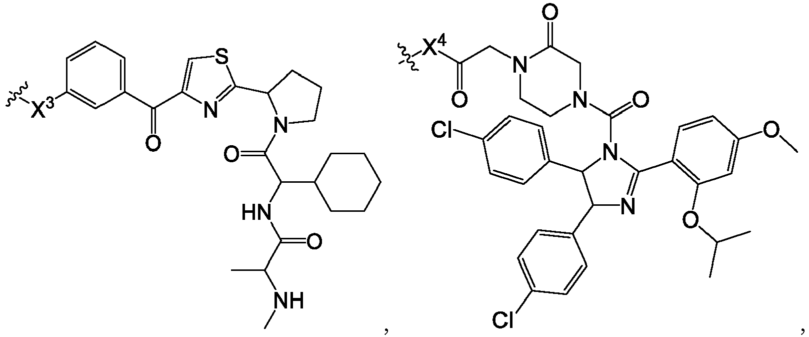 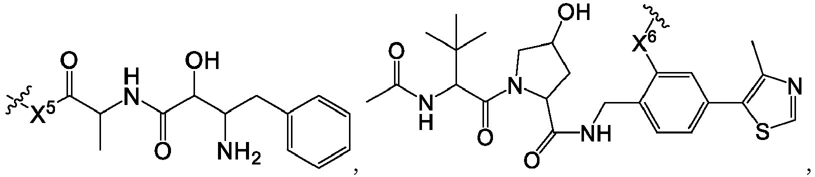 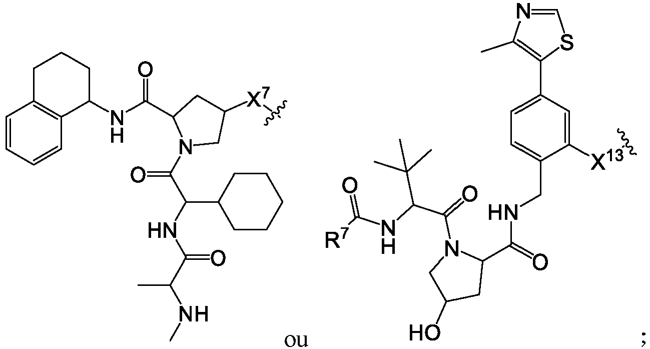
chaque R² représente indépendamment un atome d'halogène, un groupe OR³, NR³R⁴, C₁-C₆ alkyle, C₂-C₆ alcényle, C₂-C₆ alcynyle, un groupe C₆-C₁₂ aryle ou un groupe hétéroaryle de 5 à 10 chaînons ;
R³ et R⁴ représentent indépendamment un atome H, un groupe C₁-C₆ alkyle, C₂-C₆ alcényle ou C₂-C₆ alcynyle ;
p vaut 0 ou représente un entier compris entre 1 et 4 ;
X¹ à X⁷ et X¹³ représentent chacun un groupe NH ou un atome O et R⁷ représente un atome H, un groupe C₁-C₆ alkyle éventuellement substitué, un groupe C₂-C₆ alcényle éventuellement substitué, un groupe C₂-C₆ alcynyle éventuellement substitué, un groupe C₃-C₆ cycloalkyle éventuellement substitué, un groupe hétérocyclique de 3 à 6 chaînons éventuellement substitué, un groupe phényle éventuellement substitué ou un groupe hétéroaryle de 5 ou 6 chaînons éventuellement substitué ; le ou chaque groupe alkylène éventuellement substitué, alcénylène éventuellement substitué, alcynylène éventuellement substitué, hétéroaryle éventuellement substitué et hétéroarylène éventuellement substitué pouvant être non substitués ou substitués par un ou plusieurs éléments parmi un atome d'halogène, un groupe oxo, OH, NH₂, C₁-C₆ alkyle, C₂-C₆ alcényle, C₂-C₆ alcynyle, un groupe C₃-C₆ cycloalkyle, un groupe hétérocyclique de 3 à 10 chaînons, un groupe C₆-C₁₂ aryle et/ou un groupe hétérocyclique de 5 à 10 chaînons ; et
le ou chaque groupe arylène éventuellement substitué pouvant être non substitué ou substitué par un ou plusieurs éléments parmi un atome d'halogène, un groupe OH, NH₂, C₁-C₆ alkyle, C₂-C₆ alcényle, C₂-C₆ alcynyle, un groupe C₃-C₆ cycloalkyle, un groupe hétérocyclique de 3 à 10 chaînons, un groupe C₆-C₁₂ aryle et/ou un groupe hétérocyclique de 5 à 10 chaînons ;
ou sel, solvate ou forme tautomère pharmaceutiquement acceptable de celui-ci.

2. Composé selon la revendication 1, p étant 0 ou p étant 1 et ledit composé de formule (I) étant un composé de formule (Iaiii) ou (Iaiv) :

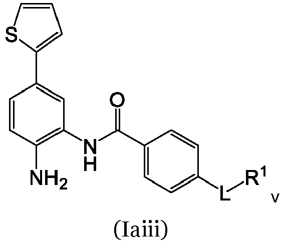

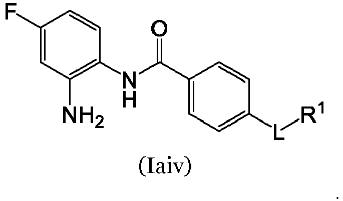

3. Composé selon la revendication 1 ou la revendication 2, R¹ représentant 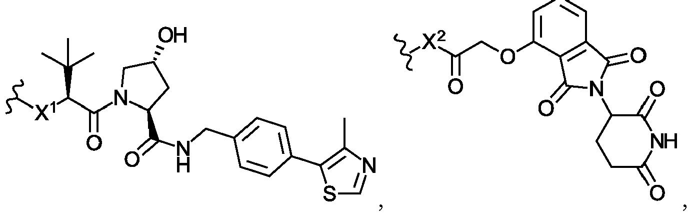 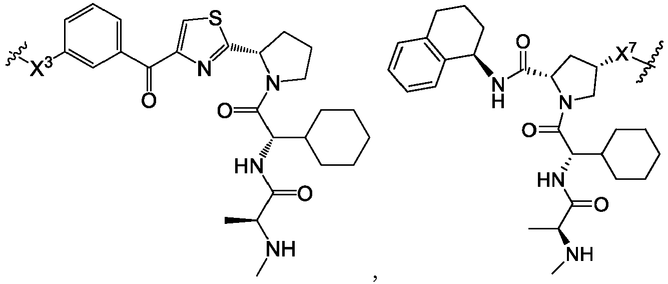 ou 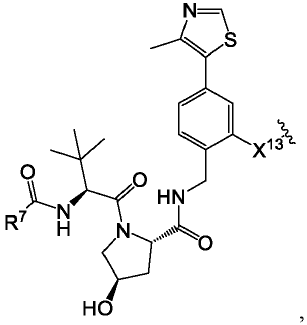 éventuellement X¹, X² et X⁷ représentant un groupe NH et X³ et X¹³ représentant un atome O et R⁷ représentant un groupe méthyle ou

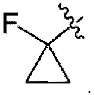

4. Composé selon l'une quelconque des revendications précédentes, ledit squelette du groupe de liaison faisant entre 7 et 40, entre 8 et 30, entre 9 et 25, entre 10 et 20 ou entre 11 et 15 atomes de long.

5. Composé selon l'une quelconque des revendications précédentes, L représentant -L¹-L²- :
L¹ étant absent ou représentant -L³-L⁴-L⁵-L⁶- et L² représentant -L⁷-L⁸-L⁹-L¹⁰- :
L³ représentant un groupe C₁-C₆ alkylène éventuellement substitué, un groupe C₂-C₆ alcénylène éventuellement substitué ou un groupe C₂-C₆ alcynylène éventuellement substitué ;
L⁴ représentant un groupe NR⁵, un atome O, S, 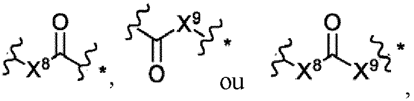 où un astérisque indique un point de liaison à L⁵ ou, si L⁵ est absent, L⁶;
L⁵ étant abset, un groupe C₁-C₆ alkylène éventuellement substitué, un groupe C₂-C₆ alcénylène éventuellement substitué ou un groupe C₂-C₆ alcynylène éventuellement substitué, un groupe C₆-C₁₂ arylène éventuellement substitué ou un groupe hétéroarylène de 5 à 10 chaînons éventuellement substitué ;
L⁶ représentant un groupe C₆-C₁₂ arylène éventuellement substitué ou un groupe hétéroarylène de 5 à 10 chaînons éventuellement substitué ;
L⁷ étant absent, un groupe C₁-C₆ alkylène éventuellement substitué, un groupe C₂-C₆ alcénylène éventuellement substitué ou un groupe C₂-C₆ alcynylène éventuellement substitué ;
L⁸ étant absent, 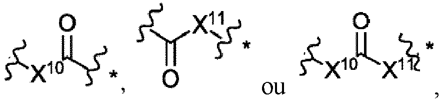 où un astérisque indique un point de liaison à L⁹ ;
L⁹ représentant un groupe C₁-C₂₀ alkylène éventuellement substitué, un groupe C₂-C₂₀ alcénylène éventuellement substitué ou un groupe C₂-C₂₀ alcynylène éventuellement substitué, ledit squelette du groupe alkylène, alcénylène ou alcynylène étant éventuellement interrompu par un ou plusieurs hétéroatomes choisis parmi un atome O ou un groupe NR⁶, ou L⁹ représentant 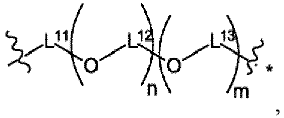 où un astérisque indique un point de liaison à L¹⁰ ou, si L¹⁰ est absent, R¹;
L¹⁰ étant absent, C(O) ou 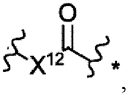 où un astérisque indique un point de liaison à R¹ ;
L¹¹ étant indépendamment absent, un groupe C₁-C₅ alkylène éventuellement substitué, un groupe C₂-C₅ alcénylène éventuellement substitué ou un groupe C₂-C₅ alcynylène éventuellement substitué ;
L¹² et L¹³ représentant indépendamment un groupe C₁-C₅ alkylène éventuellement substitué, un groupe C₂-C₅ alcénylène éventuellement substitué ou un groupe C₂-C₅ alcynylène éventuellement substitué ;
X⁸ à X¹² représentant indépendamment un atome O ou un groupe NR⁶ ;
R⁵ et R⁶ représentant indépendamment un atome H, un groupe C₁-C₆ alkyle, un groupe C₂-C₆ alcényle ou un groupe C₂-C₆ alcynyle ;
m étant 0 un entier compris entre 1 et 10 ; et
n étant un entier compris entre 1 et 10.

6. Composé selon la revendication 5, L¹ représentant 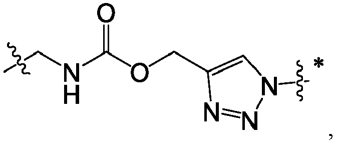 où un astérisque indique un point de liaison à L², ou L¹ étant absent.

7. Composé selon la revendication 5 ou 6, L² représentant 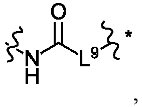 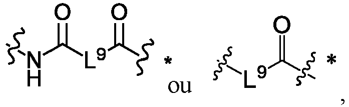 où un astérisque indique un point de liaison à R¹, de préférence L² représentant 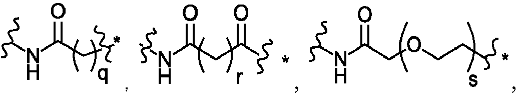 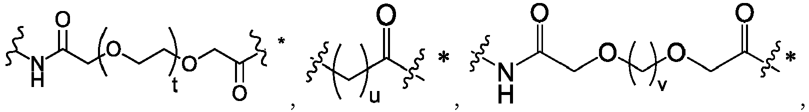 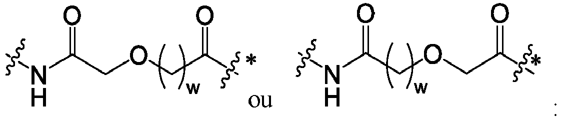
un astérisque indiquant un point de liaison à R¹ ;
q étant un entier d'au moins 5 ;
r étant un entier d'au moins 4 ;
s étant un entier d'au moins 2 ;
t étant un entier d'au moins 1 ;
u étant un entier d'au moins 3 ;
v étant un entier d'au moins 1 ; et
w étant un entier d'au moins 2,
plus préférablement q étant un entier compris entre 5 et 20, entre 7 et 15 ou entre 10 et 12 ;
r étant un entier compris entre 4 et 20, entre 7 et 15 ou entre 9 et 11 ;
s étant un entier compris entre 2 et 10 ou entre 2 et 5 ;
t étant un entier compris entre 1 et 10 ou entre 1 et 5 ;
u étant un entier compris entre 2 et 15, entre 3 et 12, entre 4 et 10 ou entre 6 et 8 ; et
w étant un entier compris entre 2 et 14, entre 3 et 12, entre 5 et 10 ou entre 7 et 9.

8. Composé selon l'une quelconque des revendications précédentes, L représentant 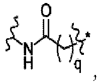 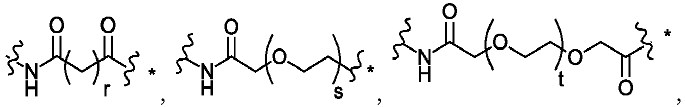 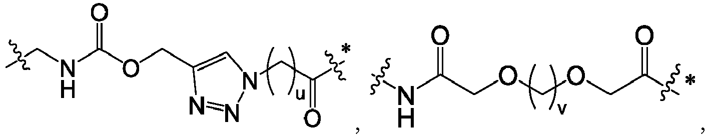 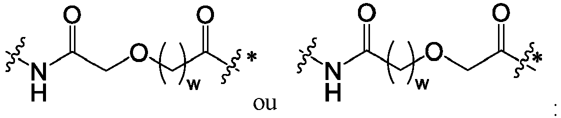
un astérisque indiquant un point de liaison à R¹ ;
q étant un entier d'au moins 5 ;
r étant un entier d'au moins 4 ;
s étant un entier d'au moins 2 ;
t étant un entier d'au moins 1 ;
u étant un entier d'au moins 3 ;
v étant un entier d'au moins 1 ; et
w étant un entier d'au moins 2,
de préférence :
q étant un entier compris entre 5 et 20, entre 7 et 15 ou entre 10 et 12 ;
r étant un entier compris entre 4 et 20, entre 7 et 15 ou entre 9 et 11 ;
s étant un entier compris entre 2 et 10 ou entre 2 et 5 ;
t étant un entier compris entre 1 et 10 ou entre 1 et 5 ;
u étant un entier compris entre 2 et 15, entre 3 et 12, entre 4 et 10 ou entre 6 et 8 ; et
w étant un entier compris entre 2 et 14, entre 3 et 12, entre 5 et 10 ou entre 7 et 9.

9. Composé selon la revendication 1, ledit composé de formule (I) étant un composé de formule (Ic), (Id), (Ie) ou (If) : 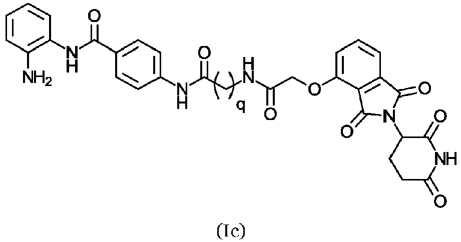 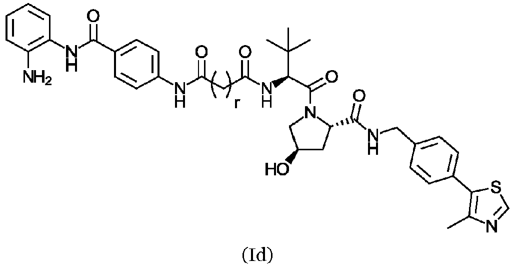 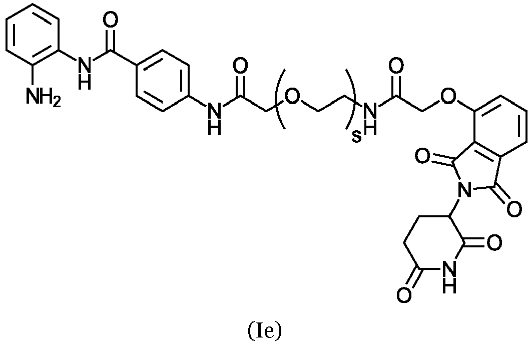 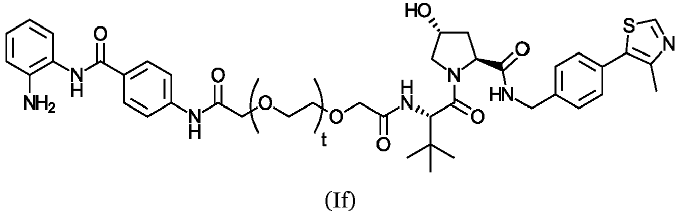 de préférence ledit composé étant un composé de formule (Ic) et q étant 11 ou ledit composé étant un composé de formule (Id) et r étant 10.

10. Composé selon la revendication 1, ledit composé étant un composé de formule (101) à (116) :

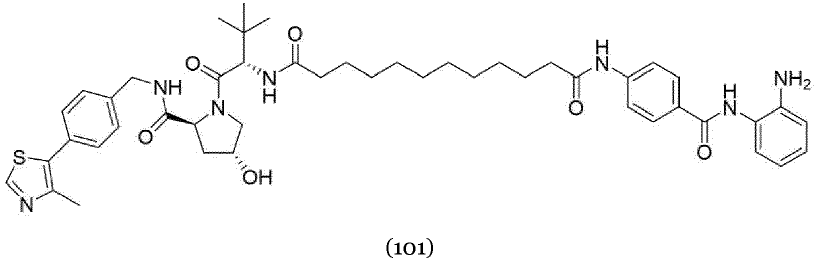

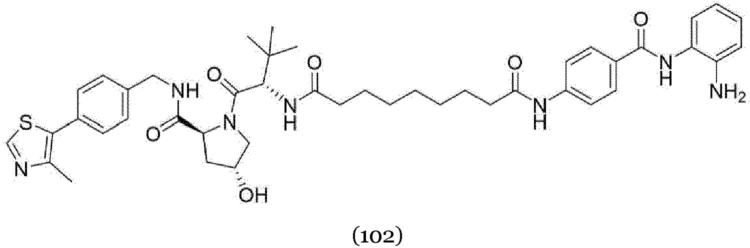

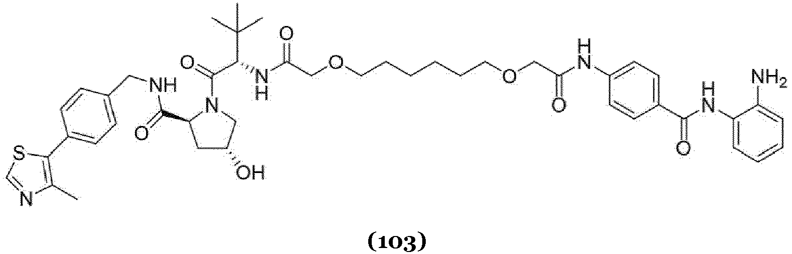

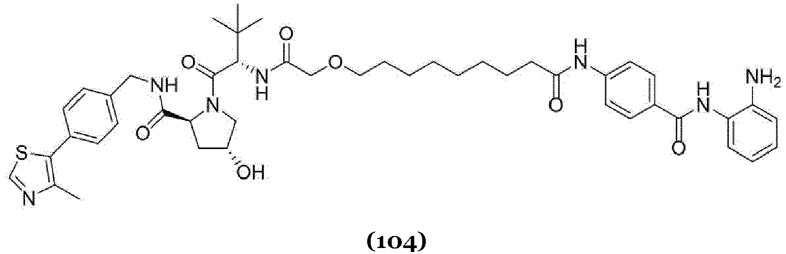

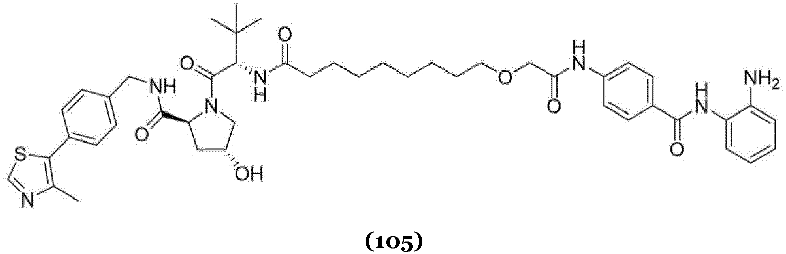

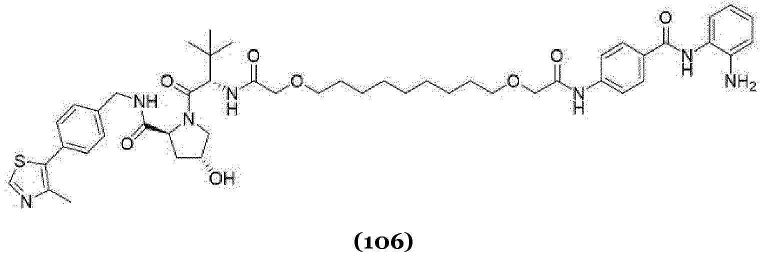

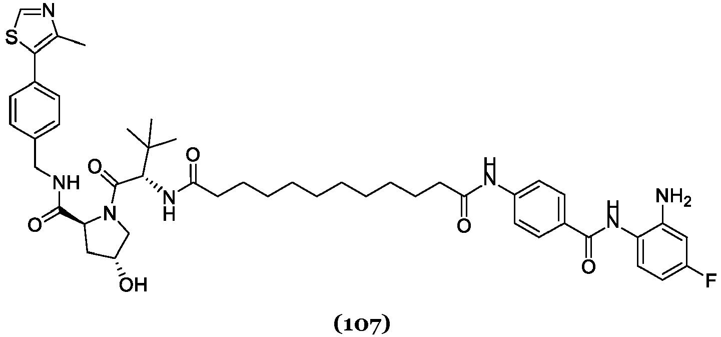

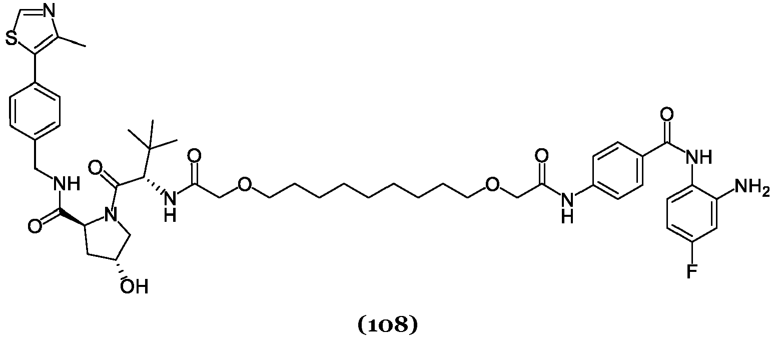

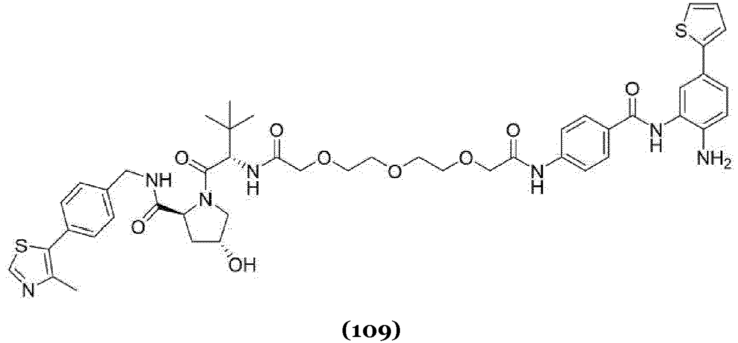

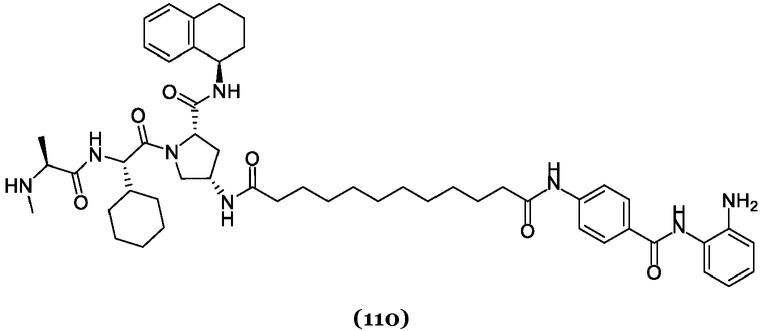

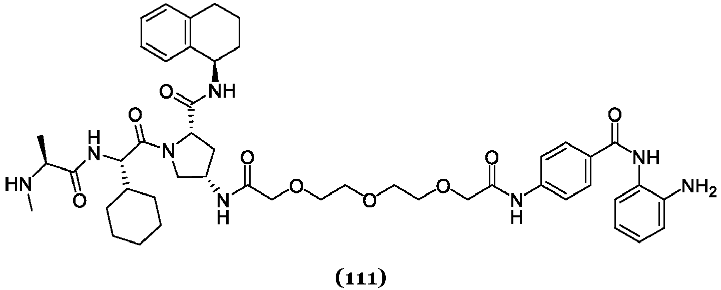

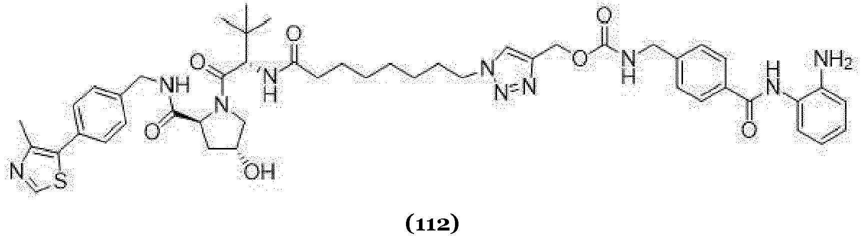

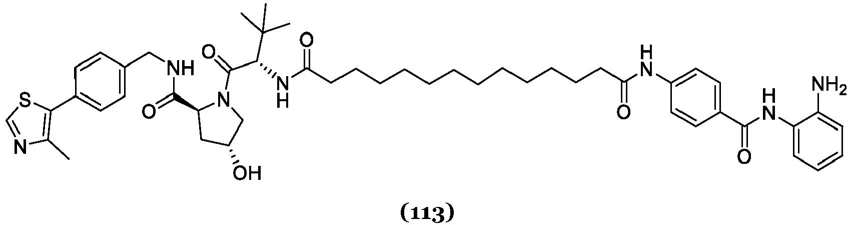

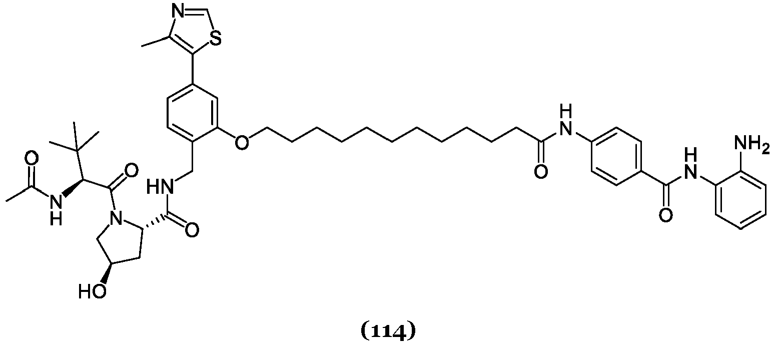

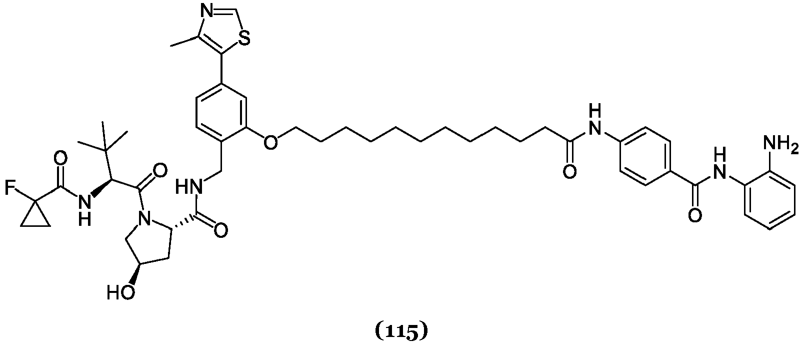

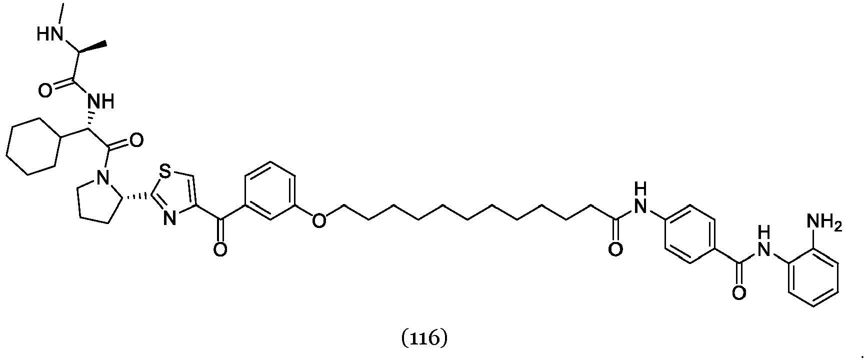

11. Composition pharmaceutique, la composition comprenant un composé de formule (I), selon l'une quelconque des revendications 1 à 10, ou complexe, sel, solvate ou forme tautomère pharmaceutiquement acceptable de celui-ci et un véhicule pharmaceutiquement acceptable.

12. Composé de formule (I), selon l'une quelconque des revendications 1 à 10, ou complexe, sel, solvate ou forme tautomère pharmaceutiquement acceptable de celui-ci, ou composition pharmaceutique, selon la revendication 11, destiné à un usage thérapeutique.

13. Composé de formule (I), selon l'une quelconque des revendications 1 à 10, ou complexe, sel, solvate ou forme tautomère pharmaceutiquement acceptable de celui-ci, ou composition pharmaceutique, selon la revendication 11, destiné à être utilisé dans le traitement d'un cancer, de préférence ledit cancer étant le cancer du sang, le cancer de l'intestin, le cancer du cerveau, le cancer du sein, le cancer du col de l'utérus, le cancer de l'endomètre, le cancer de l'estomac, le cancer du foie, le cancer du poumon, le cancer de l'ovaire, le cancer du pancréas, le cancer de la prostate ou le cancer de la peau.
